# EUROPEAN PATENT APPLICATION

(11) **EP 4 180 434 A1**
(43) Date of publication of application: **17.05.2023**
(21) Application number: 21838608.4
(22) Date of filing: 09.07.2021
(51) Int. Cl.: C07D 487/04, C07D 401/04, C07D 471/04, A61K 31/53, A61K 31/44, A61P 31/16

(54) **PB2 INHIBITOR, AND PREPARATION METHOD THEREFOR AND USE THEREOF**

(30) Priority: 10.07.2020 CN 202010658037; 20.10.2020 CN 202011121839
(71) Applicant: Sichuan Haisco Pharmaceutical Co., Ltd., Sichuan 611130 (CN)
(72) Inventor: LI, Yao, Chengdu, Sichuan 611130 (CN); CHEN, Lei, Chengdu, Sichuan 611130 (CN); SHI, Zongjun, Chengdu, Sichuan 611130 (CN); ZHANG, Guobiao, Chengdu, Sichuan 611130 (CN); WANG, Wenjing, Chengdu, Sichuan 611130 (CN); YE, Fei, Chengdu, Sichuan 611130 (CN); HU, Gang, Chengdu, Sichuan 611130 (CN); HE, Tiancheng, Chengdu, Sichuan 611130 (CN); WANG, Haodong, Chengdu, Sichuan 611130 (CN); NI, Jia, Chengdu, Sichuan 611130 (CN); ZHANG, Chen, Chengdu, Sichuan 611130 (CN); YAN, Pangke, Chengdu, Sichuan 611130 (CN)
(74) Representative: Sagittarius IP
(86) International application number: PCT/CN2021/105622
(87) International publication number: WO 2022/007966

(57) **Abstract**

The present invention relates to a compound of formula (I); a stereoisomer, a tautomer, a nitrogen oxide, a solvate, a metabolite, a pharmaceutically acceptable salt, a co-crystal or a prodrug thereof, or a pharmaceutical composition containing same; and the use thereof as a PB2 inhibitor in the preparation of a drug for treating related diseases. Each group in formula (I) is as defined in the description.

Cy₁-L₁-Cy₂-L₂-Cy₃ _{(I)}

## Description

### Technical Field

The present invention belongs to the field of drugs, and in particular relates to a bridged ring compound, or a stereoisomer, a tautomer, a nitrogen oxide, a solvate, a metabolite, a pharmaceutically acceptable salt, a co-crystal or a prodrug thereof, and the use thereof in the preparation of a drug for treating related diseases.

### Background Art

Influenza (flu for short) is an acute respiratory infection caused by influenza viruses and is also a highly infectious and fast-transmitting disease. Generally, influenza occurs most frequently in autumn and winter seasons, and causes very serious complications and deaths. According to the WHO reports, influenza viruses cause 3-5 million infections and 650,000 deaths each year. They are divided into three types: A, B and C according to different gene characteristics and nucleoprotein antigens of influenza viruses. Specifically, influenza A viruses are the most virulent of the three types to humans and may cause severe diseases and explosive epidemics. Moreover, influenza A viruses are the most susceptible to mutation, and are divided into different subtypes according to their different surface antigen hemagglutinins and neuraminidases, with a total of 17 subtypes from H1 to H17 for hemagglutinins and 10 different subtypes from N1 to N10 for neuraminidases, which hemagglutinins and neuraminidase can be combined to form different subtypes of influenza A viruses.

Influenza vaccination is the main measure to prevent and control influenza, which can reduce the chance of infecting influenza or attenuating influenza symptoms in the vaccinated person. However, seasonal influenza vaccines still have their own limitations. Firstly, influenza vaccines provide better protection in healthy adults but are less effective in older adults. Secondly, seasonal influenza vaccines must be updated every year to prevent the type of viruses which are predicted to be pandemic in a new influenza season. However, due to the emergence of new virus types, vaccine protection may not be as good as expected.

In addition, these influenza vaccines have variable efficacy. Certain influenza vaccines usually confer protection for no more than several years due to the high mutation rate of viruses. Because viruses change rapidly over time and different strains become dominant, a vaccine formulated for one year may not be effective in the next year.

Likewise, the RNA-dependent RNA polymerase of influenza vRNA generates one nucleotide insertion error approximately every 10,000 nucleotides (which is the approximate length of influenza vRNA) due to the lack of an RNA proofreading enzyme. Therefore, almost every newly generated influenza virus is due to a mutation, i.e., antigenic drift. If more than one virus line infects a single cell, the genome is segregated into 8 separate vRNA fragments to mix or reassort vRNAs. The resulting rapid changes in viral genetics produce antigenic shift, allow the virus to infect new host species, and rapidly overcome protective immunity.

Antiviral drugs can also be used to treat influenza, and existing anti-influenza virus drugs mainly target the hemagglutinin receptor, neuraminidase and matrix protein of the viral envelope. With the extensive use of existing anti-influenza virus drugs, virus strains resistant to these drugs account for an increasing proportion. However, the viruses are less likely to develop resistance to drugs that selectively act on influenza virus RNA polymerase and have no serious toxic and side effects on the human body. Influenza virus RNA polymerase plays an important role in the life cycle of influenza viruses, and has become a new target of anti-influenza viruses that has attracted much attention in recent years.

Therefore, there remains a need for a drug as an influenza virus RNA polymerase basic protein 2 inhibitor (PB2 inhibitor) for treating influenza infection, e.g., a drug with an expanded therapeutic window and/or a reduced sensitivity to viral titres.

### Summary of the Invention

An objective of the present invention is to provide an azaindole derivative with a novel structure, which has the advantages of good efficacy, high bioavailability and less side effects as an influenza virus RNA polymerase basic protein 2 inhibitor.

The present invention relates to a compound of formula (I), or a stereoisomer, a tautomer, a nitrogen oxide, a solvate, a metabolite, a pharmaceutically acceptable salt, a co-crystal or a prodrug thereof,

Cy₁-L₁-Cy₂-L₂-Cy₃ (I)

wherein C_{y1} is selected from 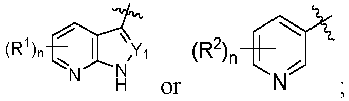 further, C_{y1} is selected from 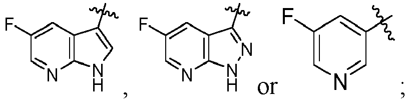 further, C_{y1} is selected from 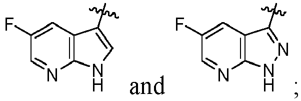 and further, C_{y1} is selected from 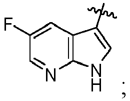
Y₁ is selected from -CH- or -N-;
n is selected from 1, 2 or 3; and further, n is selected from 1;
R¹ and R² are each independently selected from F, Cl, Br, NH₂, C₁₋₆ alkyl or C₃₋₆ monocyclic cycloalkyl, and the R¹ and R² are optionally further substituted with 1-5 groups selected from halogen, OH or deuterium; and further, R¹ and R² are each independently selected from F;
C_{y2} is selected from a bond, C₃₋₁₂ carbocycle or 3-12-membered heterocycle, and the C_{y2} is optionally further substituted with 1-5 groups selected from R^{A};
each R^{A} is independently selected from halogen, C₁₋₆ alkyl, C₂₋₆ alkenyl, C₂₋₆ alkynyl, 5-12-membered heteroaryl, phenyl, -NR^{B}C(O)C₁₋₆ alkyl, -C(O)NR^{B}C₁₋₆ alkyl, -C(O)C₁₋₆ alkyl, CN, =O, C₁₋₆ alkoxy C(O)-, -COOH, OH, -NR^{B}R^{C}, deuterium, -SH, -S(C₁₋₆ alkyl), -S(O)₂(C₁₋₆ alkyl) or C₃₋₆ monocyclic cycloalkyl, and the R^{A} is optionally further substituted with 1-5 groups selected from halogen, C₁₋₆ alkyl, C₁₋₆ alkoxy, C₁₋₆ alkylamino, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) or deuterium;
C_{y3} is selected from 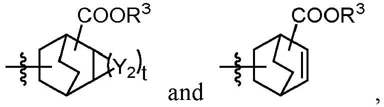 and the C_{y3} is optionally substituted with 1-5 groups selected from R_{cy3}; further, C_{y3} is selected from 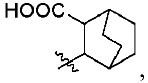 and the C_{y3} is optionally substituted with 1-3 groups selected from R_{cy3}; further, C_{y3} is selected from 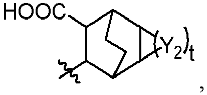 and the C_{y3} is optionally substituted with 1-3 groups selected from R_{cy3}, wherein t is selected from 1, 2 or 3, each Y₂ is independently selected from -CH₂-, -NH-, O and S, and each H atom in the Y₂ can be independently substituted with R_{cy3} group; and further, C_{y3} is selected from 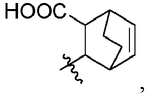 and the C_{y3} is optionally substituted with 1-3 groups selected from R_{cy3};
t is selected from 0, 1, 2, 3 or 4; further, t is selected from 0, 1, 2 or 3; further, t is selected from 0, 1 or 2; further, t is selected from 0 or 1; and further, t is selected from 0;
each Y₂ is independently selected from -CH₂-, -N-, -NH-, O, S, S(O) and S(O)₂, and each H atom in the Y₂ can be independently substituted with R_{cy3} group; further, each Y₂ is independently selected from -CH-, -CH₂-, -NH-, O and S, and each H atom in the Y₂ can be independently substituted with R_{cy3} group; further, each Y₂ is independently selected from -CH-, -CH₂-, -NH- and O, and each H atom in the Y₂ can be independently substituted with R_{cy3} group; and further, each Y₂ is independently selected from -CH- and -CH₂-, and each H atom in the Y₂ can be independently substituted with R_{cy3} group;
each R_{cy3} is independently selected from F, Cl, Br, OH, CN, C₁₋₆ alkyl, C₁₋₆ alkoxy, halo C₁₋₆ alkyl and hydroxy C₁₋₆ alkyl; further, each R_{cy3} is independently selected from F, Cl, OH, CN, C₁₋₂ alkyl, C₁₋₂ alkoxy, halo C₁₋₂ alkyl and hydroxy C₁₋₂ alkyl; further, each R_{cy3} is independently selected from F, Cl, OH, C₁₋₂ alkyl and halo C₁₋₂ alkyl; and further, each R_{cy3} is independently selected from F, Cl and OH;
each R³ is independently selected from H and C₁₋₆ alkyl; further, each R³ is independently selected from H and C₁₋₂ alkyl; and further, each R³ is independently selected from H;
L₁ is selected from a bond, C₂₋₆ alkynylene, C₂₋₆ alkenylene or -C(O)-; further, L₁ is selected from a bond, C₂₋₄ alkynylene, C₂₋₄ alkenylene or -C(O)-; further, L₁ is selected from a bond; further, L₁ is selected from C₂₋₄ alkynylene and C₂₋₄ alkenylene; and further, L₁ is selected from -C(O)-;
L₂ is selected from a bond, -NR^{B}- or -C(O)NR^{B}-; further, L₂ is selected from a bond, -NH-, -NCH₃- or -C(O)NH-; further, L₂ is selected from -NH-, -NCH₃- or - C(O)NH-; further, L₂ is selected from -NH- or -NCH₃-; and further, L₂ is selected from -NH-; and
wherein R^{B} and R^{C} are each independently selected from H, deuterium, C₁₋₆ alkyl, halo C₁₋₆ alkyl, -OC(O)CH₃ or deuterated C₁₋₆ alkyl.

As a first technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof,

Cy₁-L₁-Cy₂-L₂-C_{y3} (I)

wherein C_{y1} is selected from 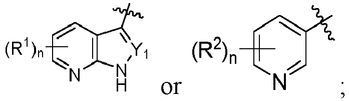
Y₁ is selected from -CH- or -N-;
n is selected from 1, 2 or 3;
R¹ and R² are each independently selected from F, Cl, Br, NH₂, C₁₋₆ alkyl or C₃₋₆ monocyclic cycloalkyl, and the R¹ and R² are optionally further substituted with 1-5 groups selected from halogen, OH or deuterium;
C_{y2} is selected from a bond, C₃₋₁₂ carbocycle or 3-12-membered heterocycle, and the C_{y2} is optionally further substituted with 1-5 groups selected from R^{A};
each R^{A} is independently selected from halogen, C₁₋₆ alkyl, C₂₋₆ alkenyl, C₂₋₆ alkynyl, 5-12-membered heteroaryl, phenyl, -NR^{B}C(O)C₁₋₆ alkyl, -C(O)NR^{B}C₁₋₆ alkyl, -C(O)C₁₋₆ alkyl, CN, =O, C₁₋₆ alkoxy C(O)-, -COOH, OH, -NR^{B}R^{C}, deuterium, -SH, -S(C₁₋₆ alkyl), -S(O)₂(C₁₋₆ alkyl) or C₃₋₆ monocyclic cycloalkyl, and the R^{A} is optionally further substituted with 1-5 groups selected from halogen, C₁₋₆ alkyl, C₁₋₆ alkoxy, C₁₋₆ alkylamino, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) or deuterium;
C_{y3} is selected from 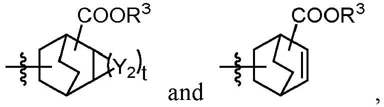 and the C_{y3} is optionally substituted with 1-5 groups selected from R_{cy3};
t is selected from 0, 1, 2, 3 or 4;
each Y₂ is independently selected from -CH₂-, -N-, -NH-, O, S, S(O) and S(O)₂, and each H atom in the Y₂ can be independently substituted with R_{cy3} group;
each R_{cy3} is independently selected from F, Cl, Br, OH, CN, C₁₋₆ alkyl, C₁₋₆ alkoxy, halo C₁₋₆ alkyl and hydroxy C₁₋₆ alkyl;
each R³ is independently selected from H and C₁₋₆ alkyl;
L₁ is selected from a bond, C₂₋₆ alkynylene, C₂₋₆ alkenylene or -C(O)-;
L₂ is selected from a bond, -NR^{B}- or -C(O)NR^{B}-;
and wherein R^{B} and R^{C} are each independently selected from H, deuterium, C₁₋₆ alkyl, -OC(O)CH₃, halo C₁₋₆ alkyl or deuterated C₁₋₆ alkyl.

As a second technical solution of the present invention, the present invention relates to the compound of formula (I), or the stereoisomer, the tautomer, the nitrogen oxide, the solvate, the metabolite, the pharmaceutically acceptable salt, the co-crystal or the prodrug thereof according to the first technical solution, having a structure of formula (II), (III), (IV), or (V):

Cy₁-C_{y2a}-NH-Cy₃ (II)

C_{y1}-C_{y2b}-C_{y3} (III)

Cy₁-L₁ₐ-C_{y2c}-L₂ₐ-Cy₃ (IV)

Cy₁-L_{1b}- L_{2b}-Cy₃ (V)

wherein
C_{y1} is selected from 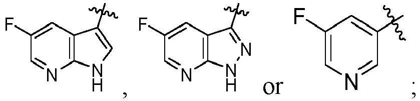
C_{y3} is selected from 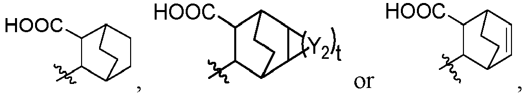 and the C_{y3} is optionally substituted with 1-5 groups selected from R_{cy3};
each Y₂ is independently selected from -CH₂-, -N-, -NH-, O, S, S(O) and S(O)₂, and each H atom in the Y₂ can be independently substituted with R_{cy3} group;
t is selected from 1, 2, 3 or 4;
each R_{cy3} is independently selected from F, Cl, Br, OH, CN, C₁₋₄ alkyl, C₁₋₄ alkoxy, halo C₁₋₄ alkyl or hydroxy C₁₋₄ alkyl;
C_{y2a} is selected from the following groups, wherein * represents the coupling site of C_{y2a} with NH:
   (a) 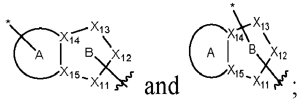
   (b) 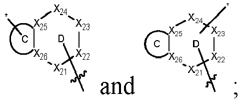
   (c) 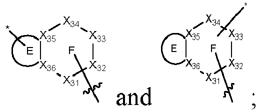
   (d) 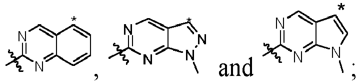
   (e) 5-membered heteroaryl, 5-membered unsaturated monocyclic heterocycloalkyl and 6-membered saturated monocyclic heterocycloalkyl;
   (f) 6-membered unsaturated monocyclic heterocycloalkyl; 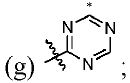
   and (h) 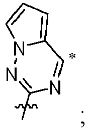
wherein the groups of (a) - (f) in C_{y2a} are optionally further substituted with 1-5 groups selected from R^{A}, and the group of (g) in C_{y2a} is substituted with 1 group selected from R^{D};
when C_{y1} is selected from 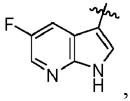 the group of (h) in C_{y2a} is substituted with 1-3 groups selected from R^{E}; or
when C_{y1} is selected from 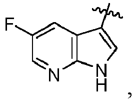 and C_{y2a} is (h), C_{y3} is substituted with 1-5 groups selected from R_{cy3}; or
when C_{y1} is selected from 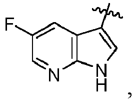 and C_{y2a} is (h), C_{y3} is selected from 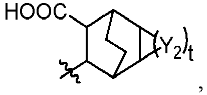 and t = 2, 3 or 4;
when C_{y1} is selected from 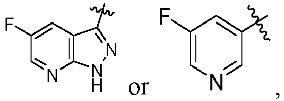 the group of (h) in C_{y2a} is optionally further substituted with 1-3 groups selected from R^{A};
each ring A is independently selected from 4-6-membered monocyclic cycloalkyl, 4-6-membered monocyclic heterocycloalkyl, 5-6-membered heteroaryl or phenyl;
each ring C is independently selected from 5-membered monocyclic cycloalkyl, 5-membered monocyclic heterocycloalkyl or 5-membered heteroaryl;
each ring E is independently selected from 6-membered monocyclic cycloalkyl, 6-membered monocyclic heterocycloalkyl, 6-membered heteroaryl or phenyl;
X₁₁, X₁₂ and X₁₃ are each independently selected from -CH-, -CR^{A}-, -CH₂-, - CHR^{A}-, -C(R^{A})₂-, -N-, -NH-, -NR^{A}-, O, S, S(O) or S(O)₂, X₁₄ and X₁₅ are each independently selected from -C-, -CH-, -CR^{A}- or -N-, and ring B is a saturated ring, a partially unsaturated ring or a heteroaryl ring;
X₂₁, X₂₂, X₂₃ and X₂₄ are each independently selected from -CH-, -CR^{A}-, -CH₂-, -CHR^{A}-, -C(R^{A})₂-, -N-, -NH-, -NR^{A}-, O, S, S(O) or S(O)₂, X₂₅ and X₂₆ are each independently selected from -C-, -CH-, -CR^{A}- or -N-, and ring D is a saturated ring, a partially unsaturated ring, a benzene ring or a heteroaryl ring, provided that D-fused ring C does not form substituted or unsubstituted 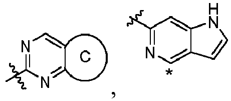 and 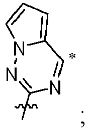
X₃₁, X₃₂, X₃₃ and X₃₄ are each independently selected from -CH-, -CR^{A}-, -CH₂-, -CHR^{A}-, -C(R^{A})₂-, -N-, -NH-, -NR^{A}-, O, S, S(O) or S(O)₂, X₃₅ and X₃₆ are each independently selected from -C-, -CH-, -CR^{A}- or -N-, and ring F is a saturated ring, a partially unsaturated ring, a benzene ring or a heteroaryl ring, provided that F-fused ring E does not form substituted or unsubstituted 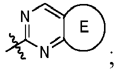
each R^{A} is independently selected from halogen, C₁₋₆ alkyl, C₁₋₆ alkoxy, C₂₋₆ alkenyl, C₂₋₆ alkynyl, 5-12-membered heteroaryl, phenyl, -NR^{B}C(O)C₁₋₆ alkyl, - C(O)NR^{B}C₁₋₆ alkyl, -C(O)C₁₋₆ alkyl, CN, =O, C₁₋₆ alkoxy C(O)-, -COOH, OH, - NR^{B}R^{C}, deuterium, -SH, -S(C₁₋₆ alkyl), -S(O)₂(C₁₋₆ alkyl) and C₃₋₆ monocyclic cycloalkyl, and the R^{A} is optionally further substituted with 1-5 groups selected from halogen, C₁₋₆ alkyl, C₁₋₆ alkoxy, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) and deuterium;
each R^{D} is selected from C₁₋₆ alkyl, 5-6-membered heteroaryl and phenyl, and the heteroaryl and phenyl are substituted with 1-2 groups selected from -Si(C₁₋₆ alkyl)₃;
each R^{E} is selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-12-membered heteroaryl, phenyl, - NR^{B}C(O)C₁₋₆ alkyl, -C(O)NR^{B}C₁₋₆ alkyl, -C(O)C₁₋₆ alkyl, =O, C₁₋₆ alkoxy C(O)-, - COOH, OH, -NR^{B}R^{C}, deuterium, -SH, -S(C₁₋₆ alkyl), -S(O)₂(C₁₋₆ alkyl), C₃₋₆ monocyclic cycloalkyl and C₃₋₆ monocyclic heterocyclic cycloalkyl, and the R^{E} is optionally further substituted with 1-5 groups selected from halogen, C₁₋₆ alkyl, C₁₋₆ alkoxy, C₁₋₆ alkylamino, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) and deuterium;
C_{y2b} is selected from 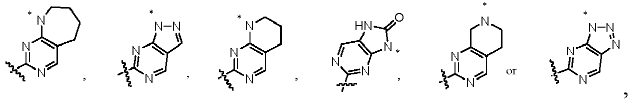 wherein * represents the coupling site of C_{y2b} with 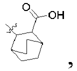 and the C_{y2b} is optionally substituted with 1-5 groups selected from R^{A};
C_{y2c} is selected from C₃₋₁₂ carbocycle and 5-12-membered heterocycle, and the C_{y2c} is optionally further substituted with 1-5 groups selected from R^{A};
L₁ₐ is selected from a bond, C₂₋₄ alkynylene, C₂₋₄ alkenylene or -C(O)-; L₂ₐ is selected from -NR^{B}- and -^{∗}C(O)NR^{B}-, wherein * represents the coupling site of L₂ₐ with C_{y2c}, provided that when L₁ₐ is selected from a bond, L₂ₐ is selected from - ^{∗}C(O)NR^{B}-;
L_{1b} is selected from C₂₋₄ alkynylene or C₂₋₄ alkenylene, and L_{2b} is selected from -NR^{B}- or -^{∗}C(O)NR^{B}-, wherein * represents the coupling site of L_{2b} with L_{1b};
and wherein R^{B} and R^{C} are each independently selected from H, deuterium, C₁₋₆ alkyl, -OC(O)CH₃, halo C₁₋₆ alkyl and deuterated C₁₋₆ alkyl;
provided that C_{y2a} is not selected from substituted or unsubstituted 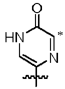 and substituted or unsubstituted

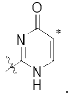

As a third technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 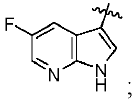 or
C_{y1} is selected from 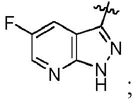 or
C_{y1} is selected from 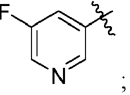
and the definitions of other groups are consistent with those of the first or second technical solution.

As a fourth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y3} is selected from 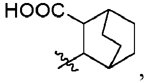 and the C_{y3} is optionally substituted with 1-3 groups selected from R_{cy3}; or
C_{y3} is selected from 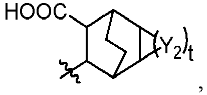 and the C_{y3} is optionally substituted with 1-3 groups selected from R_{cy3}, wherein t is selected from 1, 2 or 3, each Y₂ is independently selected from -CH₂-, -NH-, O and S, and each H atom in the Y₂ can be independently substituted with R_{cy3} group; or
C_{y3} is selected from 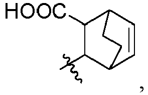 and the C_{y3} is optionally substituted with 1-3 groups selected from R_{cy3};
and the definitions of other groups are consistent with those of any one of the first to third technical solutions.

As a fifth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y3} is selected from 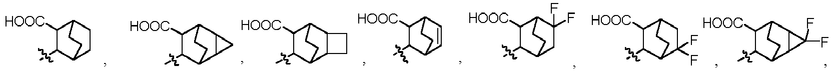 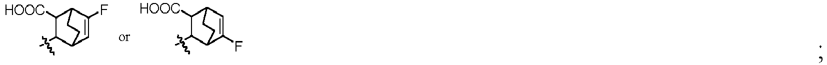
and the definitions of other groups are consistent with those of any one of the first to third technical solutions.

As a sixth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
each R_{cy3} is independently selected from F, Cl and CN;
and the definitions of other groups are consistent with those of any one of the first to fifth technical solutions.

As a seventh technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y3} is selected from 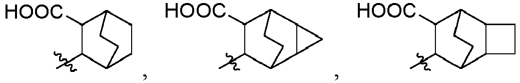 or 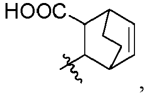 and the C_{y3} is optionally substituted with 1-3 groups selected from Rcy3;
C_{y1} is selected from 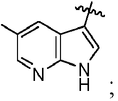
and the definitions of other groups are consistent with those of any one of the first to sixth technical solutions.

As an eighth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2a} is selected from 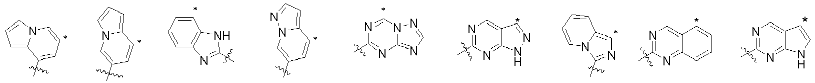 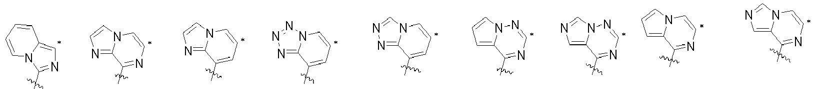 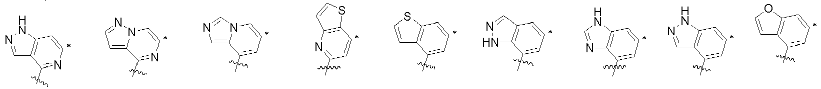 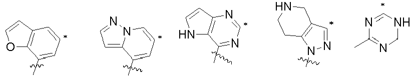 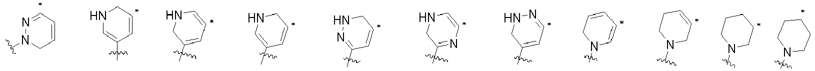 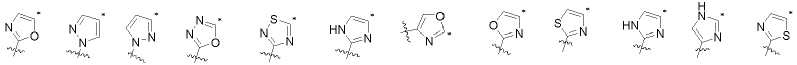 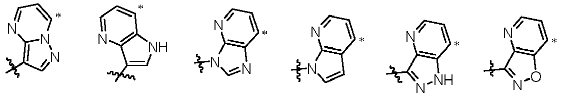 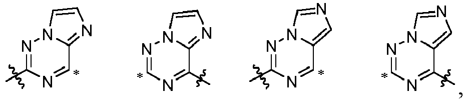 and the C_{y2a} is optionally substituted with 1-3 groups selected from R^{A}; or
C_{y2a} is selected from 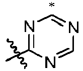 and is substituted with 1 group selected from methyl or 5-membered heteroaryl, and the 5-membered heteroaryl is substituted with 1 trimethylsilyl group; or
C_{y1} is selected from 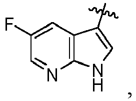 C_{y2a} is selected from 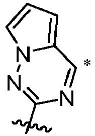 and is substituted with 1 group selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, - COOH, OH, -NR^{B}R^{C}, deuterium, C₃₋₆ monocyclic cycloalkyl or C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium; or
C_{y1} is selected from 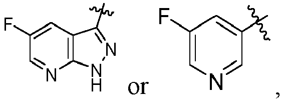 C_{y2a} is selected from 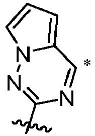 and is optionally substituted with 1-2 groups selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, -COOH, OH, -NR^{B}R^{C}, deuterium, C₃₋₆ monocyclic cycloalkyl and C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium;
and the definitions of other groups are consistent with those of any one of the first to seventh technical solutions.

As a ninth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2a} is selected from 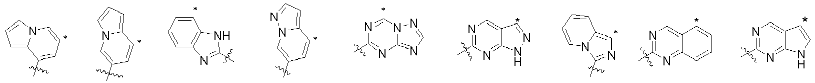 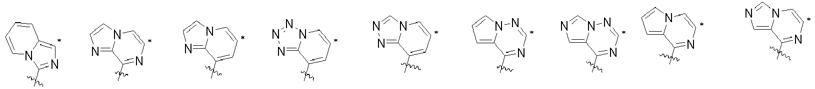 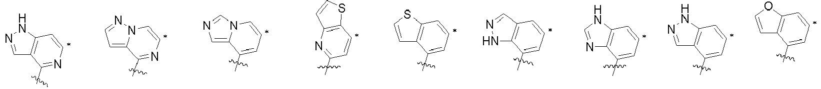 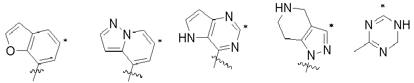 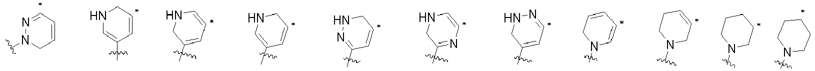 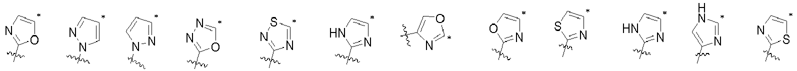 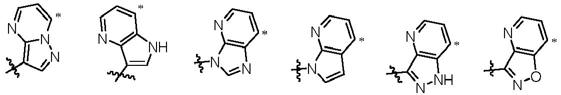 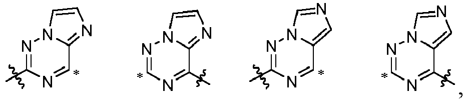 and the C_{y2a} is optionally substituted with 1-3 groups selected from R^{A};
and the definitions of other groups are consistent with those of any one of the above-mentioned technical solutions.

As a tenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2a} is selected from 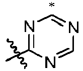 and is substituted with 1 group selected from methyl or 5-membered heteroaryl, and the 5-membered heteroaryl is substituted with 1 trimethylsilyl group;
and the definitions of other groups are consistent with those of any one of the above-mentioned technical solutions.

As an eleventh technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 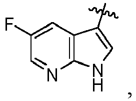 C_{y2a} is selected from 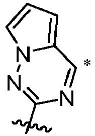 and is substituted with 1 group selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, - COOH, OH, -NR^{B}R^{C}, deuterium, C₃₋₆ monocyclic cycloalkyl or C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium;
and the definitions of other groups are consistent with those of any one of the above-mentioned technical solutions.

As a twelfth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 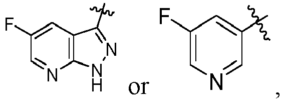 C_{y2a} is selected from 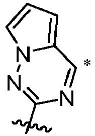 and is optionally substituted with 1-2 groups selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, -COOH, OH, -NR^{B}R^{C}, deuterium, C₃₋₆ monocyclic cycloalkyl and C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium;
and the definitions of other groups are consistent with those of any one of the above-mentioned technical solutions.

As a thirteenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 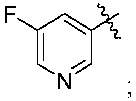
C_{y2a} is selected from a five-membered ring fused five-membered ring, a five-membered ring fused six-membered ring, a six-membered ring fused five-membered ring and a six-membered ring fused six-membered ring, and the C_{y2a} is optionally further substituted with 1-5 groups selected from R^{A}; or
C_{y2a} is selected from a five-membered heteroaryl fused six-membered heteroaryl, a six-membered heteroaryl fused six-membered heteroaryl, a five-membered heteroaryl fused five-membered heteroaryl and a six-membered heteroaryl fused five-membered heteroaryl, the C_{y2a} is optionally further substituted with 1-3 groups selected from halogen, hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, - COOH, OH, -NR^{B}R^{C}, deuterium, C₃₋₆ monocyclic cycloalkyl and C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium; or
C_{y2a} is selected from a five-membered heteroaryl fused six-membered heteroaryl, a six-membered heteroaryl fused six-membered heteroaryl, a five-membered heteroaryl fused five-membered heteroaryl and a six-membered heteroaryl fused five-membered heteroaryl, and the C_{y2a} is optionally further substituted with 1-3 groups selected from halogen and -NR^{B}R^{C}; or
C_{y2a} is selected from 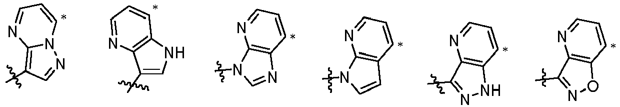 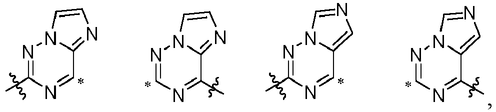 and the C_{y2a} is optionally further substituted with 1-3 groups selected from halogen and -NR^{B}R^{C}; or C_{y2a} is selected from

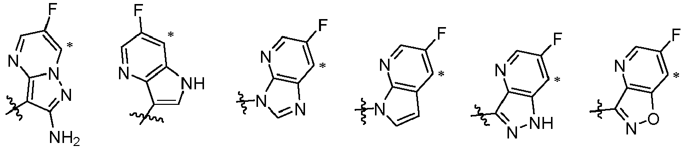

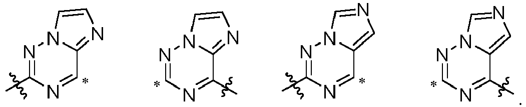

As a fourteenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 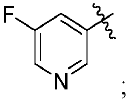
C_{y2a} is selected from a five-membered ring fused five-membered ring, a five-membered ring fused six-membered ring, a six-membered ring fused five-membered ring and a six-membered ring fused six-membered ring, and the C_{y2a} is optionally further substituted with 1-5 groups selected from R^{A};
and the definitions of other groups are consistent with those of any one of the foregoing technical solutions.

As a fifteenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 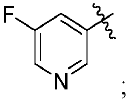
C_{y2a} is selected from a five-membered heteroaryl fused six-membered heteroaryl, a six-membered heteroaryl fused six-membered heteroaryl, a five-membered heteroaryl fused five-membered heteroaryl and a six-membered heteroaryl fused five-membered heteroaryl, the C_{y2a} is optionally further substituted with 1-3 groups selected from halogen, hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, - COOH, OH, -NR^{B}R^{C}, deuterium, C₃₋₆ monocyclic cycloalkyl and C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium;
and the definitions of other groups are consistent with those of any one of the foregoing technical solutions.

As a sixteenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 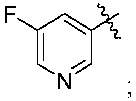
C_{y2a} is selected from a five-membered heteroaryl fused six-membered heteroaryl, a six-membered heteroaryl fused six-membered heteroaryl, a five-membered heteroaryl fused five-membered heteroaryl and a six-membered heteroaryl fused five-membered heteroaryl, and the C_{y2a} is optionally further substituted with 1-3 groups selected from halogen and -NR^{B}R^{C};
and the definitions of other groups are consistent with those of any one of the foregoing technical solutions.

As a seventeenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein 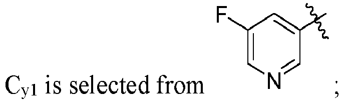
C_{y2a} is selected from 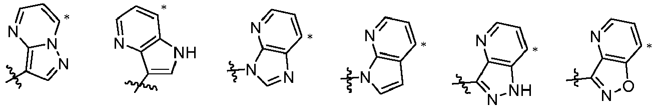 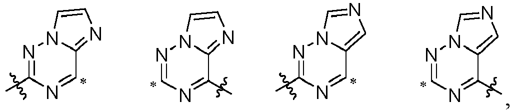 and the C_{y2a} is optionally further substituted with 1-3 groups selected from halogen and -NR^{B}R^{C};
and the definitions of other groups are consistent with those of any one of the foregoing technical solutions.

As an eighteenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 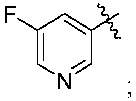
C_{y2a} is selected from 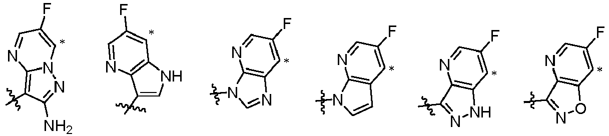 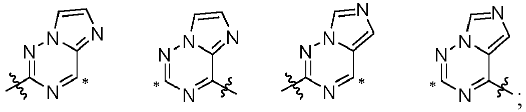
and the definitions of other groups are consistent with those of any one of the foregoing technical solutions.

As a nineteenth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 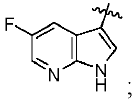
C_{y2a} is selected from 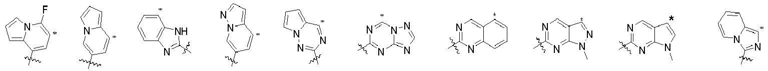 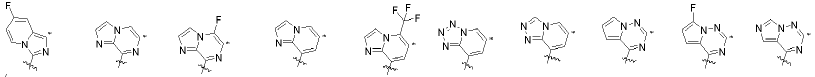 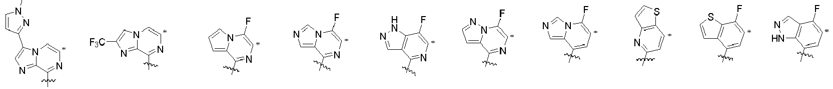 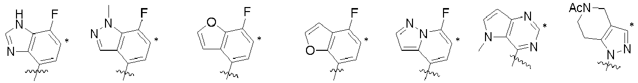 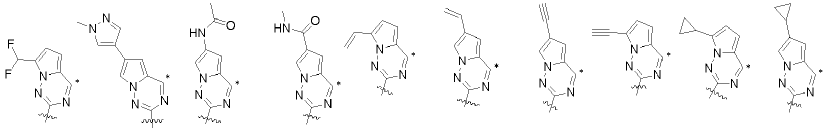 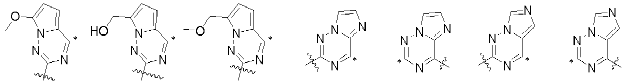 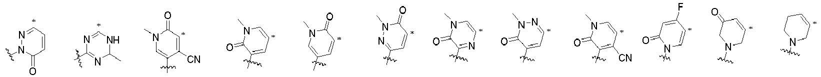 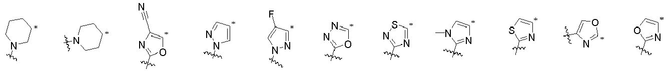 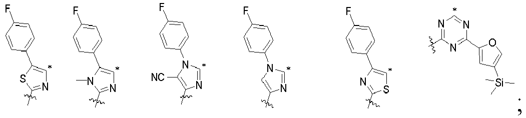
and the definitions of other groups are consistent with those of any one of the foregoing technical solutions. As a twentieth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y1} is selected from 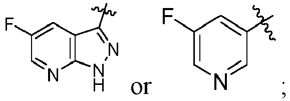
C_{y2a} is selected from 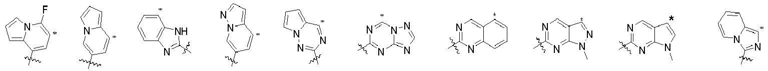 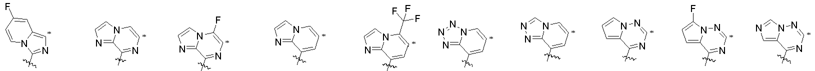 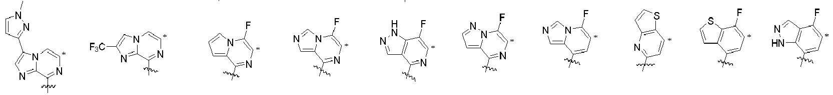 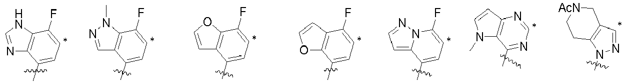 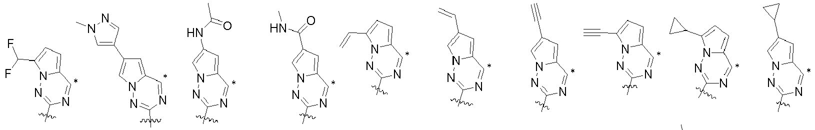 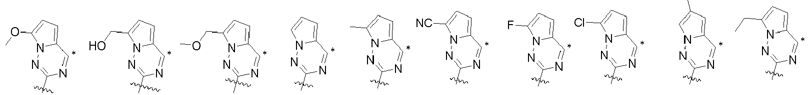 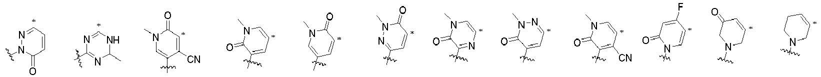 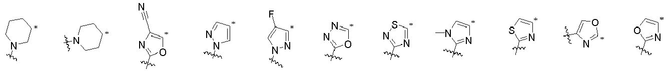 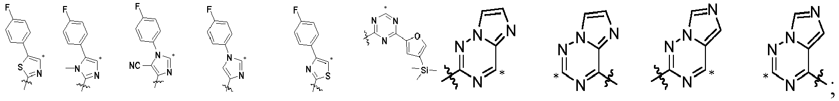
and the definitions of other groups are consistent with those of any one of the foregoing technical solutions.

As a twenty-first technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2b} is selected from 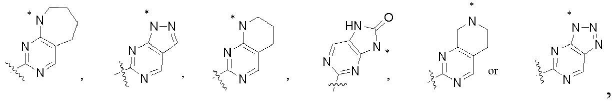 wherein * represents the coupling site of C_{y2b} with 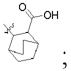
and the definitions of other groups are consistent with those of any one of the foregoing technical solutions.

As a twenty-second technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2c} is selected from 5-membered heteroaryl, 6-membered heteroaryl or C₃₋₆ cycloalkyl, and the C_{y2c} is optionally further substituted with 1-5 groups selected from R^{A}; or
C_{y2c} is selected from 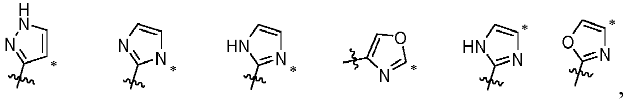 and the C_{y2c} is optionally substituted with 1-3 groups selected from R^{A}; or C_{y2c} is selected from 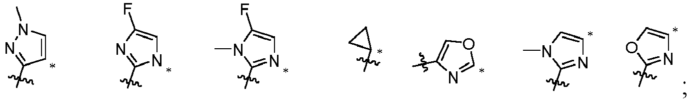
and the definitions of other groups are consistent with those of any one of the foregoing technical solutions.

As a twenty-third technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2c} is selected from 5-membered heteroaryl, 6-membered heteroaryl or C₃₋₆ cycloalkyl, and the C_{y2c} is optionally further substituted with 1-5 groups selected from R^{A};
and the definitions of other groups are consistent with those of any one of the foregoing technical solutions.

As a twenty-fourth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2c} is selected from 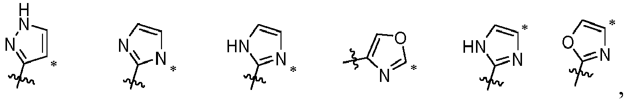 and the C_{y2c} is optionally substituted with 1-3 groups selected from R^{A};
and the definitions of other groups are consistent with those of any one of the foregoing technical solutions.

As a twenty-fifth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
C_{y2c} is selected from 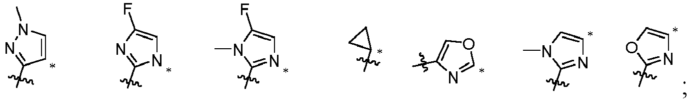
and the definitions of other groups are consistent with those of any one of the foregoing technical solutions.

As a twenty-sixth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
each R^{A} is independently selected from halogen, C₁₋₄ alkyl, 5-6-membered heteroaryl, phenyl, -NR^{B}C(O)C₁₋₄ alkyl, -C(O)NR^{B}C₁₋₄ alkyl, -C(O)C₁₋₄ alkyl, CN, =O, -C(O)C₁₋₄ alkoxy, C₂₋₆ alkenyl, C₂₋₆ alkynyl, -COOH, OH, -NR^{B}R^{C}, deuterium, -SH, -S(C₁₋₄ alkyl), -S(O)₂(C₁₋₄ alkyl) and C₃₋₆ monocyclic cycloalkyl, and the R^{A} is optionally further substituted with 1-3 groups selected from halogen, C₁₋₄ alkyl, C₁₋₄ alkoxy, halo C₁₋₄ alkyl, halo C₁₋₄ alkoxy, C₁₋₆ alkylamino, -Si(C₁₋₄ alkyl)₃, OH, 5-6-membered heteroaryl, C₃₋₆ monocyclic cycloalkyl, -S(C₁₋₄ alkyl) and deuterium; or
each R^{A} is independently selected from halogen, C₁₋₄ alkyl, 5-6-membered heteroaryl, phenyl, -NR^{B}C(O)C₁₋₄ alkyl, -C(O)NR^{B}C₁₋₄ alkyl, -C(O)C₁₋₄ alkyl, CN, =O and amino, and the R^{A} is optionally further substituted with 1-3 groups selected from halogen, C₁₋₂ alkyl and -Si(C₁₋₂ alkyl)₃;
and the definitions of other groups are consistent with those of any one of the foregoing technical solutions.

As a twenty-seventh technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
each R^{A} is independently selected from halogen, C₁₋₄ alkyl, 5-6-membered heteroaryl, phenyl, -NR^{B}C(O)C₁₋₄ alkyl, -C(O)NR^{B}C₁₋₄ alkyl, -C(O)C₁₋₄ alkyl, CN, =O and amino, and the R^{A} is optionally further substituted with 1-3 groups selected from halogen, C₁₋₂ alkyl and -Si(C₁₋₂ alkyl)₃;
and the definitions of other groups are consistent with those of any one of the foregoing technical solutions.

As a twenty-eighth technical solution of the present invention, the present invention relates to a compound of formula (I), or a stereoisomer, a tautomer, a nitrogen oxide, a solvate, a metabolite, a pharmaceutically acceptable salt, a co-crystal or a prodrug thereof, wherein
R^{B} and R^{C} are each independently selected from H, deuterium, -OC(O)CH₃ or C₁₋₃ alkyl.

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y1} is selected from

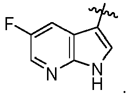

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y1} is selected from

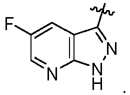

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y1} is selected from

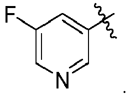

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y3} is selected from 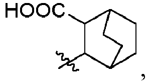 and the C_{y3} is optionally substituted with 1-3 groups selected from R_{cy3.}

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y3} is selected from 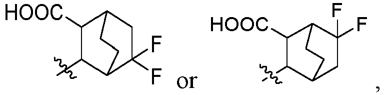 and the C_{y3} is optionally substituted with 1-3 groups selected from R_{cy3.}

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y3} is selected from 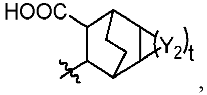 and the C_{y3} is optionally substituted with 1-3 groups selected from R_{cy3}, wherein t is selected from 1, 2 or 3, each Y₂ is independently selected from -CH₂-, -NH-, O and S, and each H atom in the Y₂ can be independently substituted with R_{cy3} group.

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y3} is selected from 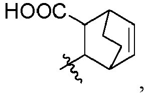 and the C_{y3} is optionally substituted with 1-3 groups selected from R_{cy3.}

According to the compound, or the stereoisomer, the tautomer, the nitrogen oxide, the solvate, the metabolite, the pharmaceutically acceptable salt, the co-crystal or the prodrug thereof described in the technical solutions hereinbefore, C_{y2} is selected from C₈₋₁₂ carbocycle or 8-12-membered heterocycle, and the C_{y2} is optionally further substituted with 1-3 groups selected from R^{A}; further, C_{y2} is selected from C₈₋₁₀ carbocycle or 8-10-membered heterocycle, and the C_{y2} is optionally further substituted with 1-2 groups selected from R^{A}; further, C_{y2} is selected from 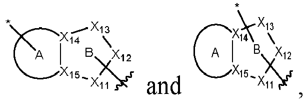 and is optionally further substituted with 1-5 groups selected from R^{A}; further, C_{y2} is selected from 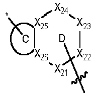 and 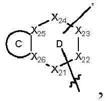 and is optionally further substituted with 1-5 groups selected from R^{A}, provided that D-fused ring C does not form substituted or unsubstituted 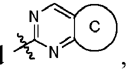 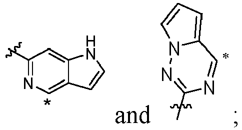 further, C_{y2} is selected from 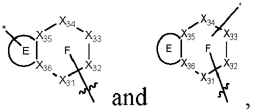 and is optionally further substituted with 1-5 groups selected from R^{A}, provided that F-fused ring E does not form substituted or unsubstituted 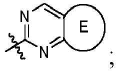 further, C_{y2} is selected from 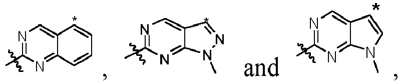 and is optionally further substituted with 1-5 groups selected from R^{A}; further, C_{y2} is selected from 5-membered heteroaryl, 5-membered unsaturated monocyclic heterocycloalkyl and 6-membered saturated monocyclic heterocycloalkyl, and is optionally further substituted with 1-5 groups selected from R^{A}; further, C_{y2} is selected from 6-membered unsaturated monocyclic heterocycloalkyl, and is optionally further substituted with 1-5 groups selected from R^{A}, provided that C_{y2} is not selected from 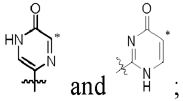 further, C_{y2} is selected from 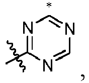 and is substituted with 1 group selected from R^{D}; and further, C_{y2} is selected from 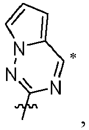 and is substituted with 1-3 groups selected from R^{E}, wherein the above * represents the coupling site of C_{y2} with L₂.

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 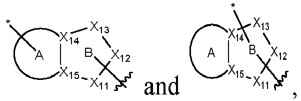 wherein * represents the coupling site of C_{y2a} with NH, and C_{y2a} is optionally further substituted with 1-3 groups selected from RA.

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 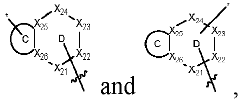 wherein * represents the coupling site of C_{y2a} with NH, and C_{y2a} is optionally further substituted with 1-3 groups selected from R^{A}, wherein X₂₁, X₂₂, X₂₃ and X₂₄ are each independently selected from -CH-, -CR^{A}-, -CH₂-, -CHR^{A}-, -C(R^{A})₂-, -N-, -NH-, -NR^{A}-, O, S, S(O) or S(O)₂, X₂₅ and X₂₆ are each independently selected from -C-, -CH-, -CR^{A}- or -N-, and ring D is a saturated ring, a partially unsaturated ring, a benzene ring or a heteroaryl ring, provided that D-fused ring C does not form substituted or unsubstituted 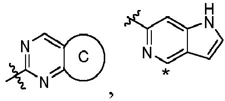 and

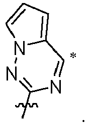

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 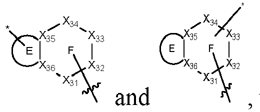 wherein * represents the coupling site of C_{y2a} with NH, and C_{y2a} is optionally further substituted with 1-3 groups selected from R^{A}, wherein X₃₁, X₃₂, X₃₃ and X₃₄ are each independently selected from -CH-, -CR^{A}-, -CH₂-, -CHR^{A}-, -C(R^{A})₂-, -N-, -NH-, -NR^{A}-, O, S, S(O) or S(O)₂, X₃₅ and X₃₆ are each independently selected from -C-, -CH-, -CR^{A}- or -N-, and ring F is a saturated ring, a partially unsaturated ring, a benzene ring or a heteroaryl ring, provided that F-fused ring E does not form substituted or unsubstituted

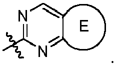

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 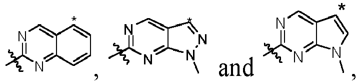 wherein * represents the coupling site of C_{y2a} with NH, and C_{y2a} is optionally further substituted with 1-3 groups selected from RA.

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 5-membered heteroaryl, 5-membered unsaturated monocyclic heterocycloalkyl and 6-membered saturated monocyclic heterocycloalkyl, and is optionally further substituted with 1-3 groups selected from RA.

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 6-membered unsaturated monocyclic heterocycloalkyl, and is optionally further substituted with 1-3 groups selected from R^{A}, provided that C_{y2a} is not selected from substituted or unsubstituted 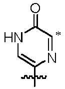 and substituted or unsubstituted

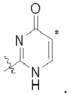

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 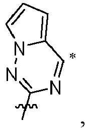 wherein * represents the coupling site of C_{y2a} with NH, provided that when C_{y1} is selected from 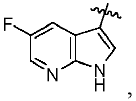 C_{y2a} is substituted with 1-3 groups selected from R^{E}; or when C_{y1} is selected from 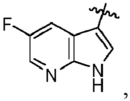 and C_{y2a} is (h), C_{y3} is substituted with 1-5 groups selected from R_{cy3}; or when C_{y1} is selected from 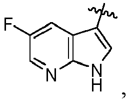 and C_{y2a} is (h), C_{y3} is selected from 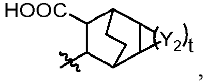 and t = 2, 3 or 4; or when C_{y1} is selected from 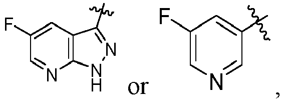 C_{y2a} is optionally further substituted with 1-3 groups selected from RA.

According to the compound of formula (II), or the stereoisomer, the tautomer, the nitrogen oxide, the solvate, the metabolite, the pharmaceutically acceptable salt, the co-crystal or the prodrug thereof described hereinbefore, C_{y2a} is selected from 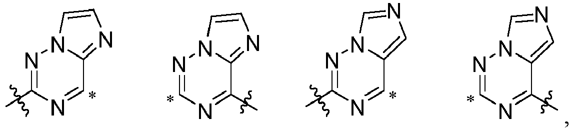 wherein * represents the coupling site of C_{y2a} with NH, and C_{y2a} is optionally further substituted with 1-3 groups selected from RA.

According to the compound of formula (II), or the stereoisomer, the tautomer, the nitrogen oxide, the solvate, the metabolite, the pharmaceutically acceptable salt, the co-crystal or the prodrug thereof described hereinbefore, each R^{A} is independently selected from halogen, C₁₋₄ alkyl, C₁₋₄ alkoxy, C₂₋₄ alkenyl, C₂₋₄ alkynyl, 5-10-membered heteroaryl, phenyl, -NR^{B}C(O)C₁₋₄ alkyl, -C(O)NR^{B}C₁₋₄ alkyl, -C(O)C₁₋₄ alkyl, CN, =O, -C(O)C₁₋₄ alkoxy, -COOH, OH, -NR^{B}R^{C}, deuterium, -SH, -S(C₁₋₄ alkyl), -S(O)₂(C₁₋₄ alkyl) and C₃₋₆ monocyclic cycloalkyl, and the R^{A} is optionally further substituted with 1-3 groups selected from halogen, C₁₋₃ alkyl, C₁₋₃ alkoxy, halo C₁₋₃ alkyl, halo C₁₋₃ alkoxy, -Si(C₁₋₃ alkyl)₃, OH, 5-6-membered heteroaryl, C₃₋₆ monocyclic cycloalkyl, -S(C₁₋₃ alkyl) and deuterium; and further, each R^{A} is independently selected from halogen, C₁₋₂ alkyl, 5-10-membered heteroaryl, -NR^{B}C(O)C₁₋₄ alkyl, -NR^{B}C(O)C₁₋₄ alkyl, CN and phenyl, and the R^{A} is optionally further substituted with 1-2 groups selected from halogen, C₁₋₂ alkyl, C₁₋₂ alkoxy, C₂₋₄ alkenyl, C₂₋₄ alkynyl, C₃₋₆ monocyclic cycloalkyl, OH, =O, -C(O)C₁₋₂ alkyl and C₁₋₃ alkoxy;
R^{B} and R^{C} are each independently selected from H, deuterium, C₁₋₂ alkyl, halo C₁₋₂ alkyl and deuterated C₁₋₂ alkyl; further, R^{B} and R^{C} are each independently selected from H, deuterium and C₁₋₂ alkyl; and further, R^{B} and R^{C} are each independently selected from H and deuterium.

According to the compound of formula (II), or the stereoisomer, the tautomer, the nitrogen oxide, the solvate, the metabolite, the pharmaceutically acceptable salt, the co-crystal or the prodrug thereof described hereinbefore, each R^{E} is selected from hydroxy C₁₋₄ alkyl, halo C₁₋₄ alkyl, C₁₋₄ alkoxy, C₁₋₄ alkoxy-C₁₋₄ alkyl, C₂₋₄ alkenyl, C₂₋₄ alkynyl, 5-6-membered heteroaryl, phenyl, -NR^{B}C(O)C₁₋₄ alkyl, -C(O)NR^{B}C₁₋₄ alkyl, -C(O)C₁₋₄ alkyl, =O, -C(O)C₁₋₄ alkoxy, -COOH, OH, -NR^{B}R^{C}, deuterium, - SH, -S(C₁₋₄ alkyl), -S(O)₂(C₁₋₄ alkyl), C₃₋₆ monocyclic cycloalkyl and C₃₋₆ monocyclic heterocyclic cycloalkyl, and the R^{E} is optionally further substituted with 1-3 groups selected from halogen, C₁₋₄ alkyl, C₁₋₄ alkoxy, halo C₁₋₄ alkyl, C₁₋₆ alkylamino, halo C₁₋₄ alkoxy, -Si(C₁₋₄ alkyl)₃, OH, 5-6-membered heteroaryl, C₃₋₆ monocyclic cycloalkyl, -S(C₁₋₄ alkyl) and deuterium; and further, R^{E} is selected from hydroxy C₁₋₄ alkyl, C₂₋₄ alkenyl, C₂₋₄ alkynyl, C₁₋₄ alkoxy-C₁₋₄ alkyl, 5-6-membered heteroaryl, -NR^{B}C(O)C₁₋₄ alkyl, -C(O)NR^{B}C₁₋₄ alkyl, -C(O)C₁₋₄ alkyl and -C(O)C₁₋₄ alkoxy, and the R^{E} is optionally further substituted with 1-3 groups selected from halogen and C₁₋₂ alkyl.

According to the compound of formula (I), or the stereoisomer, the tautomer, the nitrogen oxide, the solvate, the metabolite, the pharmaceutically acceptable salt, the co-crystal or the prodrug thereof of the present invention, the compound is selected from one of the following structures:

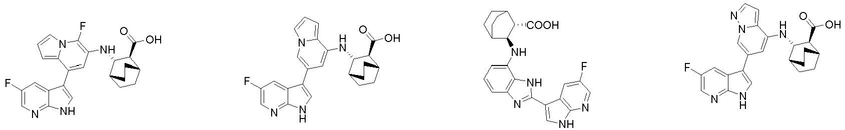

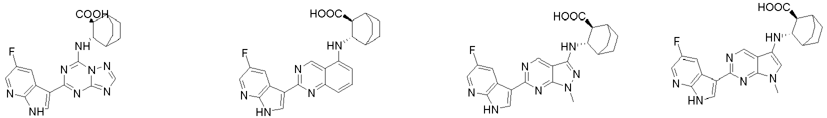

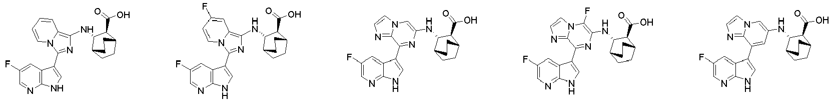

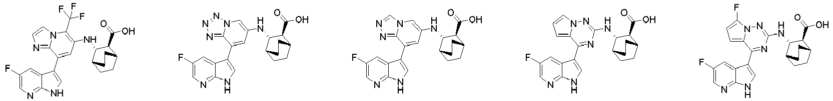

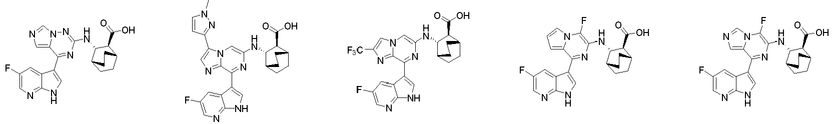

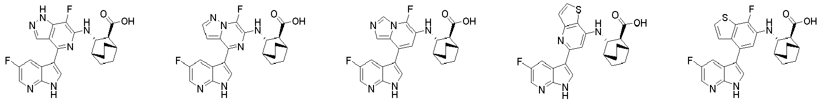

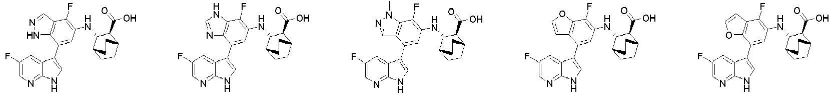

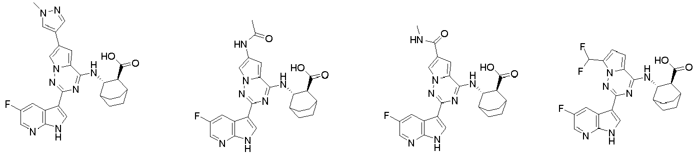

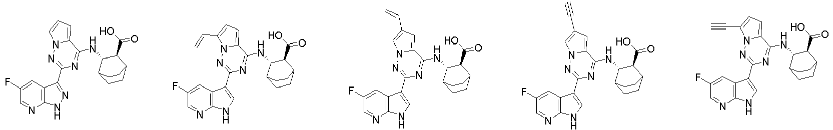

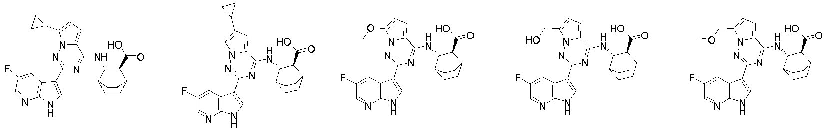

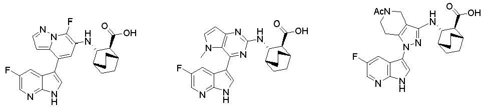

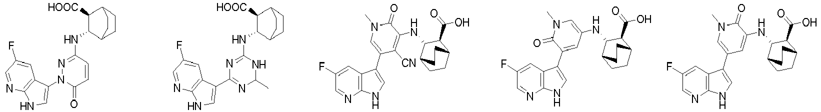

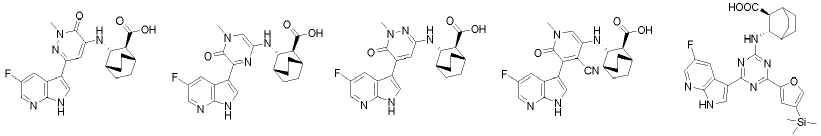

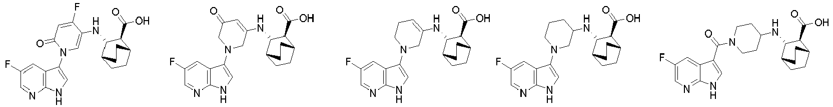

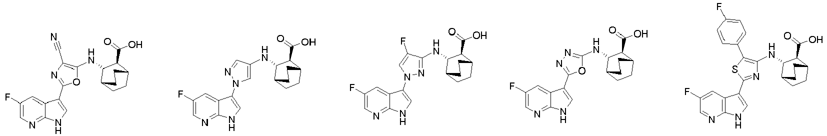

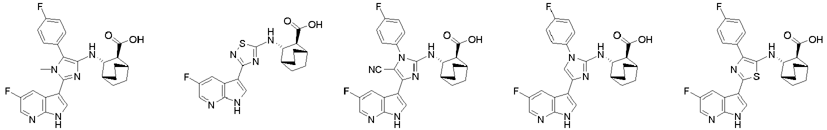

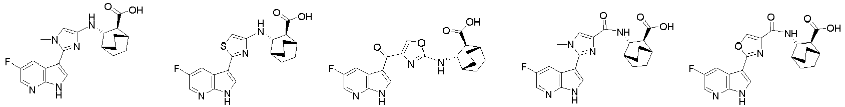

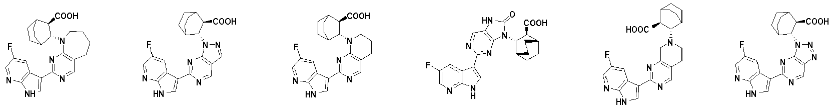

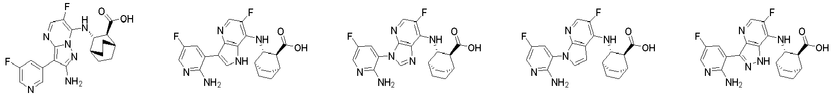

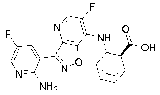

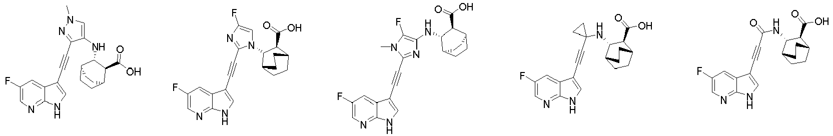

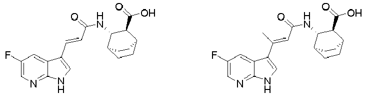

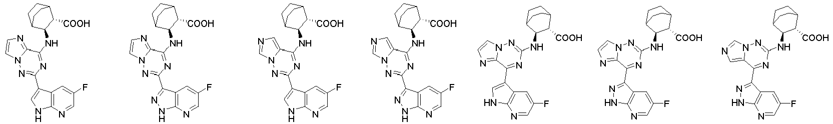

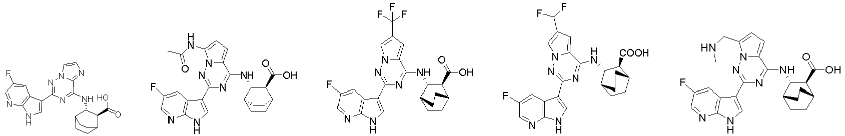

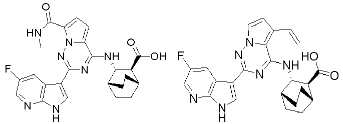

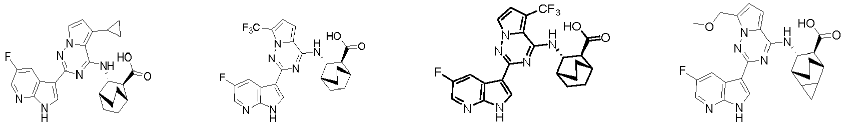

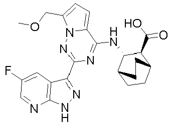

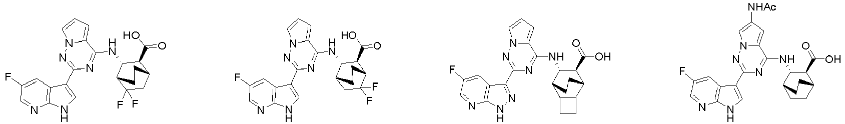

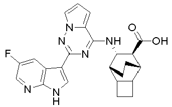

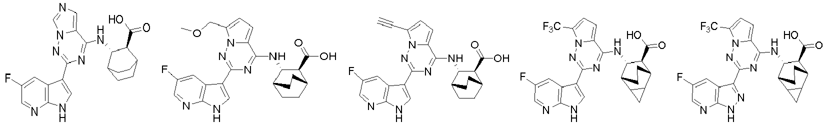

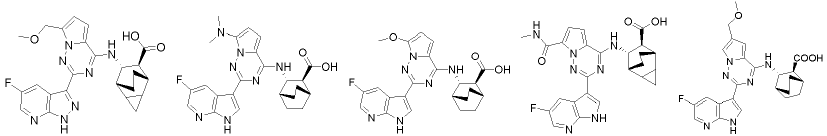

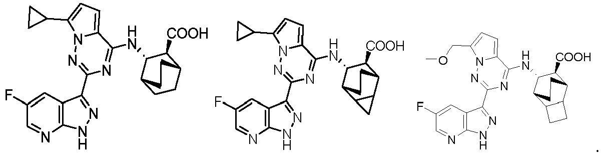

As another technical solution of the present invention, the present invention provides a pharmaceutical composition containing the compound, or the stereoisomer, the pharmaceutically acceptable salt, the solvate, the co-crystal or the deuterated product thereof according to any one of the above-mentioned technical solutions, and a pharmaceutically acceptable carrier and/or excipient.

As yet another technical solution of the present invention, the present invention provides the use of the compound, or the stereoisomer, the pharmaceutically acceptable salt, the solvate, the co-crystal or the deuterated product thereof according to any one of the technical solutions, or the above-mentioned composition in the preparation of a drug for treating a PB2-mediated disease. The PB2-mediated disease is a tumour or an autoimmune disease.

### Synthetic Route

Patent document WO 2010148197 A1 introduces a method for preparing a class of PB2 inhibitors, and those skilled in the art would have been able to prepare the compounds of the present invention by means of combining the document and known organic synthesis techniques, wherein the starting materials used therein are commercially available chemicals and (or) compounds described in chemical documents.

### Synthetic method I:

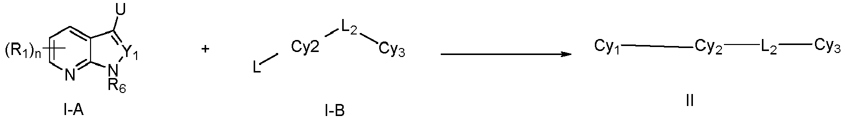

a compound of general formula (II) can be obtained by a coupling reaction of a compound of general formula I-A with a compound of general formula I-B under the condition of a suitable catalyst, base and solvent, wherein U and L represent the same or different leaving groups, such as 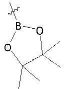 and halogen, and wherein C_{y1} is

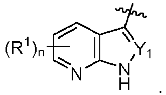

### Term

Unless otherwise specified, the terms of the present invention have the following meanings.

The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise ¹²C, ¹³C and ¹⁴C; the isotopes of hydrogen comprise protium (H), deuterium (D, also known as heavy hydrogen), and tritium (T, also known as superheavy hydrogen); the isotopes of oxygen comprise ¹⁶O, ¹⁷O and ¹⁸O; the isotopes of sulphur comprise ³²S, ³³S, ³⁴S and ³⁶S; the isotopes of nitrogen comprise ¹⁴N and ¹⁵N; the isotope of fluorine comprises ¹⁹F; the isotopes of chlorine comprise ³⁵Cl and ³⁷Cl; and the isotopes of bromine comprise ⁷⁹Br and ⁸¹Br.

The expression "C_{x-y} group" refers to a group comprising x to y carbon atoms, for example, "C₁₋₆ alkyl" refers to an alkyl group comprising 1-6 carbon atoms.

The term "halogen" refers to fluorine (F), chlorine (Cl), bromine (Br), iodine (I) or isotopes thereof.

The term "halo" or "substituted with halogen" means that the hydrogen atoms are substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen.

The term "halo C₁₋₆ alkyl" refers to an alkyl group comprising 1-6 carbon atoms in which one or more hydrogens are substituted with one or more halogen atoms (e.g., fluorine, chlorine, bromine, and iodine), wherein the upper limit of the number of halogen substituents is equal to the sum of the number of hydrogens that can be substituted in the alkyl group. Without particular limitation, the number of halogen substituents is any integer between 1 and the upper limit, preferably 1-5 halogen, 1-3 halogen, 1-2 halogen, or 1 halogen; and when the number of halogen substituents is greater than 1, the alkyl group can be substituted with the same or different halogen. Examples include, but are not limited to -CF₃, -CH₂Cl, -CH₂CF₃, -CCl₂, CF₃, etc.

The term "deuterium" refers to the isotope deuterium of hydrogen (H).

The term "deuterated" or "deuterated product" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, 1-10 deuterium atoms, 1-6 deuterium atoms, 1-3 deuterium atoms, 1-2 deuterium atoms or 1 deuterium atom.

The term "alkyl" refers to a straight or branched saturated aliphatic hydrocarbon group. Unless otherwise specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples of alkyl include methyl, ethyl, n-propyl, isopropyl, n-butyl, sec-butyl, neobutyl, tert-butyl, n-pentyl, isoamyl, neopentyl, n-hexyl, etc. The alkyl can be further substituted with any substituent.

The term "alkenyl" refers to a straight or branched hydrocarbon group comprising at least one carbon-carbon double bond (C=C). Unless otherwise specified, the main chain comprises 2 to 18 (such as 2 to 8, further such as 2 to 6, and more further such as 2 to 4) carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The alkenyl can be optionally further substituted with any group.

The term "alkynyl" refers to a straight or branched hydrocarbon group containing at least one carbon-carbon triple bond (C=C). The main chain comprises 2 to 18 (such as 2 to 8, further such as 2 to 6, and more further such as 2 to 4) carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc. The alkynyl can be optionally further substituted with any substituent.

The term "alkoxy" or "alkyloxy" refers to -O-alkyl. Without particular limitation, alkoxy or alkyloxy is -O-C₁₋₈ alkyl, preferably -O-C₁₋₆ alkyl, more preferably -O-C₁₋₄ alkyl, and further preferably -O-C₁₋₂ alkyl. Non-limiting examples of alkoxy or alkyloxy include methoxy, ethoxy, n-propoxy, isopropoxy, n-butoxy, secbutoxy, tert-butoxy, n-pentoxy, n-hexyloxy, cyclopropoxy, cyclobutoxy, etc. The alkoxy can be optionally further substituted with any substituent.

The term "haloalkoxy" refers to -O-haloalkyl. Without particular limitation, haloalkoxy is -O-halo C₁₋₈ alkyl, preferably -O-halo C₁₋₆ alkyl, more preferably -O-halo C₁₋₄ alkyl, and further preferably -O-halo C₁₋₂ alkyl, wherein the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, etc.

The term "C₁₋₆ alkylacyl" refers to C₁₋₆ alkyl-C(O)-. Non-limiting examples of C₁₋₆ alkylacyl include formyl, acetyl and propionyl.

The term "C₁₋₆ alkylsulfonyl" refers to C₁₋₆ alkyl-S(O)₂-. Non-limiting examples of C₁₋₆ alkylsulfonyl include methylsulfonyl, ethylsulfonyl and propylsulfonyl.

The term "carbocycle" or "carbocyclyl" refers to a substituted or unsubstituted, saturated, partially unsaturated or fully unsaturated, non-aromatic or aromatic hydrocarbon ring. Unless otherwise specified, carbocycle or carbocyclyl refers to a hydrocarbon ring comprising 3-15 carbon atoms (C₃₋₁₅ carbocycle, further such as C₃₋₁₀ carbocycle, more further such as C₃₋₈ carbocycle, and more further such as C₃₋₆ carbocycle), and can comprise any stable 3-, 4-, 5-, 6-, 7- or 8-membered monocyclic or bicyclic or 7-, 8-, 9-, 10-, 11-, 12-, 13-, 14- or 15-membered bicyclic or polycyclic hydrocarbon ring, wherein the bicyclic or tricyclic hydrocarbon ring comprises a spiro ring, a bridged ring and a fused ring; and the definition thereof comprises cycloalkyl and aryl. Non-limiting examples of carbocycle or carbocyclyl include cyclopropyl, cyclobutyl, cyclobutenyl, cyclopentyl, cyclopentenyl, cyclohexyl, cyclohexenyl, cycloheptyl, cycloheptenyl, adamantyl, cyclooctyl, cyclooctenyl, cyclooctadienyl, phenyl, naphthyl, indanyl, anthryl, 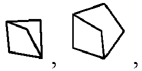 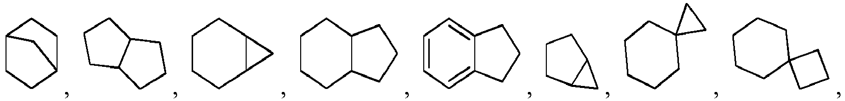 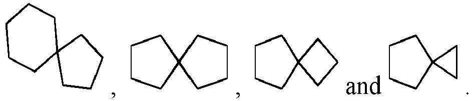 The carbocycle can be optionally further substituted with any group.

The term "heterocycle" or "heterocyclyl" refers to a substituted or unsubstituted, saturated, partially unsaturated or fully unsaturated, non-aromatic or aromatic ring containing at least one heteroatom, wherein the heteroatom is selected from N, P, O, S and Si and respective oxidation state thereof. Unless otherwise specified, heterocycle or heterocyclyl refers to a ring comprising 3-15 ring atoms (3-15-membered heterocycle, further such as 3-10-membered heterocycle, and more further such as 3-8-membered heterocycle), and can comprise any stable 3-, 4-, 5-, 6-, 7- or 8-membered monocyclic or bicyclic or 7-, 8-, 9-, 10-, 11-, 12-, 13-, 14- or 15-membered bicyclic or polycyclic ring, wherein the bicyclic or tricyclic ring comprises a spiro ring, a bridged ring and a fused ring. When heterocycle is a bicyclic or polycyclic ring, at least one ring contains at least one heteroatom, and the heterocycle can be a bicyclic or polycyclic ring formed by a ring containing the heteroatom(s) and a ring containing no heteroatom. When heterocycle is connected to other groups, a connection point can be at a heteroatom or a carbon atom. Heterocycle includes heterocycloalkyl and heteroaryl. Non-limiting examples of heterocycle include epoxyethyl, azacyclopropyl, oxetanyl, azetidinyl, 1,3-dioxolanyl, 1,4-dioxolanyl, 1,3-dioxanyl, azacycloheptyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, dihydroxazolyl, tetrahydrooxazolyl, tetrahydrothiazolyl, tetrahydropyranyl, furyl, thienyl, pyrrolyl, oxazolyl, thiazolyl, imidazolyl, pyrazolyl, pyridyl, pyrimidinyl, pyranyl, pyridazinyl, pyrazinyl, indolyl, quinolyl, isoquinolinyl, purinyl, benzofuryl, benzothienyl, benzopyrrolyl, benzopyranyl, benzoimidazolyl, benzopyridyl, pyrrolopyridinyl, benzodihydrofuryl, azabicyclo[3.2.1]octanyl, azabicyclo[5.2.0]nonanyl, oxatricyclo[5.3.1.1]dodecyl, azaadamantyl and oxaspiro[3 .3]heptanyl, 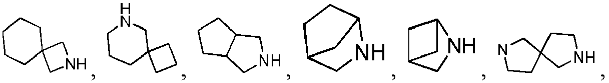 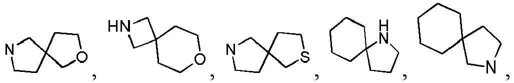 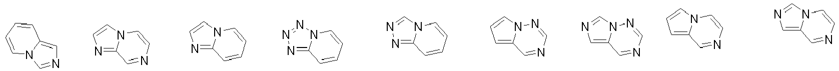 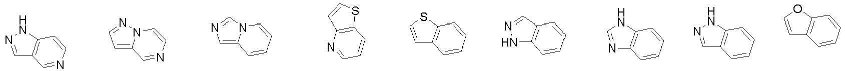 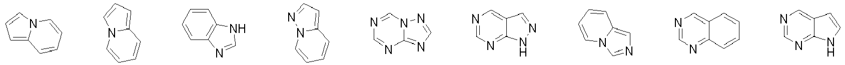 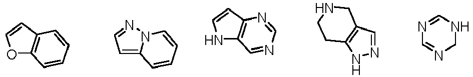 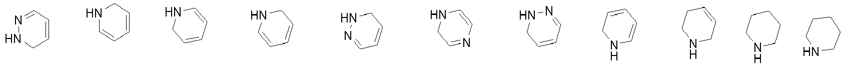 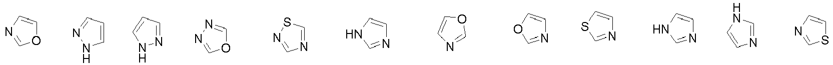 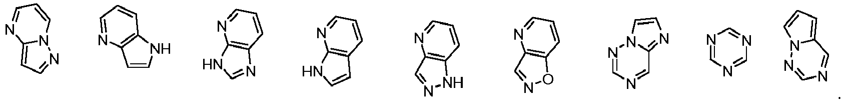 The heterocycle can be optionally further substituted with any group.

The term "cycloalkyl" refers to a substituted or unsubstituted, saturated, partially unsaturated or fully unsaturated non-aromatic hydrocarbon ring. Cycloalkyl can be a monocyclic, bicyclic or polycyclic ring, wherein the bicyclic or polycyclic ring can be a fused ring, a spiro ring or a bridged ring. Unless otherwise specified, cycloalkyl usually contains 3 to 20 carbon atoms. When cycloalkyl is monocyclic cycloalkyl, the cycloalkyl contains preferably 3-15 carbon atoms, preferably 3-10 carbon atoms, also preferably 3-8 carbon atoms, more preferably 3-6 carbon atoms, and further preferably 3-4 carbon atoms; and when cycloalkyl is bicyclic or polycyclic cycloalkyl, the cycloalkyl contains preferably 4-12 carbon atoms, preferably 4-11 carbon atoms, also preferably 5-11 carbon atoms, more preferably 6-11 carbon atoms, and further preferably 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl, 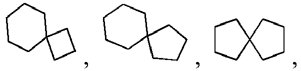 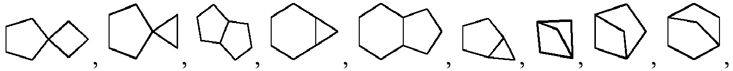 etc.

The term "heterocycloalkyl" refers to a substituted or unsubstituted, saturated, partially unsaturated or fully unsaturated non-aromatic ring containing at least one heteroatom. Unless otherwise specified, heterocycloalkyl is a 3- to 20-membered ring. When heterocycloalkyl is monocyclic heterocycloalkyl, the heterocycloalkyl is preferably a 3- to 15-membered, preferably 3-10-membered, also preferably 3-8-membered, and further preferably 3-6-membered ring; and when heterocycloalkyl is bicyclic or polycyclic heterocycloalkyl, the heterocycloalkyl is preferably a 4-12-membered, preferably 4-11-membered, also preferably 5-11-membered, more preferably 6-11-membered, and further preferably 6-10-membered ring. Heterocycloalkyl can be a monocyclic, bicyclic or polycyclic ring, wherein the bicyclic or polycyclic ring can be a bridged ring, a fused ring and a spiro ring, in which the heteroatoms are selected from heteroatoms N, S, O, P and Si and oxidation states thereof. When heterocycloalkyl is a bicyclic or polycyclic ring, at least one ring contains at least one heteroatom, and the heterocycloalkyl can be a bicyclic or polycyclic ring formed by a ring containing the heteroatom(s) and a ring containing no heteroatom. When heterocycloalkyl is connected to other groups, a connection point can be at a heteroatom or a carbon atom. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc.

The term "aryl" refers to a substituted or unsubstituted aromatic 5- to 15-membered carbocycle, and includes monocyclic aryl and fused aryl. Aryl is preferably a 5- to 10-membered aromatic ring, and further preferably a 5- to 8-membered aromatic ring. Aryl ring can be fused to a non-aryl ring (such as a heteroaryl, heterocycloalkyl or cycloalkyl ring), wherein the aryl ring is the connection site, and non-limiting examples thereof comprise phenyl, naphthyl, anthryl, phenanthryl, 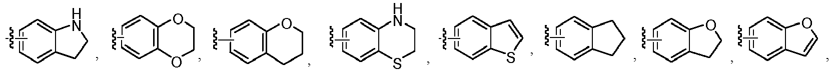 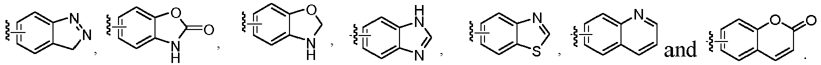 The aryl can be optionally further substituted with any substituent.

The term "heteroaryl ring" or "heteroaryl" refers to a substituted or unsubstituted aromatic ring containing at least one heteroatom or group selected from heteroatoms N, S, O, P and Si and oxidation states thereof. Heteroaryl ring or heteroaryl can be a monocyclic, bicyclic or polycyclic ring, wherein the bicyclic or polycyclic ring can be a bridged ring, a fused ring and a spiro ring. Bicyclic or polycyclic heteroaryl ring or heteroaryl can be formed by fusion of heteroaryl to a non-heteroaryl ring such as cycloalkyl, heterocycloalkyl and aryl, or of heteroaryl to heteroaryl, wherein the heteroaryl ring is the connection site. Non-limiting examples of heteroaryl ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl, 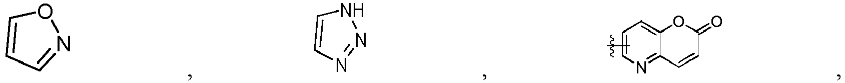 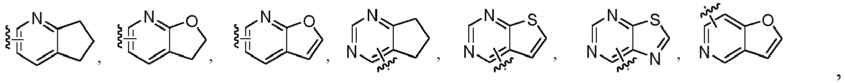 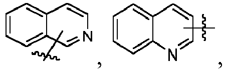 etc. The heteroaryl can be optionally further substituted with any substituent.

The term "carboxyl" refers to -C(=O)-OH.

The term "optional" or "optionally" refers to that the event or circumstance subsequently described may but not necessarily occur, and the description includes the occasions where the events or circumstances occur or do not occur. For example, "alkyl optionally substituted with F" means that an alkyl group may but not necessarily be substituted with F, and the description includes the case where the alkyl group is substituted with F and the case where the alkyl group is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, the solvates, the pharmaceutically acceptable salts, the co-crystals or the deuterated products thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

### Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the scope of protection of the present invention.

Unless otherwise specified, the raw materials are purchased from Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The structures of the compounds are determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvent for determination is deuterated dimethyl sulfoxide (DMSO-d6), deuterated chloroform (CDCl₃) and deuterated methanol (CD₃OD); and the internal standard is tetramethylsilane (TMS).

MS is determined with Agilent 6120B (ESI) and Agilent 6120B (APCI).

HPLC is determined with Agilent 1260DAD high pressure liquid chromatography (Zorbax SB-C18 100 × 4.6 mm, 3.5 µM).

Yantai Huanghai HSGF254 or Qingdao GF254 silica gel plate is used as a thin layer chromatography silica gel plate; the specification of the silica gel plate used for thin layer chromatography (TLC) is 0.15 mm-0.20 mm; and the specification when separating and purifying a product by thin layer chromatography is 0.4 mm - 0.5 mm.

For the column chromatography, Yantai Huanghai silica gel of 200-300 mesh silica gel is generally used as a carrier.
THF: Tetrahydrofuran
NBS: N-bromosuccinimide
DCM: Dichloromethane
Pd(dppf)Cl₂ [1,1'-bis(diphenylphosphino)ferrocene]palladium dichloride
TFA: Trifluoroacetic acid
DIPEA: N,N-diisopropylethylamine
PE: Petroleum ether
EA: Ethyl acetate
DMF: N,N dimethylformamide
DIBAL-H: Diisobutylaluminium hydride
DAST: Diethylaminosulphur trifluoride
NMP: N-methylpyrrolidone
HATU: 2-(7-azobenzotriazole)-N,N,N' ,N' -tetramethyluronium hexafluorophosphate
X-phos: 2-dicyclohexylphosphino-2,4,6-triisopropylbiphenyl
DCE: 1,2 dichloroethane
NIS: N-iodosuccinimide
DPPA: Diphenylphosphoryl azide
DIEA: N,N-diisopropylethylamine

### Intermediate 1: 5-fluoro-3-(4,4,5,5-tetramethyl-1,3,2-dioxaborolan-2-yl)-1-tosyl-1H-pyrrolo[2,3-b]pyridine (intermediate int-1)

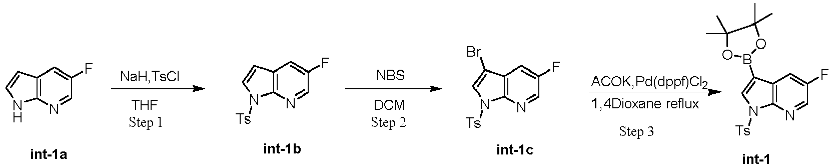

### Step 1: 5-fluoro-1-tosyl-1H-pyrrolo[2,3-b]pyridine (int-1b)

Under nitrogen protection, compound **int-1a** (5.0 g, 36.7 mmol) was dissolved in THF (50 ml), and the mixture was cooled to 0-5°C. Sodium hydride (1.76 g, 44.0 mmol) was slowly added and after the addition, the mixture was stirred for 30 min at 0°C. P-toluenesulfonyl chloride (7.3 g, 38.5 mmol) was dissolved in THF (10 ml), and slowly added dropwise to the reaction solution while the temperature was controlled at 0-10°C. After the addition, the mixture was naturally warmed to room temperature and reacted for 15 hours. After completion of the reaction, ice water (100 ml) was slowly added to quench the reaction. The mixture was extracted with ethyl acetate (100 ml*2), washed with saturated brine, dried over anhydrous sodium sulphate, filtered and concentrated, and the residue was recrystallized with ethyl acetate/petroleum ether (1/6, v/v) to obtain compound **int-1b** (8.5 g, 80%).

LC-MS (ESI): m/z =290.1 [M+H]⁺.

### Step 2:

### 3-bromo-5-fluoro-1-tosyl-1H-pyrrolo[2,3-b]pyridine (int-1c)

Compound **int-1b** (8.5 g, 29.4 mmol) was dissolved in dichloromethane (85 ml). NBS (10.5 g, 58.8 mmol) was added in portions and after the addition, the mixture was reacted at room temperature for 18 hours. An aqueous sodium thiosulphate solution was added to quench the reaction, and the mixture was extracted twice with DCM (100 ml*2). The organic layers were combined, washed with water and brine, dried and concentrated. The residue was purified by silica gel column (PE/EA = 10/1-5/1, v/v) to obtain compound **int-1c** (6.5 g, 60%).

LC-MS (ESI): m/z =369.1 [M+H]⁺.

### Step 3:

### 5-fluoro-3-(4,4,5,5-tetramethyl-1,3,2-dioxaborolan-2-yl)-1-tosyl-1H-pyrrolo[2,3-b]pyridine (intermediate int-1)

Under nitrogen protection, compound **int-1c** (6.5 g, 17.6 mmol) was dissolved in dioxane (65 ml). Potassium acetate (5.17 g, 52.8 mmol), bis(pinacolato)diboron (6.7 g, 26.4 mmol), and Pd(dppf)Cl₂ (1.2 g, 1.7 mmol) were successively added and after the addition, the mixture was warmed to 100°C and reacted for 16 hours. After completion of the reaction, the mixture was filtered, and the filtrate was concentrated. Water (150 ml) was added, and the mixture was extracted twice with ethyl acetate (100 ml*2). The organic layers were combined, washed with water and brine, dried and concentrated. The residue was purified by a column with dichloromethane/petroleum ether (0/1-3/1, v/v) to obtain intermediate **int-1** (1.5 g, 20%).

LC-MS (ESI): m/z =417.2[M+H]⁺.

¹H NMR (400 MHz, CDCl₃) δ 8.26-8.25 (m, 1H),8.17 (s, 1H), 8.16 (d, 2H), 7.88-7.86 (m, 1H), 7.27 (d, 2H), 2.37(s, 3H), 1.35(s, 12H).

### Intermediate 2:

### 5-fluoro-3-(4,4,5,5-tetramethyl-1,3,2-dioxaborolan-2-yl)-1-trityl-1H-pyrazolo[3,4-b]pyridine (Int-2)

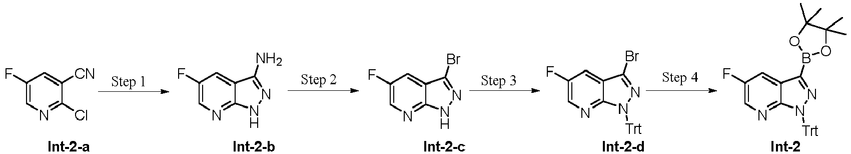

### Step 1:

### 5-fluoro-1H-pyrazolo[3,4-b]pyridin-3-amine (Int-2-b)

At room temperature, compound **Int-2-a** (45 g, 0.29 mol) was added to a mixed solution of n-butanol (300 mL) and hydrazine hydrate (200 mL), and the mixture was reacted at 100°C for 5 hours. The reaction solution was then concentrated to about half of the volume remaining and filtered. The resulting solid was rinsed with isopropanol (200 mL) and the solid was dried to obtain compound **Int-2-b** (40 g, yield: 91%).

LC-MS (ESI): m/z =153.1 [M+H]⁺.

### Step 2:

### 3-bromo-5-fluoro-1H-pyrazolo[3,4-b]pyridine (Int-2-c)

At room temperature, compound **Int-2-b** (40 g, 0.26 mol) was added to tribromomethane (250 mL), and tert-butyl nitrite (54 g, 0.52 mol) was added dropwise to the mixture at room temperature. The mixture was then warmed to 60°C and reacted for 2 hours, and warmed to 90°C and reacted for 2 hours. The obtained reaction solution was poured into ice water (1 L) and extracted with dichloromethane (500 mL × 1). The organic phase was concentrated to dryness. The obtained solid was slurried with PE/EA = 3/1 (v/v, 200 mL) and filtered to obtain compound **Int-2-c** (38 g, yield: 65%).

LC-MS (ESI): m/z =215.0 [M+H]⁺.

### Step 3:

### 3-bromo-5-fluoro-1-trityl-1H-pyrazolo[3,4-b]pyridine (Int-2-d)

At room temperature, compound **Int-2-c** (15 g, 69.7 mmol) was added to DMF (100 mL), and the mixture was cooled to 0°C. Potassium carbonate (24 g, 174 mmol) was added, and the mixture was stirred and reacted for 20 minutes. Triphenylmethyl chloride (25 g, 89.7 mmol) was added, and the mixture was naturally warmed to room temperature and reacted for 16 hours. The reaction solution was poured into water (500 mL) and extracted with EA (300 mL × 1). The organic phase was concentrated to dryness, and the residue was purified by column chromatography (PE/EA, 50/1-10/1, v/v) to obtain compound **Int-2-d** (10 g, yield: 31%).

### Step 4:

### 5-fluoro-3-(4,4,5,5-tetramethyl-15-tetramethyl-1,3,2-dioxaborolan-2-yl)-1-trityl-1H-pyrazolo[3,4-b]pyridine (Int-2)

At room temperature, compound **Int-2-d** (1 g, 2.2 mmol) was added to ethylene glycol dimethyl ether (10 mL), and bis(pinacolato)diboron (1.1 g, 4.4 mmol), potassium acetate (0.65 g, 6.6 mmol) and Pd(dppf)Cl₂ (150 mg, 0.2 mmol) were successively added to the mixture. The mixture was subjected to nitrogen replacement and protection, and was reacted under microwave at 95°C for 1 hour. The reaction solution was poured into water (40 mL) and extracted with ethyl acetate (40 mL × 1). The organic phase was concentrated to dryness to obtain crude compound **Int-2** (1.2 g), which was directly used in the next step.

### Example 1 (1R,2S,3S,4R)-3-((E)-3-(5-fluoro-1H-pyrrolo[2,3-b]pyridin-3-yl)acrylamido)bicyclo[2.2.2]octane-2-carboxylic acid (compound 1)

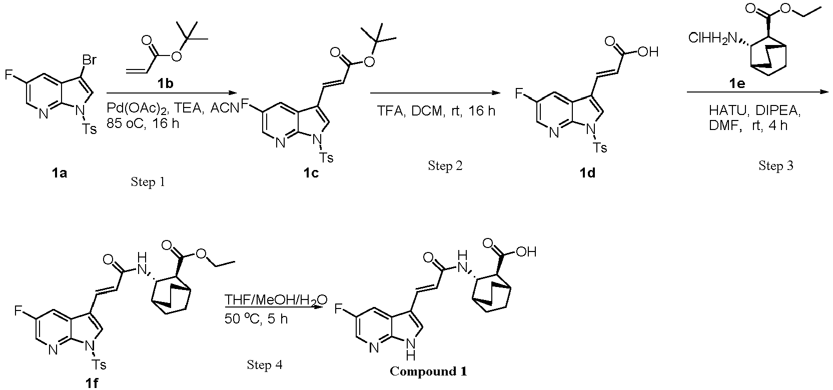

Step 1:

### tert-butyl (E)-3-(5-fluoro-1-tosyl-1H-pyrrolo[2,3-b]pyridin-3-yl)acrylate (1c)

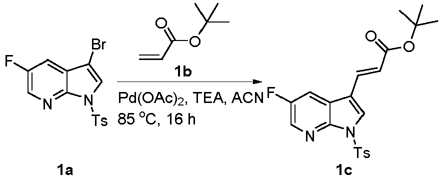

To a solution of compound **1a** (2.0 g, 5.43 mmol) in acetonitrile (20 ml) were added compound **1b** (1.04 g, 8.1 mmol), triethylamine (1.64 g, 16.3 mmol) and palladium acetate (0.12 g, 0.54 mmol), and the mixture was stirred at 85°C for 16 hours. After completion of the reaction, the mixture was diluted with distilled water (30 mL), and extracted with ethyl acetate (50 mL*3). The organic phase was washed with distilled water and saturated brine, dried over anhydrous sodium sulphate and concentrated. The residue was separated and purified by silica gel column (PE/EA = 10/1-5/1, v/v) to obtain title compound 1c as a brown solid (1.3 g, 57%).

LC-MS(ESI) : m/z =417.2[M+H]⁺

¹H NMR (400 MHz, DMSO-d6) δ 8.61 (s, 1H), 8.45 (t, *J*= 2.8 Hz, 1H), 8.44 (s, 1H), 8.00 (s, 1H), 7.98 (s, 1H), 7.70 (d, *J*= 16.0 Hz, 1H), 7.45 (s, 1H), 7.43 (s, 1H), 6.60 (d, *J*= 16.0 Hz, 1H), 2.35(s, 3H), 1.45 (s, 9H).

### Step 2:

### (E)-3-(5-fluoro-1-tosyl-1H-pyrrolo[2,3-b]pyridin-3-yl)acrylic acid (1d)

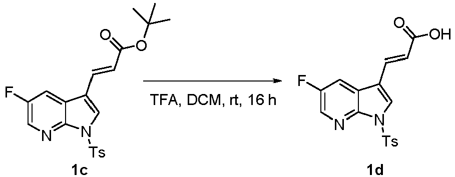

To a solution of compound **1c** (0.5 g, 1.20 mmol) in dichloromethane (10 mL) was added TFA (4 ml), and the mixture was reacted at room temperature for 16 hours. After completion of the reaction, the reaction solution was diluted with dichloromethane (20 mL). The organic phase was washed with distilled water and saturated brine, dried over anhydrous sodium sulphate, and concentrated to obtain title compound **1d** as a light yellow solid (340 mg, 79%).

LC-MS(ESI) : m/z =361.1[M+H]⁺

¹H NMR (400 MHz, DMSO-d6) δ 12.37 (s, 1H), 8.59 (s, 1H), 8.44 (s, 1H), 8.42 (t, *J*= 2.8 Hz, 1H), 8.01 (s, 1H), 7.99 (s, 1H), 7.74 (d, *J*= 16.4 Hz, 1H), 7.45 (s, 1H), 7.43 (s, 1H), 6.63 (d, *J*= 16.4 Hz, 1H), 2.35(s, 3H).

### Step 3:

### ethyl (1R,2S,3S,4R)-3-((E)-3-(5-fluoro-1-tosyl-1H-pyrrolo[2,3-b]pyridin-3-yl)acrylamido)bicyclo[2.2.2]octane-2-carboxylate (1f)

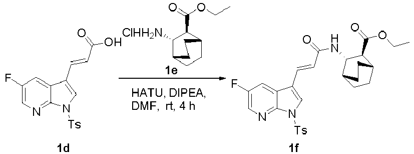

To a solution of compound **1d** (0.35 g, 0.94 mmol) in N,N-dimethylformamide (10 mL) were added 1e (0.26 g, 1.13 mmol), 2-(7-azabenzotriazole)-N,N,N',N'-tetramethyluronium hexafluorophosphate (0.43 g, 1.13 mmol) and N,N-diisopropylethylamine (0.36 g, 2.82 mmol), and the mixture was reacted at room temperature for 4 hours. After completion of the reaction, the mixture was diluted with distilled water (30 mL), and extracted with dichloromethane (20 mL*3). The organic phase was washed with distilled water and saturated brine, dried over anhydrous sodium sulphate and concentrated. Title compound **1f** was obtained as a light yellow solid (450 mg, 89%).

LC-MS(ESI) : m/z =540.2[M-H]⁺

¹H NMR (400 MHz, DMSO-d6) δ 8.48 (s, 1H), 8.43 (s, 1H), 8.23 (d, *J*= 9.2 Hz, 1H), 8.01-7.96 (m, 3H), 7.51 (d, *J*= 16.0 Hz, 1H), 7.45 (s, 1H), 7.43 (s, 1H), 6.78 (d, *J*= 16.0 Hz, 1H), 4.27 (t, *J*= 6.4 Hz, 1H), 4.17-4.00 (m, 2H), 2.89(s, 1H), 1.98(s, 1H), 2.35(s, 3H), 1.67-1.37 (m, 9H), 1.20-1.58 (m, 3H).

### Step 4:

### (1R,2S,3S,4R)-3-((E)-3-(5-fluoro-1H-pyrrolo[2,3-b]pyridin-3-yl)acrylamido)bicyclo[2.2.2]octane-2-carboxylic acid (compound 1)

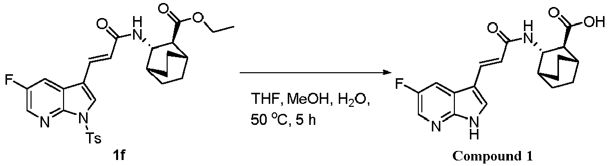

Compound **1f** (0.2 g, 0.37 mmol) was dissolved in tetrahydrofuran (6 mL)/methanol (2 ml) and distilled water (2 ml), and then lithium hydroxide monohydrate (0.12 g, 2.96 mmol) was added to the mixture. Then the mixture was reacted at 50°C for 5 hours. After completion of the reaction, 1 N hydrochloric acid was added to adjust pH to 3, and the solvent was removed under reduced pressure. The residue was purified by column chromatography (PE/EA = 10/1-1/2, v/v) to obtain target **compound 1** as a white solid (78 mg, 59%).

LC-MS(ESI) : m/z =358.2[M+H]⁺

¹H NMR (400 MHz, DMSO-d6) δ 12.21 (s, 1H), 8.31 (s, 1H), 8.11 (d, *J*= 10.0 Hz, 1H), 7.98 (s, 1H), 7.83 (d, *J=* 7.2 Hz, 1H), 7.51 (d, *J*= 16.0 Hz, 1H), 6.63 (d, *J*= 16.0 Hz, 1H), 4.25 (s 1H), 2.30(s, 1H), 1.93(s, 1H), 1.71-1.23 (m, 9H).

### Example 2 (1R,2S,3S,4R)-3-(3-(5-fluoro-1H-pyrrolo[2,3-b]pyridin-3-yl)propiolamido)bicyclo[2.2.2]octane-2-carboxylic acid (compound 2)

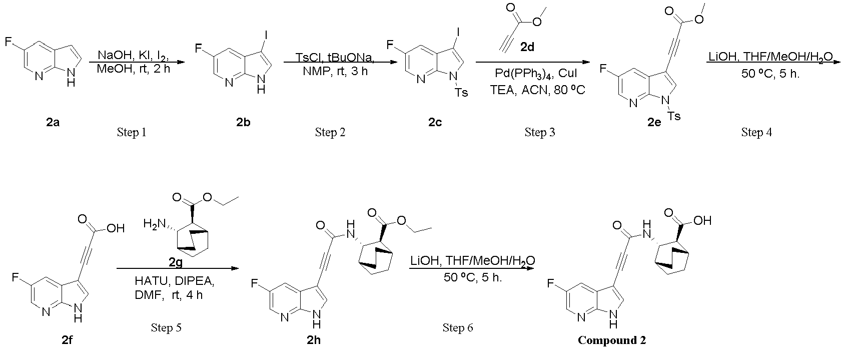

### Step 1:

### 5-fluoro-3-iodo-1H-pyrrolo[2,3-b]pyridine (2b)

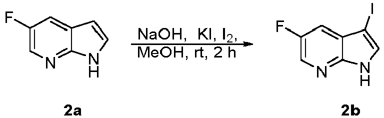

Compound **2a** (0.2 g, 1.47 mmol) was dissolved in methanol (10 ml) and distilled water (0.5 ml), and sodium hydroxide (0.06 g, 1.47 mmol), potassium iodide (0.24 g, 1.47 mmol) and iodine (0.37 g, 1.47 mmol) were added to the mixture. Then the mixture was reacted at room temperature for 2 hours. After completion of the reaction, the mixture was diluted with distilled water (30 mL), and extracted with ethyl acetate (30 mL*3). The organic phase was washed with distilled water and saturated brine, dried over anhydrous sodium sulphate and concentrated. The residue was separated and purified by silica gel column (PE/EA = 10/1-5/1, v/v) to obtain title compound **2b** as a brown solid (0.35 g, 90%).

LC-MS(ESI) : m/z =263.1[M+H]⁺

¹H NMR (400 MHz, DMSO-d6) δ 12.24 (s, 1 H), 8.24 (s, 1 H), 7.81 (s, 1 H), 7.55 (dd, J = 9.0, 2.6 Hz, 1 H).

### Step 2:

### 5-fluoro-3-iodo-1-tosyl-1H-pyrrolo[2,3-b]pyridine (2c)

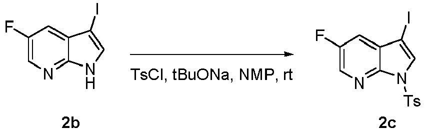

Compound **2b** (0.2 g, 0.76 mmol) was dissolved in N-methylpyrrolidone (10 mL), and tert-butoxy sodium (0.87 g, 0.91 mmol) and 4-toluenesulfonyl chloride (0.17 g, 0.91 mmol) were added to the mixture. Then the mixture was reacted at room temperature for 2 hours. After completion of the reaction, the mixture was diluted with distilled water (30 mL), and extracted with ethyl acetate (30 mL*3). The organic phase was washed with distilled water and saturated brine, dried over anhydrous sodium sulphate and concentrated. The residue was separated and purified by silica gel column (PE/EA = 10/1-5/1, v/v) to obtain title compound 2c as a light brown solid (0.3 g, 95%).

LC-MS(ESI) : m/z =417.2[M+H]⁺

¹H NMR (400 MHz, DMSO-d6) δ 8.43 (s, 1 H), 8.24 (s, 1 H), 8.00 (d, J = 8.4 Hz, 2H), 7.74 (dd, J = 8.5, 2.6 Hz, 1 H), 7.43 (d, J = 8.2 Hz, 2H), 2.35 (s, 3H).

### Step 3:

### methyl 3-(5-fluoro-1-tosyl-1H-pyrrolo[2,3-b]pyridin-3-yl)propiolate (2e)

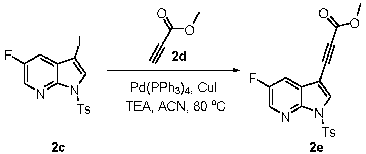

To a solution of compound **2c** (1.0 g, 2.4 mmol) in acetonitrile (10 ml) were added compound **2d** (0.60 g, 7.2 mmol), triethylamine (0.73 g, 7.2 mmol), cuprous iodide (0.046 g, 0.24 mmol) and tetrakis(triphenylphosphine)palladium (0.139 g, 0.12 mmol), and the mixture was stirred at 80°C for 16 hours. After completion of the reaction, the mixture was diluted with distilled water (30 mL), and extracted with ethyl acetate (30 mL*3). The organic phase was washed with distilled water and saturated brine, dried over anhydrous sodium sulphate and concentrated. The residue was separated and purified by silica gel column (PE/EA = 10/1-1/2, v/v) to obtain title compound **2e** (0.08 g, 9%).

LC-MS(ESI) : m/z =373.1[M+H]⁺

### Step 4:

### 3-(5-fluoro-1H-pyrrolo[2,3-b]pyridin-3-yl)propiolic acid (2f)

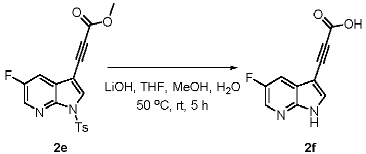

Compound **2e** (0.08 g, 0.22 mmol) was dissolved in tetrahydrofuran (6 mL)/methanol (2 ml) and distilled water (2 ml), and then lithium hydroxide monohydrate (0.07 g, 1.72 mmol) was added to the mixture. Then the mixture was reacted at 50°C for 5 hours. After completion of the reaction, water (20 mL) was added and 1 N hydrochloric acid was added to adjust pH to 3. Then the mixture was extracted with DCM (20 mL*3). The organic phase was dried over anhydrous sodium sulphate and filtered. The filtrate was concentrated under reduced pressure to obtain **2f** as an off-white solid (0.04 g, 89%).

LC-MS(ESI) : m/z =205.2[M+H]⁺

¹H NMR (400 MHz, DMSO-d6) δ 12.68 (s, 1 H), 8.36 (s, 1 H), 8.28 (s, 1 H), 7.89 (dd, *J1*= 2.4 Hz, *J2*= 8.8 Hz, 1H).

### Step 5:

### ethyl (1R,2S,3 S,4R)-3-(3-(5-fluoro-1H-pyrrolo[2,3-b]pyridin-3-yl)propiolamido)bicyclo[2.2.2]octane-2-carboxylate (2h)

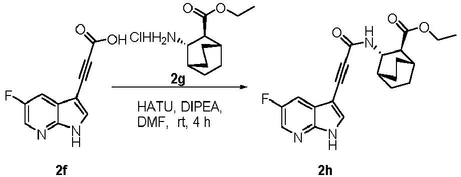

To a solution of compound **2f (40** mg, 0.20 mmol) in N,N-dimethylformamide (6 mL) were added **2g** (55 mg, 0.24 mmol), 2-(7-azabenzotriazole)-N,N,N',N'-tetramethyluronium hexafluorophosphate (91 mg, 0.24 mmol) and N,N-diisopropylethylamine (77 mg, 0.6 mmol), and the mixture was reacted at room temperature for 4 hours. After completion of the reaction, the mixture was diluted with distilled water (30 mL), and extracted with dichloromethane (20 mL*3). The organic phase was washed with distilled water and saturated brine, dried over anhydrous sodium sulphate and concentrated. Title compound **2h** was thereby obtained (0.03 g, 40%).

LC-MS(ESI) : m/z =384.2[M+H]⁺

### Step 6:

### (1R,2S,3S,4R)-3-(3-(5-fluoro-1H-pyrrolo[2,3-b]pyridin-3-yl)propiolamido)bicyclo[2.2.2]octane-2-carboxylic acid (compound 2)

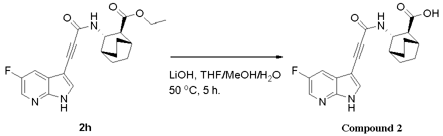

Compound **2h** (0.03 g, 0.08 mmol) was dissolved in tetrahydrofuran (4 mL)/methanol (1 ml) and distilled water (1 ml), and then lithium hydroxide monohydrate (0.01 g, 0.24 mmol) was added to the mixture. Then the mixture was reacted at 50°C for 5 hours. After completion of the reaction, 1 N hydrochloric acid was added to adjust pH to 3. The mixture was subjected to rotary evaporation. The residue was separated by column chromatography (PE/EA = 10/1-1/2, v/v) to obtain target **compound 2** as a white solid (7 mg, 65%).

LC-MS(ESI) : m/z =356.2[M+H]⁺

¹H NMR (400 MHz, DMSO- d6) δ 11.05 (s, 1H), 8.56 (d, *J*= 7.2 Hz, 1H), 8.34 (t, *J*= 2.4 Hz, 1H), 8.13 (s, 1H), 7.98 (dd, *J1*= 2.4 Hz, *J2*= 8.8 Hz,1H), 4.24 (t, *J*= 6.4 Hz, 1H), 4.24 (t, *J*= 6.0 Hz, 1H), 1.93 (s, 1H), 1.67- 1.36 (m, 9H).

### Example 3

### (2S,3S)-3-((2-(5-fluoro-1H-pyrrolo[2,3-b]pyridin-3-yl)imidazo[2,1-f][1,2,4]triazin-4-yl)amino)bicyclo[2.2.2]octane-2-carboxylic acid (compound 3)

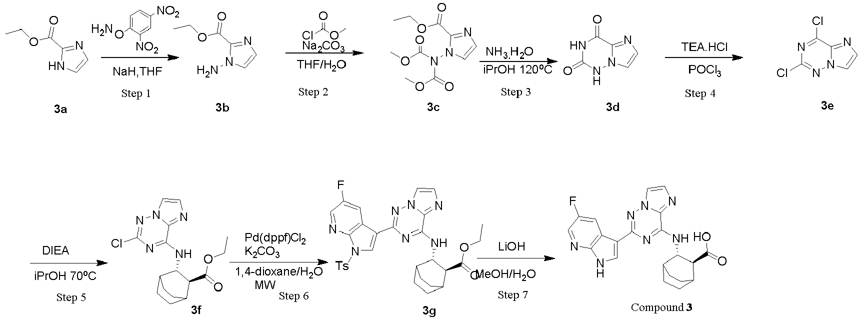

### Step 1:

### ethyl 1-amino-1H-imidazole-2-carboxylate (3b)

Under nitrogen protection, compound **3a** (5.0 g, 35.7 mmol) was dissolved in tetrahydrofuran (100 ml), and the mixture was cooled to 0-5°C in an ice bath. Sodium hydride (1.7 g, 42.8 mmol, content: 60%) was added in portions and after the addition, the mixture was stirred for 30 minutes while the temperature was controlled at 0-5°C. 2,4-dinitrophenylhydroxylamine (10.6 g, 53.6 mmol) was then added and after the addition, the mixture was warmed to room temperature and stirred for 15 hours. After completion of the reaction, the reaction solution was slowly poured into 200 ml of ice water and extracted with ethyl acetate (150 ml*4). The ethyl acetate phases were combined, washed with saturated brine, dried over anhydrous sodium sulphate and filtered. The filtrate was concentrated under reduced pressure, and then the residue was separated and purified by column chromatography (petroleum ether: ethyl acetate (v/v) = 10 : 1-1 : 1) to obtain compound **3b** (3.6 g, 65%).

LC-MS (ESI): m/z =156.1[M+H]⁺.

### Step 2:

### ethyl 1-(bis(methoxycarbonyl)amino)-1H-imidazole-2-carboxylate (3c)

Compound **3b** (3.6 g, 23.2 mmol) was dissolved in a mixed solvent of tetrahydrofuran (100 ml) and water (20 ml), and sodium carbonate (12.2 g, 116.0 mmol) was added to the mixture. Methyl chloroformate (8.7 g, 92.8 mmol) was added dropwise while the temperature was controlled at 0-5°C and after the addition, the mixture was naturally warmed to room temperature and reacted for 3 hours. After completion of the reaction, the mixture was filtered, and the filtrate was extracted with ethyl acetate (100 ml^{∗}2). The ethyl acetate phases were combined, washed with saturated brine, dried over anhydrous sodium sulphate and filtered. The filtrate was concentrated under reduced pressure, and then the residue was separated and purified by column chromatography (petroleum ether: ethyl acetate (v/v) = 10 : 1-5 : 1) to obtain compound **3c** (4.2 g, 67%).

LC-MS (ESI): m/z =272.1[M+H]⁺.

### Step 3:

### imidazo[2,1-f][1,2,4]triazine-2,4(1H,3H)-dione (3d)

To a 120 ml sealed tube were added compound **3c** (4.2 g, 15.5 mmol), isopropanol (20 ml) and ammonia water (60 ml, 28%-30%). After the tube was sealed, the mixture was warmed to 110°C and reacted for 15 hours. After completion of the reaction, the mixture was cooled to room temperature and then concentrated under reduced pressure. The concentrated residue was slurried with a mixed system of diethyl ether and methanol (10 : 1, 20 ml, v/v) and filtered. The filter cake was rinsed with diethyl ether (5 ml) and dried to obtain compound **3d** (1.2 g, 51%).

LC-MS (ESI): m/z =151.1[M-H]⁻.

### Step 4:

### 2,4-dichloroimidazo[2,1-f][1,2,4]triazine (3e)

To a 48 ml sealed tube were added compound **3d** (1.2 g, 7.9 mmol), triethylamine hydrochloride (543.5 mg, 3.9 mmol) and phosphorus oxychloride (30 ml). After the addition and the tube was sealed, the mixture was warmed to 110°C and reacted for 23 h. After completion of the reaction, the mixture was cooled to room temperature and then concentrated under reduced pressure. The residue was separated and purified by column chromatography (dichloromethane: methanol (v/v) = 1 : 0-20 : 1) to obtain compound **3e** (720 mg, 48%).

### Step 5:

### Ethyl-(2S,3S)-3-((2-chloroimidazo[2,1-f][1,2,4]triazin-4-yl)amino)bicyclo[2.2.2]octane-2-carboxylate (3f)

Compound **3e** (720 mg, 3.8 mmol) was dissolved in isopropanol (15 ml). DIPEA (1.5 g, 11.4 mmol) and ethyl-(2S,3S)-3-aminobicyclo[2.2.2]octane-2-carboxylate hydrochloric acid (977.0 mg, 4.1 mmol) were added and after the addition, the mixture was warmed to 70°C and reacted for 6 hours. After completion of the reaction, the mixture was directly concentrated under reduced pressure, and the residue was separated and purified by column chromatography (petroleum ether: ethyl acetate (v/v) = 10 : 1-3 : 1) to obtain compound **3f** (870 mg, 66%).

LC-MS (ESI): m/z =350.1[M+H]⁺.

### Step 6: Ethyl-(2S,3S)-3-((2-(5-fluoro-1-tosyl-1H-pyrrolo[2,3-b]pyridin-3-yl)imidazo[2,1-f][1,2,4]triazin-4-yl)amino)bicyclo[2.2.2]octane-2-carboxylate (3g)

Compound **3f** (450.0 mg, 1.3 mmol) was dissolved in a mixed solvent of 1,4-dioxane (5 ml) and water (1 ml). Intermediate 1 (650 mg, 1.5 mmol), potassium carbonate (538 mg, 3.9 mmol) and Pd(dppf)Cl₂ (95 mg, 0.13 mmol) were added and after the addition, the mixture was reacted in a microwave reactor at 120°C for 1 hour. After completion of the reaction, the mixture was filtered, and the filtrate was concentrated. The residue was separated and purified by column chromatography (petroleum ether: ethyl acetate (v/v) = 10 : 1-5 : 1) to obtain compound **3g** (110 mg, 14%).

LC-MS (ESI): m/z =604.2[M+H]⁺.

### Step 7: (2S,3S)-3-((2-(5-fluoro-1H-pyrrolo[2,3-b]pyridin-3-yl)imidazo[2,1-f][1,2,4]triazin-4-yl)amino)bicyclo[2.2.2]octane-2-carboxylic acid (compound 3)

Compound **3g** (110 mg, 0.18 mmol) was dissolved in methanol (5 ml). Lithium hydroxide (70.5 mg, 1.7 mmol, dissolved in 2 ml of water) was added and after the addition, the mixture was warmed to 60°C and reacted for 5 hours. After completion of the reaction, 1 N hydrochloric acid was added to adjust PH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound **3** (24.0 mg, 32%).

LC-MS (ESI): m/z =422.2[M+H]⁺.

¹H NMR (400 MHz, DMSO-d6) δ 12.31 (s, 2H), 8.73 (d, 1H), 8.52-8.48 (m,1H),8.32-8.30 (m, 1H), 8.23 (d, 1H),8.10(s, 1H), 7.58(s, 1H), 4.85-4.81 (m, 1H), 3.03(d, 1H), 2.02(s, 2H), 1.81-1.39(m, 8H).

### Example 4: (1R,2S,3S,4R)-3-((7-acetamido-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 4)

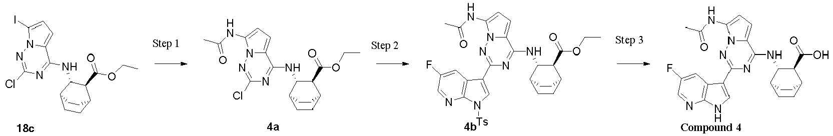

### Step 1:

### Ethyl (1R,2S,3S,4R)-3-((7-acetamido-2-chloropyrrolo[2,1-f][1,2,4]triazin-4-yl)amino)bicyclo[2.2.2]octane-2-carboxylate (4a)

**18c** (300 mg, 0.63 mmol) was dissolved in dioxane (20 mL), and acetamide (186 mg, 3.15 mmol), potassium phosphate (400 mg, 1.89 mmol), cuprous iodide (24 mg, 0.13 mmol) and trans-(1R,2R)-N,N'-dimethyl 1,2-cyclohexanediamine (18 mg, 0.13 mmol) were added to the mixture. Under nitrogen protection, the mixture was reacted at 110°C for 4 hours and concentrated. Then the residue was separated and purified by silica gel column chromatography (petroleum ether: ethyl acetate (v/v) = 1 : 5-1 : 2) to obtain crude title compound (**4a**) as a light yellow liquid (130 mg, yield: 50%).

LCMS m/z =406.16 [M+1]

### Step 2:

### ethyl (1R,2S,3S,4R)-3-((7-acetamido-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (4b)

(**4a**) (100 mg, 0.25 mmol) was dissolved in dioxane (10 mL), and 5-fluoro-3-(4,4,5,5-tetramethyl-1,3,2-dioxaboran-2-yl)-1-tosyl-1H-pyrrolo [2,3-b]pyridine (156 mg, 0.38 mmol), potassium carbonate (104 mg, 0.75 mmol) and [1,1'-bis(diphenylphosphino)ferrocene]palladium dichloride (50 mg, 0.07 mmol) were added to the mixture, followed by water (1 mL). Under nitrogen protection, the mixture was reacted under microwave at 120°C for 1 hour and concentrated. Then the residue was separated and purified by silica gel column chromatography (petroleum ether: ethyl acetate (v/v) = 1 : 5-1 : 2) to obtain title compound (**4b**) as a yellow solid (60 mg, yield: 36%).

LCMS m/z =660.23 [M+1]

### Step 3:

### (1R,2S,3S,4R)-3-((7-acetamido-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 4)

**(4b)** (60 mg, 0.09 mmol) was dissolved in methanol (10 mL). Water (5 mL) and lithium hydroxide (22 mg, 0.9 mmol) were added, and the mixture was reacted at 60°C for 2 hours. 1 N hydrochloric acid was added to adjust ph to 5-6. Water (20 mL) was added, and the mixture was extracted with dichloromethane (50 mL × 3). The organic phases were combined, washed with a saturated aqueous sodium chloride solution (50 mL), dried over anhydrous sodium sulphate and concentrated. The obtained crude compound was separated and purified by a preparative liquid chromatography column (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain title compound **(compound 4)** (10 mg, 21%, retention time: about 6 min).

¹H NMR (400 MHz, DMSO-d6) δ 12.18 (d, 2H), 9.97 (s, 1H), 8.65 - 8.60 (m, 1H), 8.29 - 8.24 (m, 2H), 7.84 (d, 1H), 6.93 (d, 1H), 6.64 (d, 1H), 4.83 - 4.76 (m, 1H), 2.76 (d, 1H), 2.21 (s, 3H), 2.05 - 2.02 (m, 2H), 1.79 - 1.46 (m, 8H).

MS M/Z (ESI): m/z =478.19 (M+1)

### Example 5: (1R,2S,3S,4R)-3-((2-(5-fluoro-1H-pyrrolo[2,3-b]pyridin-3-yl)-6-(trifluoromethyl)pyrrolo[2,1-f][1,2,4]triazin-4-yl)amino)bicyclo[2.2.2]octane-2-carboxylic acid (compound 5)

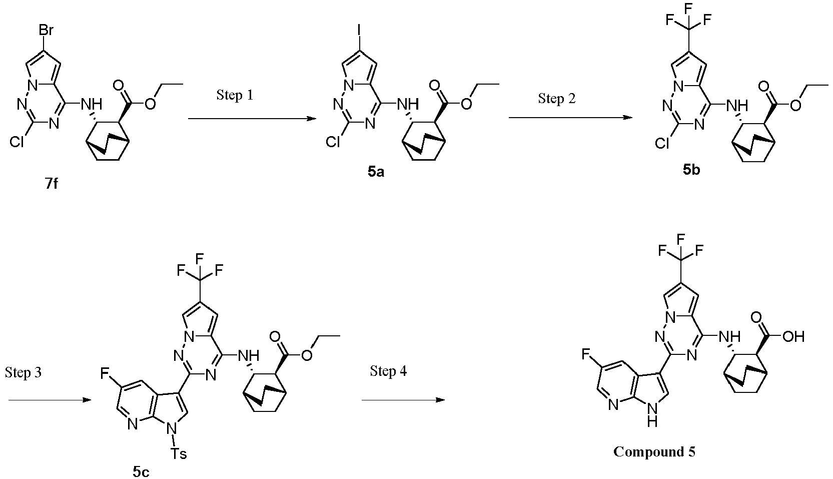

### Step 1:

### Ethyl (1R,2S,3S,4R)-3-((2-chloro-6-iodopyrrolo[2,1-f][1,2,4]triazin-4-yl)amino)bicyclo[2.2.2]octane-2-carboxylate (5a)

**7f** (300 mg, 0.7 mmol) was dissolved in dioxane (20 mL), and sodium iodide (300 mg, 0.7 mmol), cuprous iodide (50 mg, 0.27 mmol) and (1R,2R)-N1,N2-dimethyl cyclohexane-1,2-diamine (50 mg, 0.35 mmol) were added to the mixture. Under nitrogen protection, the mixture was reacted at 100°C for 4 hours and concentrated. Then the residue was separated and purified by silica gel column chromatography (petroleum ether: ethyl acetate (v/v) = 0.1 : 1-0.3 : 1) to obtain title compound (**5a**) as a yellow solid (260 mg, yield: 70%).

LCMS m/z =475.03 [M+1]

### Step 2:

### ethyl (1R,2S,3S,4R)-3-((2-chloro-6-(trifluoromethyl)pyrrolo[2,1-f][1,2,4]triazin-4-yl)amino)bicyclo[2.2.2]octane-2-carboxylate (5b)

**5a** (260 mg, 0.55 mmol) was dissolved in DMF (20 mL), and cuprous iodide (314 mg, 1.65 mmol) and methyl 2,2-difluoro-2-(fluorosulfonyl)acetate (1056 mg, 5.5 mmol) were added to the mixture. Under nitrogen protection, the mixture was reacted at 110°C for 4 hours. Water (30 mL) was added, and the mixture was extracted with ethyl acetate (30 mL × 3), washed with water (30 mL × 3), dried over anhydrous sodium sulphate, and separated and purified by silica gel column chromatography (petroleum ether: ethyl acetate (v/v) = 0.1 : 1-0.3 : 1) to obtain title compound (**5b**) as a light yellow solid (180 mg, yield: 78.5%).

LCMS m/z =417.12 [M+1]

### Step 3:

### ethyl (1R,2S,3S,4R)-3-((2-(5-fluoro-1-tosyl-1H-pyrrolo[2,3-b]pyridin-3-yl)-6-(trifluoromethyl)pyrrolo[2,1-f][1,2,4]triazin-4-yl)amino)bicyclo[2.2.2]octane-2-carboxylate (5c)

**5b** (180 mg, 0.43 mmol) was dissolved in dioxane (10 mL), and 5-fluoro-3-(4,4,5,5-tetramethyl-1,3,2-dioxaboran-2-yl)-1-tosyl-1H-pyrrolo [2,3-b]pyridine (268 mg, 0.65 mmol), potassium carbonate (178 mg, 1.29 mmol) and [1,1'-bis(diphenylphosphino)ferrocene]palladium dichloride (50 mg, 0.07 mmol) were added to the mixture, followed by water (1 mL). Under nitrogen protection, the mixture was reacted under microwave at 120°C for 1 hour and concentrated. Then the residue was separated and purified by silica gel column chromatography (petroleum ether: ethyl acetate (v/v) = 1 : 5-1 : 2) to obtain title compound (**5c**) as a yellow solid (80 mg, yield: 27%).

LCMS m/z =671.02 [M+1]

### Step 4:

### (1R,2S,3 S,4R)-3-((2-(5-fluoro-1H-pyrrolo[2,3-b]pyridin-3-yl)-6-(trifluoromethyl)pyrrolo[2,1-f][1,2,4]triazin-4-yl)amino)bicyclo[2.2.2]octane-2-carboxylic acid (compound 5)

**5c** (80 mg, 0.12 mmol) was dissolved in methanol (10 mL). Water (5 mL) and lithium hydroxide (29 mg, 1.2 mmol) were added, and the mixture was reacted at 60°C for 2 hours. 1 N hydrochloric acid was added to adjust ph to 5-6. Water (20 mL) was added, and the mixture was extracted with dichloromethane (50 mL × 3). The organic phases were combined, washed with a saturated aqueous sodium chloride solution (50 mL), dried over anhydrous sodium sulphate and concentrated. The obtained crude compound was separated and purified by a preparative liquid chromatography column (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain title compound **(compound 5**) (30 mg, 51%, retention time: about 6.5 min).

¹H NMR (400 MHz, DMSO-d6) δ 12.28 (d, 2H), 8.54 - 8.50 (m, 1H), 8.30 (d, 1H), 8.26 (s, 1H), 8.23 (d, 1H), 8.18 (d, 1H), 7.35 (s, 1H), 4.82 - 4.78 (m, 1H), 2.72 (d, 1H), 2.05 - 2.02 (m, 2H), 1.82 - 1.42 (m, 8H).

MS M/Z (ESI): m/z =489.16 (M+1)

### Example 6: (1R,2S,3S,4R)-3-((2-(5-fluoro-1H-pyrrolo[2,3-b]pyridin-3-yl)-6-(1-methyl-1H-pyrazol-4-yl)pyrrolo[2,1-f][1,2,4]triazin-4-yl)amino)bicyclo[2.2.2]octane-2-carboxylic acid (compound 6)

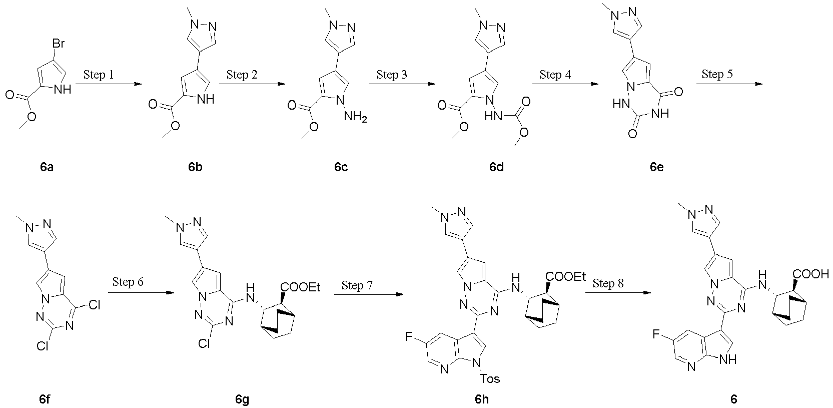

### Step 1:

### methyl 4-(1-methyl-1H-pyrazol-4-yl)-1H-pyrrole-2-carboxylate (6b)

At room temperature, compound **6a** (3 g, 14.7 mmol) was added to a mixed solution of dioxane (60 mL) and water (6 mL). 1-methyl-1H-pyrazole-4-boronic acid pinacol ester (4.3 g, 20.6 mmol), potassium acetate (4.3 g, 44.1 mmol) and Pd(dppf)Cl₂ (150 mg, 0.2 mmol) were successively added to the mixture. The mixture was subjected to nitrogen replacement and protection, and was reacted at 100°C for 4 hours. The reaction solution was poured into 200 mL of water and extracted with ethyl acetate (200 mL × 1). The organic phase was concentrated to dryness, and the residue was purified by column chromatography (PE/EA, 5/1-1/1, v/v) to obtain compound **6b** (2 g, yield: 65%).

LC-MS (ESI): m/z =206.1 [M+H]⁺.

### Step 2:

### methyl 1-amino-4-(1-methyl-1H-pyrazol-4-yl)-1H-pyrrole-2-carboxylate (6c)

At room temperature, compound **6b** (1.7 g, 8.3 mmol) was added to DMF (250 mL). Under nitrogen protection, the mixture was cooled to 0°C, and sodium hydride (0.4 g, 10.0 mmol) was added to the mixture in portions at 0-5°C. The mixture was naturally warmed and reacted for 1 hour. Diphenylphosphinyl hydroxylamine (2.4 g, 10.0 mmol) was added, and the mixture was reacted at room temperature for 1 hour. The reaction solution was poured into ice water (1 L) and extracted with ethyl acetate (300 mL × 1). The organic phase was concentrated to dryness to obtain compound **6c** (1.5 g, yield: 82%).

LC-MS (ESI): m/z =221.1 [M+H]⁺.

### Step 3:

### Methyl-1-((methoxycarbonyl)amino)-4-(1-methyl-1H-pyrazol-4-yl)-1H-pyrrole-2-carboxylate (6d)

At room temperature, compound **6c** (1.5 g, 6.8 mmol) was added to DCM (30 mL), and the mixture was cooled to 0°C. Under nitrogen protection, pyridine (0.65 g, 8.2 mmol) was added to the mixture. Methyl chloroformate (0.77 g, 8.2 mmol) was added dropwise to the mixture at 0-2°C. The mixture was naturally warmed to room temperature and reacted for 1 hour. The reaction solution was poured into water (100 mL) and extracted with DCM (50 mL × 1). The organic phase was concentrated to dryness to obtain compound **6d** (1.7 g, yield: 90%).

LC-MS (ESI): m/z =279.1 [M+H]⁺.

### Step 4:

### 6-(1-methyl-1H-pyrazol-4-yl)pyrrolo[2,1-f][1,2,4]triazine-2,4(1H,3H)-dione (6e)

At room temperature, compound **6d** (1 g, 3.6 mmol) was added to a mixed solution of dioxane (10 mL) and ammonia water (50 mL). The tube was sealed, and the mixture was heated to 120°C and reacted for 5 hours. The reaction solution was cooled to room temperature, concentrated to dryness, slurried with isopropanol (50 mL) and filtered. The solid was dried to obtain compound **6e** (0.5 g, yield: 61%).

LC-MS (ESI): m/z =232.1 [M+H]⁺.

### Step 5:

### 2,4-dichloro-6-(1-methyl-1H-pyrazol-4-yl)pyrrolo[2,1-f][1,2,4]triazine (6f)

At room temperature, compound **6e** (0.5 g, 2.2 mmol) was added to toluene (20 mL). Phosphorus oxychloride (0.8 g, 5.5 mmol) and DIPEA (0.8 g, 6.6 mmol) were successively added, and the mixture was reacted at 130°C for 5 hours. The reaction solution was cooled to room temperature, poured into 50 mL of ice water and extracted with ethyl acetate (30 mL × 1). The organic phase was concentrated to dryness, and the residue was purified by column chromatography (PE/EA, 10/1-5/1, v/v) to obtain compound **6f** (100 mg, yield: 17%).

LC-MS (ESI): m/z =269.1 [M+H]⁺.

### Step 6:

### (1R,2S,3S,4R)-ethyl-3-((2-chloro-6-(1-methyl-1H-pyrazol-4-yl)pyrrolo[2,1-f][1,2,4]triazin-4-yl)amino)bicyclo[2.2.2]octane-2-carboxylate (6g)

At room temperature, compound **6f** (0.1 g, 0.37 mmol) was added to isopropanol (10 mL), and ethyl (1R,2S,3S,4R)-3-aminobicyclo[2.2.2]octane-2-carboxylate hydrochloride (0.1 g, 0.44 mmol) and DIPEA (0.1 g, 0.81 mmol) were successively added to the mixture. The mixture was reacted at 90°C for 2 hours. The reaction solution was concentrated to dryness, poured into 50 mL of water and extracted with ethyl acetate (50 mL × 1). The organic phase was concentrated to dryness, and the residue was purified by column chromatography (PE/EA, 10/1-2/1, v/v) to obtain compound **6g** (80 mg, yield: 51%).

LC-MS (ESI): m/z =429.2 [M+H]⁺.

### Step 7:

### (1R,2S,3S,4R)-ethyl-3-((2-(5-fluoro-1-tosyl-1H-pyrrolo[2,3-b]pyridin-3-yl)-6-(1-methyl-1H-pyrazol-4-yl)pyrrolo[2,1-f][1,2,4]triazin-4-yl)amino)bicyclo[2.2.2]octane-2-carboxylate (6h)

At room temperature, compound **6g** (80 mg, 0.19 mmol) was added to a mixed solution of dioxane (10 mL) and water (1 mL), and 5-fluoro-3-(4,4,5,5-tetramethyl-1,3,2-dioxaborolan-2-yl)-1-tosyl-1H-pyrrolo[2,3-b]pyridine (93 mg, 0.22 mmol), potassium carbonate (26 mg, 0.38 mmol) and Pd(dppf)Cl₂ (20 mg, 0.027 mmol) were successively added to the mixture. The mixture was reacted under microwave at 120°C for 1 hour. The reaction solution was poured into 30 mL of water and extracted with ethyl acetate (30 mL × 1). The organic phase was concentrated to dryness, and the residue was purified by a preparative plate (PE/EA, 3/1, v/v) to obtain compound **6h** (20 mg, yield: 15%).

### Step 8: (1R,2S,3S,4R)-3-((2-(5-fluoro-1H-pyrrolo[2,3-b]pyridin-3-yl)-6-(1-methyl-1H-pyrazol-4-yl)pyrrolo[2,1-f][1,2,4]triazin-4-yl)amino)bicyclo[2.2.2]octane-2-carboxylic acid (compound 6)

At room temperature, to compound **6h** (20 mg, 0.03 mmol) were successively added THF (2 mL), water (1 mL) and sodium hydroxide (23 mg, 0.6 mmol). The mixture was reacted at room temperature for 18 hours. The reaction solution was adjusted to pH 5-6 by adding 6 N hydrochloric acid, and concentrated to dryness. The residue was separated and purified by a preparative plate (DCM/MeOH, 10/1, v/v) to obtain **compound 6** (4 mg, yield: 27%).

LC-MS (ESI): m/z =501.2[M+H]⁺.

¹H NMR (400 MHz, CD₃OD) 88.61-8.56 (m,1H), 8.28 (s,1H), 8.18-8.17 (m,1H), 7.84-7.82 (m,2H),7.73 (s,1H), 7.10 (s,1H), 4.90-4.89 (m,1H), 3.93 (s,3H), 2.79-2.78 (m,1H), 2.13-2.08 (m,2H), 1.98-1.86 (m,3H), 1.76-1.68 (m,3H), 1.59-1.56 (m,2H).

### Example 7: (1R,2S,3S,4R)-3-((2-(5-fluoro-1H-pyrrolo[2,3-b]pyridin-3-yl)-6-vinylpyrrolo[2,1-f][1,2,4]triazin-4-yl)amino)bicyclo[2.2.2]octane-2-carboxylic acid (compound 7)

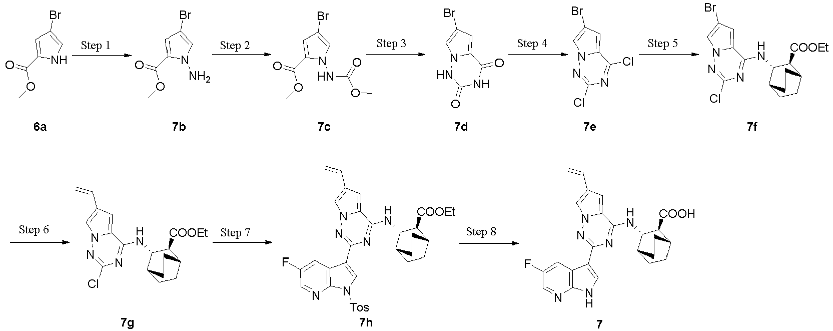

### Step 1:

### methyl 1-amino-4-bromo-1H-pyrrole-2-carboxylate (7b)

At room temperature, compound **6a** (12.0 g, 58.8 mmol) was added to DMF (300 mL). Under nitrogen protection, the mixture was cooled to 0°C, and sodium hydride (2.8 g, 70.6 mmol) was added to the mixture in portions at 0-5°C. The mixture was naturally warmed and reacted for 1 hour. Diphenylphosphinyl hydroxylamine (15.2 g, 64.7 mmol) was added, and the mixture was reacted at room temperature for 1 hour. The reaction solution was poured into ice water (1.5 L) and extracted with ethyl acetate (500 mL × 1). The organic phase was concentrated to dryness to obtain compound **7b** (12 g, yield: 94%).

LC-MS (ESI): m/z =220.1 [M+H]⁺.

### Step 2:

### methyl 4-bromo-1-((methoxycarbonyl)amino)-1H-pyrrole-2-carboxylate (7c)

At room temperature, compound **7b** (12.0 g, 55.0 mmol) was added to DCM (120 mL), and the mixture was cooled to 0°C. Under nitrogen protection, pyridine (8.7 g, 110.1 mmol) was added to the mixture. Methyl chloroformate (6.2 g, 66.0 mmol) was added dropwise to the mixture at 0-2°C. The mixture was naturally warmed to room temperature and reacted for 1 hour. The reaction solution was poured into water (150 mL) and extracted with DCM (80 mL × 1). The organic phase was concentrated to dryness to obtain compound **7c** (12 g, yield: 79%).

LC-MS (ESI): m/z =278.1 [M+H]⁺.

### Step 3:

### 6-bromopyrrolo[2,1-f][1,2,4]triazine-2,4(1H,3H)-dione (7d)

At room temperature, compound **7c** (12 g, 43.3 mmol) was added to a mixed solution of dioxane (25 mL) and ammonia water (75 mL). The tube was sealed, and the mixture was heated to 120°C and reacted for 5 hours. The reaction solution was cooled to room temperature, concentrated to dryness, slurried with isopropanol (50 mL) and filtered. The solid was dried to obtain compound **7d** (8 g, yield: 80%).

LC-MS (ESI): m/z =231.1 [M+H]⁺.

### Step 4:

### 6-bromo-2,4-dichloropyrrolo[2,1-f][1,2,4]triazine (7e)

At room temperature, compound **7d** (7.0 g, 30.4 mmol) was added to phosphorus oxychloride (70 mL). Triethylamine hydrochloride (12.5 g, 91.2 mmol) was added, and the mixture was reacted at 100°C for 16 hours. The reaction solution was cooled to room temperature and concentrated to remove phosphorus oxychloride. The concentrate was poured into ice water (200 mL) and extracted with ethyl acetate (200 mL × 1). The organic phase was concentrated to dryness to obtain compound **7e** (5 g, yield: 62%).

### Step 5:

### (1R,2S,3S,4R)-ethyl-3-((6-bromo-2-chloropyrrolo[2,1-f][1,2,4]triazin-4-yl)amino)bicyclo[2.2.2]octane-2-carboxylate (7f)

At room temperature, compound **7e** (1.0 g, 3.7 mmol) was added to isopropanol (20 mL), and ethyl (1R,2S,3S,4R)-3-aminobicyclo[2.2.2]octane-2-carboxylate hydrochloride (1.0 g, 4.4 mmol) and DIPEA (1.0 g, 8.1 mmol) were successively added to the mixture. The mixture was reacted at 90°C for 2 hours. The reaction solution was concentrated to dryness, poured into 50 mL of water and extracted with ethyl acetate (50 mL × 1). The organic phase was concentrated to dryness, and the residue was purified by column chromatography (PE/EA, 10/1-2/1, v/v) to obtain compound **7f** (1 g, yield: 63%).

LC-MS (ESI): m/z =428.1 [M+H]⁺.

### Step 6:

### (1R,2S,3S,4R)-ethyl-3-((2-chloro-6-vinylpyrrolo[2,1-f][1,2,4]triazin-4-yl)amino)bicyclo[2.2.2]octane-2-carboxylate (7g)

At room temperature, compound **7f** (1 g, 2.3 mmol) was added to a mixed solution of dioxane (20 mL) and water (2 mL), and ethenyl boronic acid pinacol ester (0.4 g, 2.5 mmol), potassium carbonate (0.8 g, 5.8 mmol) and Pd(dppf)Cl₂ (150 mg, 0.2 mmol) were successively added to the mixture. The mixture was subjected to nitrogen replacement and protection, and was reacted at 100°C for 16 hours. The reaction solution was poured into 40 mL of water and extracted with ethyl acetate (40 mL × 1). The organic phase was concentrated to dryness, and the residue was purified by column chromatography (PE/EA, 5/1-1/1, v/v) to obtain compound **7g** (0.3 g, yield: 35%).

LC-MS (ESI): m/z =375.1 [M+H]⁺.

### Step 7:

### (1R,2S,3 S,4R)-ethyl-3-((2-(5-fluoro-1-tosyl-1H-pyrrolo[2,3-b]pyridin-3-yl)-6-vinylpyrrolo[2,1-f][1,2,4]triazin-4-yl)amino)bicyclo[2.2.2]octane-2-carboxylate (7h)

At room temperature, compound **7g** (300 mg, 0.8 mmol) was added to a mixed solution of dioxane (5 mL) and water (0.5 mL), and 5-fluoro-3-(4,4,5,5-tetramethyl-1,3,2-dioxaborolan-2-yl)-1-tosyl-1H-pyrrolo[2,3-b]pyridine (400 mg, 0.96 mmol), potassium carbonate (220 mg, 1.6 mmol) and Pd(dppf)Cl₂ (30 mg, 0.04 mmol) were successively added to the mixture. The mixture was reacted under microwave at 120°C for 1 hour. The reaction solution was poured into water (30 mL) and extracted with ethyl acetate (30 mL × 1). The organic phase was concentrated to dryness, and the residue was purified by column chromatography (PE/EA, 10/1-2/1, v/v) to obtain compound **7h** (200 mg, yield: 40%).

### Step 8: (1R,2S,3S,4R)-3-((2-(5-fluoro-1H-pyrrolo[2,3-b]pyridin-3-yl)-6-vinylpyrrolo[2,1-f][1,2,4]triazin-4-yl)amino)bicyclo[2.2.2]octane-2-carboxylic acid (compound 7)

At room temperature, to compound **7h** (50 mg, 0.08 mmol) were successively added THF (2 mL), water (1 mL) and sodium hydroxide (63 mg, 0.16 mmol). The mixture was reacted at room temperature for 18 hours. The reaction solution was adjusted to pH 5-6 by adding 6 N hydrochloric acid, and concentrated to dryness. The residue was separated and purified by a preparative plate (DCM/MeOH, 10/1, v/v) to obtain **compound 7** (20 mg, yield: 56%).

LC-MS (ESI): m/z =447.2[M+H]⁺.

¹H NMR (400 MHz,DMSO-d6) δ12.19 (s,1H), 8.52-8.49 (s,1H), 8.29-8.28 (m,1H), 8.18-8.17 (m,1H),7.92-7.70 (m,1H), 7.83-7.82 (m,1H), 7.10-7.09 (m,1H), 6.76-6.69 (m,1H), 5.64-5.59 (m,1H), 5.16-5.13 (m,1H), 4.81-4.77 (m,1H), 2.77-2.73 (m,1H), 2.03 (m,2H), 1.81-1.74 (m,3H), 1.62-1.58 (m,3H), 1.49-1.42 (m,2H).

### Example 8: (1R,2S,3S,4R)-3-((6-cyclopropyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 8)

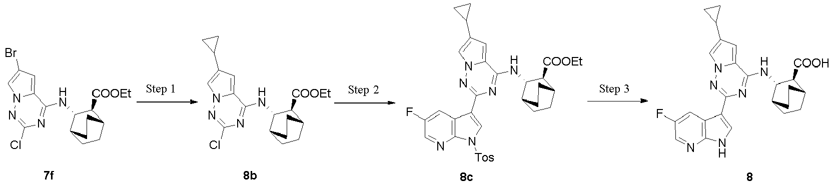

### Step 1:

### (1R,2S,3S,4R)-ethyl-3-((2-chloro-6-cyclopropylpyrrolo[2,1-f][1,2,4]triazin-4-yl) amino)bicyclo[2.2.2]octane-2-carboxylate (8b)

At room temperature, compound **7f** (1 g, 2.3 mmol) was added to a mixed solution of DMF (50 mL) and water (5 mL), and cyclopropyl boronic acid (0.5 g, 5.9 mmol), potassium carbonate (1 g, 6.9 mmol) and Pd(dppf)Cl₂ (150 mg, 0.2 mmol) were successively added to the mixture. The mixture was subjected to nitrogen replacement and protection, and was reacted at 100°C for 16 hours. The reaction solution was poured into 200 mL of water and extracted with ethyl acetate (200 mL × 1). The organic phase was concentrated to dryness, and the residue was purified by column chromatography (PE/EA, 5/1-1/1, v/v) to obtain compound **8b** (0.2 g, yield: 22%).

LC-MS (ESI): m/z =389.2 [M+H]⁺.

### Step 2:

### (1R,2S,3 S,4R)-ethyl-3-((6-cyclopropyl-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (8c)

At room temperature, compound **8b** (200 mg, 0.5 mmol) was added to a mixed solution of dioxane (5 mL) and water (0.5 mL), and 5-fluoro-3-(4,4,5,5-tetramethyl-1,3,2-dioxaborolan-2-yl)-1-tosyl-1H-pyrrolo[2,3-b]pyridine (400 mg, 0.96 mmol), potassium carbonate (220 mg, 1.6 mmol) and Pd(dppf)Cl₂ (30 mg, 0.04 mmol) were successively added to the mixture. The mixture was reacted under microwave at 120°C for 1 hour. The reaction solution was poured into water (50 mL) and extracted with ethyl acetate (50 mL × 1). The organic phase was concentrated to dryness, and the residue was purified by column chromatography (PE/EA, 10/1-2/1, v/v) to obtain compound **8c** (30 mg, yield: 10%).

### Step 8:

### (1R,2S,3 S,4R)-3-((6-cyclopropyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 8)

At room temperature, to compound **8c** (30 mg, 0.05 mmol) were successively added THF (2 mL), water (1 mL) and sodium hydroxide (56 mg, 1.5 mmol). The mixture was reacted at room temperature for 18 hours. The reaction solution was adjusted to pH 5-6 by adding 6 N hydrochloric acid, concentrated and then extracted with EA (20 mL × 1). The organic phase was concentrated to dryness, and the residue was separated and purified by a preparative plate (DCM/MeOH, 10/1, v/v) to obtain **compound 8** (10 mg, yield: 43%).

LC-MS (ESI): m/z =461.2 [M+H]⁺.

¹H NMR (400 MHz,DMSO-d6) δ12.14 (s,1H), 8.51-8.48 (m,1H), 8.27 (m,1H), 8.15-8.14 (m,1H),7.73-7.71 (m,1H), 7.54-7.53 (m,1H), 6.09-6.08 (m,1H), 4.79-4.75 (m,1H), 2.75-2.73 (m,1H), 2.01-2.00 (m,2H), 1.92-1.88 (m,1H),1.80-1.73 (m,3H), 1.61-1.57 (m,3H), 1.49-1.43 (m,2H), 0.95-0.90 (m,2H), 0.62-0.58 (m,2H).

### Example 9: (1R,2S,3S,4R)-3-((6-(difluoromethyl)-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 9)

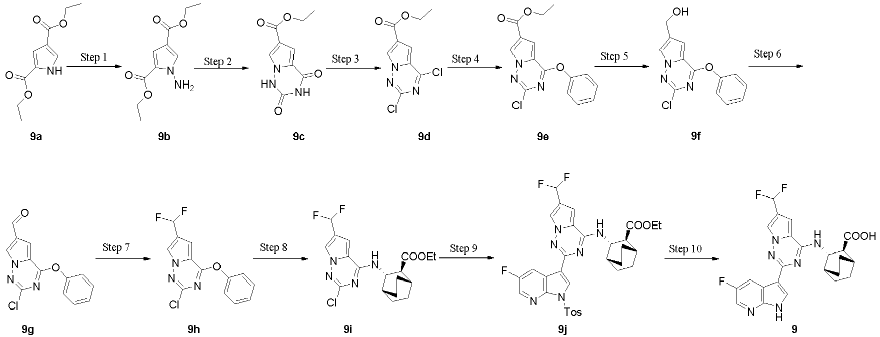

### Step 1:

### diethyl 1-amino-1H-pyrrole-2,4-dicarboxylate (9b)

At room temperature, compound **9a** (10 g, 47.4 mmol) was added to THF. Under nitrogen protection, sodium hydride (1.4 g, 56.8 mmol) was added to the mixture in portions at 0°C and after the addition, the mixture was naturally warmed and reacted for 1 hour. 2,4-dinitrophenylhydroxylamine (10.4 g, 52.1 mmol) was added, and the mixture was reacted at room temperature for 1 hour. The reaction solution was poured into water (500 mL) and extracted with EA (300 mL × 1). The organic phase was concentrated to dryness, and the residue was purified by column chromatography (PE/EA=10/1-5/1, v/v) to obtain compound **9b** (8 g, yield: 75%).

LC-MS (ESI): m/z =227.1 [M+H]⁺.

### Step 2:

### ethyl 2,4-dioxo-1,2,3,4-tetrahydropyrrolo[2,1-f][1,2,4]triazine-6-carboxylate (9c)

At room temperature, urea (21.2 g, 354.4 mmol) was heated to 180°C for melting. Compound **9b** (8 g, 35.4 mmol) was added and the mixture was reacted for 4 hours with the temperature maintained. The mixture was cooled to 100°C. Ethanol (100 mL) was added, and the mixture was refluxed, reacted and filtered. The filtrate was concentrated to dryness to obtain crude compound **9c** (8 g), which was directly used in the next step.

LC-MS (ESI): m/z =224.1 [M+H]⁺.

### Step 3:

### ethyl 2,4-dichloropyrrolo[2,1-f][1,2,4]triazine-6-carboxylate (9d)

At room temperature, crude compound **9c** (8 g) and triethylamine hydrochloride (14.8 g, 107.5 mmol) were added to phosphorus oxychloride (100 mL), and the mixture was reacted at 100°C for 16 hours, and concentrated to remove most of phosphorus oxychloride. The concentrate was poured into ice water (100 mL) and extracted with EA (100 mL × 1). The organic phase was concentrated to dryness to obtain compound **9d** (2 g, yield: 22%).

### Step 4:

### ethyl 2-chloro-4-phenoxypyrrolo[2,1-f][1,2,4]triazine-6-carboxylate (9e)

At room temperature, **9d** (2 g, 7.7 mmol) was added to THF. Sodium phenate (1.3 g, 10.8 mmol) was added, and the mixture was reacted at room temperature for 2 hours. The reaction solution was poured into water (150 mL) and extracted with EA (50 mL × 1). The organic phase was concentrated to dryness, and the residue was purified by column chromatography (PE/EA = 10/1-5/1, v/v) to obtain compound **9e** (1.5 g, yield: 61%).

LC-MS (ESI): m/z =318.1 [M+H]⁺.

### Step 5:

### (2-chloro-4-phenoxypyrrolo[2,1-f][1,2,4]triazin-6-yl)methanol (9f)

At room temperature, compound **9e** (1.5 g, 4.7 mmol) was added to DCM (50 mL), and DIBAL-H (16 mL, 23.6 mmol) was added at 0°C. The mixture was naturally warmed to room temperature and reacted for 2 hours. The reaction solution was poured into water (100 mL). DCM (100 mL) was added, and the mixture was filtered. The filtrate was concentrated to dryness to obtain compound **9f** (800 mg, yield: 61%).

LC-MS (ESI): m/z =276.1 [M+H]⁺.

### Step 6:

### 2-chloro-4-phenoxypyrrolo[2,1-f][1,2,4]triazine-6-carbaldehyde (9g)

At room temperature, compound **9f** (0.3 g, 1.1 mmol) and manganese dioxide (0.95 g, 11.0 mmol) were added to dichloromethane (10 mL), and the mixture was reacted at room temperature for 16 hours. The reaction solution was filtered. The filtrate was concentrated to dryness, and the residue was purified by column chromatography (PE/EA = 20/1-5/1, v/v) to obtain compound **9g** (200 mg, yield: 67%).

LC-MS (ESI): m/z =274.1 [M+H]⁺.

### Step 7:

### 2-chloro-6-(difluoromethyl)-4-phenoxypyrrolo[2,1-f][1,2,4]triazine (9h)

At room temperature, compound **9g** (0.2 g, 0.73 mmol) and DAST (0.6 g, 3.6 mmol) were successively added to 100 ml of DCM, and the mixture was reacted for 16 hours. The reaction solution was poured into water (50 mL) and extracted with DCM (50 mL × 1). The organic phase was concentrated to dryness, and the residue was purified by column chromatography (PE/EA = 20/1-5/1, v/v) to obtain compound **9h** (150 mg, yield: 70%).

LC-MS (ESI): m/z =296.1 [M+H]⁺

### Step 8:

### (1R,2S,3S,4R)-ethyl3-((2-chloro-6-(difluoromethyl)pyrrolo[2,1-f][1,2,4]triazin-4-yl)amino)bicyclo[2.2.2]octane-2-carboxylate (9i)

At room temperature, compound **9h** (0.15 g, 0.51 mmol), ethyl (1R,2S,3S,4R)-3-aminobicyclo[2.2.2]octane-2-carboxylate hydrochloride (0.18 g, 0.77 mmol) and DIPEA (0.16 g, 1.3 mmol) were successively added to NMP (5 mL). The mixture was reacted at 120°C for 2 hours. The reaction solution was poured into water (50 mL) and extracted with EA (50 mL × 1). The organic phase was concentrated to dryness, and the residue was purified by column chromatography (PE/EA = 10/1-2/1, v/v) to obtain compound **9i** (100 mg, yield: 49%).

LC-MS (ESI): m/z =399.1 [M+H]⁺

### Step 9:

### (1R,2S,3S,4R)-ethyl-3-((6-(difluoromethyl)-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (9j)

At room temperature, compound **9i** (100 mg, 0.25 mmol) was added to a mixed solution of dioxane (5 mL) and water (0.5 mL), and 5-fluoro-3-(4,4,5,5-tetramethyl-1,3,2-dioxaborolan-2-yl)-1-tosyl-1H-pyrrolo[2,3-b]pyridine (160 mg, 0.38 mmol), potassium carbonate (87 mg, 0.63 mmol) and Pd(dppf)Cl₂ (18 mg, 0.025 mmol) were successively added to the mixture. The mixture was subjected to nitrogen replacement and protection, heated to 120°C under microwave and reacted for 1 hour. The reaction solution was concentrated to dryness, and the residue was purified by column chromatography (PE/EA = 10/1-1/1, v/v) to obtain compound **9j** (40 mg, yield: 25%).

### Step 10:

### (1R,2S,3 S,4R)-3-((6-(difluoromethyl)-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 9)

At room temperature, to compound **9j** (30 mg, 0.046 mmol) were successively added ethanol (1 mL), water (2 mL) and sodium hydroxide (37 mg, 0.92 mmol). The mixture was reacted at room temperature for 18 hours. The reaction solution was adjusted to pH 5-6 by adding 6 N hydrochloric acid, and concentrated to dryness. The residue was separated and purified by a preparative plate (DCM/MeOH, 10/1, v/v) to obtain **compound 9** (9 mg, yield: 41%).

LC-MS (ESI): m/z =470.1[M+H]⁺.

¹H NMR (400 MHz,DMSO-d6) δ12.24 (s,1H), 8.53-8.50 (m,1H), 8.30-8.29 (m,1H), 8.22-8.21 (m,1H), 8.22-8.10 (m,1H), 7.98-7.96 (m,1H), 7.28-7.00 (m,2H), 4.82-4.79 (m,1H),, 2.76-2.74 (m,1H), 2.03-2.01 (m,2H), 1.81-1.71 (m,3H), 1.64-1.56 (m,3H), 1.50-1.40 (m,2H).

### Example 10: (1R,2S,3S,4R)-3-((6-ethynyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 10)

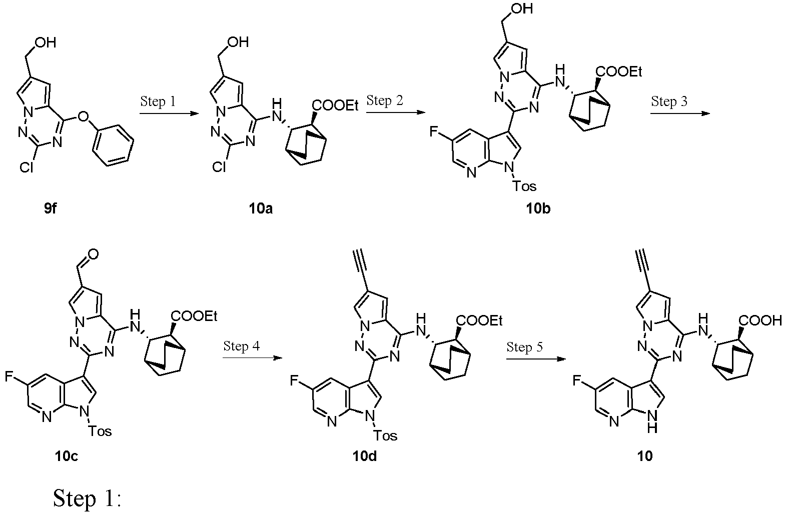

### (1R,2S,3 S,4R)-ethyl3-((2-chloro-6-(hydroxymethyl)pyrrolo[2,1-f][1,2,4]triazin-4-yl)amino)bicyclo[2.2.2]octane-2-carboxylate (10a)

At room temperature, compound **9f** (0.5 g, 1.8 mmol), ethyl (1R,2S,3S,4R)-3-aminobicyclo[2.2.2]octane-2-carboxylate hydrochloride (0.5 g, 2.2 mmol) and DIPEA (0.6 g, 4.6 mmol) were successively added to NMP (10 mL). The mixture was reacted at 120°C for 2 hours. The reaction solution was poured into water (50 mL) and extracted with EA (50 mL × 1). The organic phase was concentrated to dryness, and the residue was purified by column chromatography (PE/EA = 10/1-1/1, v/v) to obtain compound **10a** (500 mg, yield: 73%).

LC-MS (ESI): m/z =379.2 [M+H]⁺

### Step 2:

### (1R,2S,3 S,4R)-ethyl-3-((2-(5-fluoro-1-tosyl-1H-pyrrolo[2,3-b]pyridin-3-yl)-6-(hydroxymethyl)pyrrolo[2,1-f][1,2,4]triazin-4-yl)amino)bicyclo[2.2.2]octane-2-carboxylate (10b)

At room temperature, compound **10a** (500 mg, 1.3 mmol) was added to a mixed solution of dioxane (10 mL) and water (1 mL). 5-fluoro-3-(4,4,5,5-tetramethyl-1,3,2-dioxaborolan-2-yl)-1-tosyl-1H-pyrrolo[2,3-b]pyridine (800 mg, 2.0 mmol), potassium carbonate (550 mg, 4.0 mmol) and Pd(dppf)Cl₂ (100 mg, 0.13 mmol) were successively added, and the mixture was subjected to nitrogen replacement and protection, heated to 120°C under microwave and reacted for 1 hour. The reaction solution was concentrated to dryness, and the residue was purified by column chromatography (PE/EA = 10/1-1/1, v/v) to obtain compound **10b** (300 mg, yield: 36%).

### Step 3:

### (1R,2S,3 S,4R)-ethyl-3-((2-(5-fluoro-1-tosyl-1H-pyrrolo[2,3-b]pyridin-3-yl)-6-formylpyrrolo[2,1-f][1,2,4]triazin-4-yl)amino)bicyclo[2.2.2]octane-2-carboxylate (10c)

At room temperature, compound **10b** (0.3 g, 0.47 mmol) and manganese dioxide (0.4 g, 4.7 mmol) were added to dichloromethane (10 mL), and the mixture was reacted at room temperature for 16 hours. The reaction solution was filtered. The filtrate was concentrated to dryness, and the residue was purified by column chromatography (PE/EA = 10/1-1/1, v/v) to obtain compound **10c** (200 mg, yield: 67%).

### Step 4:

### (1R,2S,3S,4R)-ethyl-3-((6-ethynyl-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (10d)

At room temperature, compound **10c** (0.2 g, 0.32 mmol) was added to methanol (3 mL) and tetrahydrofuran (1 mL). At room temperature, potassium carbonate (0.18 g, 1.3 mmol) and dimethyl (1-diazo-2-oxopropyl)phosphonate (0.12 g, 0.64 mmol) were added, and the mixture was reacted for 16 hours. The reaction solution was poured into water (50 mL) and extracted with EA (50 mL × 1). The organic phase was concentrated to dryness, and the residue was purified by column chromatography (PE/EA = 10/1-1/1, v/v) to obtain compound **10d** (60 mg, yield: 30%).

### Step 5:

### (1R,2S,3 S,4R)-3-((6-ethynyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 10)

At room temperature, to compound **10d** (60 mg, 0.1 mmol) were successively added ethanol (1 mL), water (2 mL) and sodium hydroxide (77 mg, 2 mmol). The mixture was reacted at room temperature for 18 hours. The reaction solution was adjusted to pH 5-6 by adding 6 N hydrochloric acid, and concentrated to dryness. The residue was separated and purified by column chromatography (PE/EA = 10/1-1/2, v/v) to obtain **compound 10** (20 mg, yield: 46%).

LC-MS (ESI): m/z =445.1[M+H]⁺.

¹H NMR (400 MHz,DMSO-d6) δ12.32 (s,1H), 12.24 (s,1H), 8.51-8.48 (m,1H), 8.29-8.28 (m,1H), 8.20-8.19 (m,1H),7.98-7.97 (m,1H), 7.94-7.93 (m,1H), 7.13-7.12 (m,1H), 4.81-4.77 (m,1H), 4.03 (s,1H), 2.74-2.72 (m,1H), 2.03-2.01 (m,2H), 1.80-1.74 (m,3H), 1.62-1.58 (m,3H), 1.46-1.42 (m,2H).

### Example 11: (1R,2S,3S,4R)-3-((7-(difluoromethyl)-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 11)

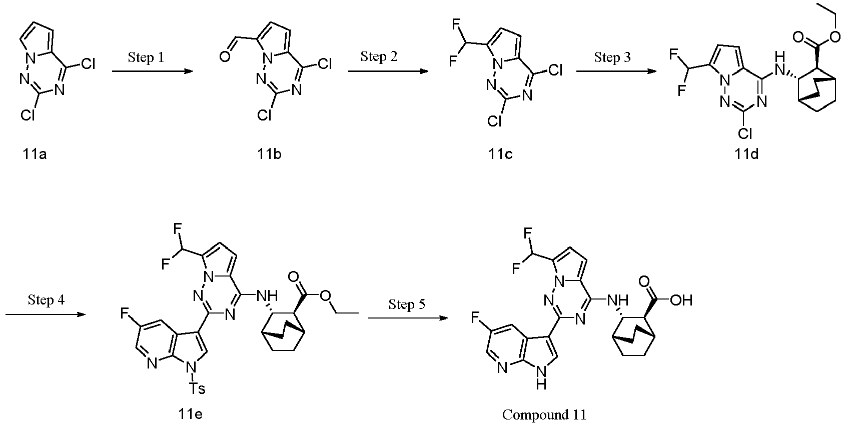

### Step 1:

### 2,4-dichloropyrrolo[2,1-f][1,2,4]triazine-7-carbaldehyde (11b)

DMF (19.3 g, 0.265 mol) was added to a three-necked flask (250 ml), and the mixture was subjected to N₂ replacement and cooled to 0°C. Phosphorus oxychloride (81.4 g, 0.53 mol) was added dropwise, and the mixture was stirred at 0°C for 15 min. (11a) (10 g, 0.053 mol) was added, and the mixture was warmed to 95°C and reacted for 5 h. The mixture was cooled to room temperature. The reaction solution was slowly poured into water (500 ml) and extracted with ethyl acetate (200 ml*3). The organic phases were combined, washed with an aqueous sodium bicarbonate solution and then a saturated sodium chloride solution, dried over anhydrous sodium sulphate and filtered. The filtrate was concentrated, and the residue was separated and purified by silica gel column chromatography (petroleum ether: dichloromethane (v/v) = 1 : 0-1 : 3) to obtain compound (**11b**) (7 g, yield: 61%).

### Step 2:

### 2,4-dichloro-7-(difluoromethyl)pyrrolo[2,1-f][1,2,4]triazine (11c)

(**11b**) (1 g, 4.6 mmol) was added to DCM (15 ml). Diethylaminosulphur trifluoride (3.7 g, 23 mmol) was added dropwise at 0°C, and the mixture was reacted at room temperature for 2 h. The reaction solution was added dropwise to ice water (30 ml), and the mixture was extracted with DCM (15 ml*2). The organic phases were combined, washed with a saturated sodium chloride solution, dried over anhydrous sodium sulphate and filtered. The filtrate was concentrated under reduced pressure to obtain (**11c**) (1.16 g).

### Step 3:

### (1R,2S,3 S,4R)-ethyl3-((2-chloro-7-(difluoromethyl)pyrrolo[2,1-f][1,2,4]triazin-4-yl)amino)bicyclo[2.2.2]octane-2-carboxylate (11d)

(**11c**) (340 mg, 1.43 mmol) and ethyl (1R,2S,3S,4R)-3-aminobicyclo[2.2.2]octane-2-carboxylate (350 mg, 1.5 mmol) were added to DCM (10 mL). DIPEA (53 mg, 4.3 mmol) was added, and the mixture was reacted at room temperature for 1 h. Water (20 mL) was added, and the mixture was extracted with DCM (15 ml*2). The organic phases were combined, washed with an aqueous hydrochloric acid solution (1 mol/L) and then a saturated sodium chloride solution, dried over anhydrous sodium sulphate and filtered. The filtrate was concentrated to obtain crude compound (**11d**) (560 mg).

LC-MS (ESI): m/z =399.1 [M+H]⁺.

### Step 4: (1R,2S,3S,4R)-ethyl3-((7-(difluoromethyl)-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (11e)

(**11d**) (560 mg, 1.4 mmol), intermediate 1 (700 mg, 1.68 mmol), Pd(dppf)Cl₂ (114 mg, 0.14 mmol) and potassium carbonate (400 mg, 2.8 mmol) were added to dioxane (10 mL). Then water (0.5 ml) was added, and the mixture was subjected to N₂ replacement, then warmed to 120°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**11e**) (370 mg, yield: 40%).

LC-MS (ESI): m/z =653.2 [M+H]⁺.

Step 5: (1R,2S,3S,4R)-3-((7-(difluoromethyl)-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (**compound 11**) At room temperature, (**11e**) (170 mg, 0.26 mmol) and LiOH.H₂O (110 mg, 2.6 mmol) were added to THF (3 ml). Then ethanol (3 ml) and water (1 ml) were added, and the mixture was warmed to 60°C and reacted for 2 h. The mixture was cooled to room temperature, adjusted to pH = 5-6 with dilute hydrochloric acid and extracted with EA (15 ml*3). The organic phases were combined, washed with a saturated sodium chloride solution, dried over anhydrous sodium sulphate and filtered. The filtrate was concentrated under reduced pressure, and then the obtained crude product was separated and purified by preparative HPLC to obtain **compound 11** (5 mg, 4%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for **compound 11:** 14.2 min;
¹H NMR (400 MHz, DMSO-d6) δ 12.26 (m, 2H), 8.49-8.46 (m, 1H), 8.32 - 8.29 (m, 1H), 8.24 (d, 1H), 8.13 (d, 1H), 7.59 (t, 1H), 7.05 (d, 1H), 6.90 (d, 1H), 4.80 (t, 1H), 2.76 (d, 1H), 2.03 (s, 2H), 1.85 - 1.70 (m, 3H), 1.66 - 1.52 (m, 3H), 1.49-1.40(m, 2H).

LC-MS (ESI): m/z =471.2[M⁺H]⁺.

### Example 12: (1R,2S,3S,4R)-3-((2-(5-fluoro-1H-pyrrolo[2,3-b]pyridin-3-yl)-7-vinylpyrrolo[2,1-f][1,2,4]triazin-4-yl)amino)bicyclo[2.2.2]octane-2-carboxylic acid (compound 12)

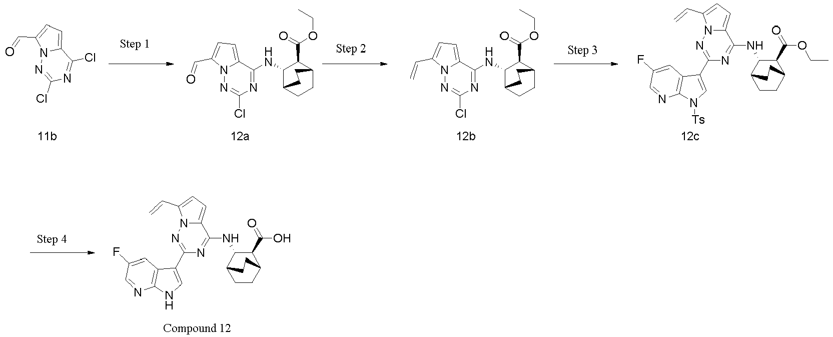

### Step 1:

### (1R,2S,3S,4R)-ethyl3-((2-chloro-7-formylpyrrolo[2,1-f][1,2,4]triazin-4-yl)amino)bicyclo[2.2.2]octane-2-carboxylate (12a)

(**11b**) (500 mg, 2.32 mmol) and ethyl (1R,2S,3S,4R)-3-aminobicyclo[2.2.2]octane-2-carboxylate hydrochloride (568 mg, 2.43 mmol) were added to DCM (10 mL). DIPEA (895 mg, 6.95 mmol) was added, and the mixture was reacted at room temperature for 1 h. Water (20 mL) was added, and the mixture was extracted with DCM (15 ml*2). The organic phases were combined, washed with an aqueous hydrochloric acid solution (1 mol/L) and then a saturated sodium chloride solution, dried over anhydrous sodium sulphate and filtered. The filtrate was concentrated, and then the residue was separated and purified by silica gel column chromatography (petroleum ether: ethyl acetate (v/v) = 10 : 1-1 : 9) to obtain compound (**12a**) (700 mg, yield: 80%).

LC-MS (ESI): m/z =377.1 [M+H]+.

### Step 2:

### (1R,2S,3S,4R)-ethyl3-((2-chloro-7-vinylpyrrolo[2,1-f][1,2,4]triazin-4-yl)amino)bicyclo[2.2.2]octane-2-carboxylate (12b)

Methyltriphenylphosphonium bromide (2 g, 5.59 mmol) was added to THF (10 ml). A solution of potassium tert-butoxide in tetrahydrofuran (1 mol/L, 5.6 ml) was added dropwise at 0°C, and the mixture was reacted at 0°C for 30 min. Then (**12a**) (700 mg, 1.86 mmol) was added, and the mixture was warmed to room temperature and reacted for 1 h. An aqueous ammonium chloride solution was added, and the mixture was extracted with EA (30 ml*3). The organic phases were combined, washed with a saturated sodium chloride solution, dried over anhydrous sodium sulphate and filtered. The filtrate was concentrated, and the residue was separated and purified by silica gel column chromatography (petroleum ether: ethyl acetate (v/v) = 10 : 1-1 : 9) to obtain compound (**12b**) (500 mg, yield: 76%).

LC-MS (ESI): m/z =375.2 [M+H]+.

### Step 3:

### (1R,2S,3S,4R)-ethyl3-((2-(5-fluoro-1-tosyl-1H-pyrrolo[2,3-b]pyridin-3-yl)-7-vinylpyrrolo[2,1-f][1,2,4]triazin-4-yl)amino)bicyclo[2.2.2]octane-2-carboxylate (12c)

(**12b**) (500 mg, 1.34 mmol), intermediate 1 (667 mg, 1.6 mmol), Pd(dppf)Cl₂ (114 mg, 0.14 mmol) and potassium carbonate (370 mg, 2.68 mmol) were added to dioxane (10 mL). Then water (0.5 ml) was added, and the mixture was subjected to N2 replacement, then warmed to 110°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**12c**) (240 mg, yield: 29%).

### Step 4: (1R,2S,3S,4R)-3-((2-(5-fluoro-1H-pyrrolo[2,3-b]pyridin-3-yl)-7-vinylpyrrolo[2,1-f][1,2,4]triazin-4-yl)amino)bicyclo[2.2.2]octane-2-carboxylic acid (compound 12)

At room temperature, (**12c**) (240 mg, 0.38 mmol) and LiOH·H2O (160 mg, 3.8 mmol) were added to THF (3 ml). Then ethanol (3 ml) and water (1 ml) were added, and the mixture was warmed to 60°C and reacted for 2 h. The mixture was cooled to room temperature, adjusted to pH = 5-6 with dilute hydrochloric acid and extracted with EA (15 ml*3). The organic phases were combined, washed with a saturated sodium chloride solution, dried over anhydrous sodium sulphate and filtered. The filtrate was concentrated under reduced pressure, and then the obtained crude product was separated and purified by preparative HPLC to obtain **compound 12** (40 mg, 24%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for compound 12: 14.5 min;
¹H NMR (400 MHz, DMSO-d6) δ 12.23 (d, 2H), 8.46 (dd, 1H), 8.30-8.29 (m, 1H), 8.24 (d, 1H), 7.89 (d, 1H), 7.26 (dd, 1H), 7.02 (d, 1H), 6.89 (d, 1H), 5.97 (dd, 1H), 5.36 (dd, 1H), 4.81 (t, 1H), 2.77 (d, 1H), 2.03 (s, 2H), 1.82-1.74(m, 3H), 1.64 - 1.55 (m, 3H), 1.49-1.42 (m, 2H).

LC-MS (ESI): m/z =447.2[M +H]+.

### Example 13: (1R,2S,3S,4R)-3-((2-(5-fluoro-1H-pyrrolo[2,3-b]pyridin-3-yl)-7-((methylamino)methyl)pyrrolo[2, 1-f] [1,2,4]triazin-4-yl)amino)bicyclo[2.2.2]octane-2-carboxylic acid (compound 13)

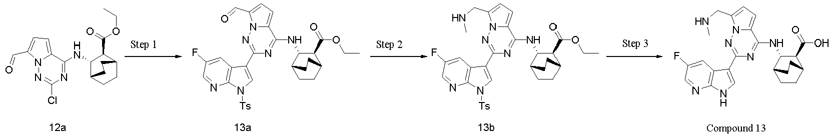

### Step 1:

### (1R,2S,3 S,4R)-ethyl3-((2-(5-fluoro-1-tosyl-1H-pyrrolo[2,3-b]pyridin-3-yl)-7-formylpyrrolo[2,1-f][1,2,4]triazin-4-yl)amino)bicyclo[2.2.2]octane-2-carboxylate (13a)

(**12a**) (600 mg, 1.6 mmol), intermediate 1 (730 mg, 1.76 mmol), Pd(dppf)Cl₂ (260 mg, 0.32 mmol) and potassium carbonate (442 mg, 3.2 mmol) were added to dioxane (10 mL). Then water (0.5 ml) was added, and the mixture was subjected to N2 replacement, then warmed to 100°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**13a**) (600 mg, yield: 60%).

### Step 2:

### (1R,2S,3S,4R)-ethyl3-((2-(5-fluoro-1-tosyl-1H-pyrrolo[2,3-b]pyridin-3-yl)-7-((methylamino)methyl)pyrrolo[2,1-f] [1,2,4]triazin-4-yl)amino)bicyclo[2.2.2]octane-2-carboxylate (13b)

(**13a**) (300 mg, 0.476 mmol), methanamine (a methanol solution) (0.2 ml) and acetic acid (0.1 ml) were added to methanol (5 ml), and the mixture was reacted at room temperature for 1 h. Then sodium borohydride (54 mg, 1.543 mmol) was added, and the mixture was reacted at room temperature for 2 h. Water (15 ml) was added, and the mixture was extracted with ethyl acetate (15 ml*3), washed with a saturated sodium chloride solution, dried over anhydrous sodium sulphate and filtered. The filtrate was concentrated under reduced pressure to obtain crude compound (**13b**) (330 mg).

LC-MS (ESI): m/z =646.3 [M+H]+.

### Step 3: (1R,2S,3S,4R)-3-((2-(5-fluoro-1H-pyrrolo[2,3-b]pyridin-3-yl)-7-((methylamino)methyl)pyrrolo[2,1-f] [1,2,4]triazin-4-yl)amino)bicyclo[2.2.2]octane-2-carboxylic acid (compound 13)

At room temperature, (**13b**) (330 mg, 0.51 mmol) and LiOH H2O (214 mg, 5.1 mmol) were added to THF (3 ml). Then ethanol (3 ml) and water (2 ml) were added, and the mixture was warmed to 60°C and reacted for 2 h. The mixture was cooled to room temperature and adjusted to pH = 6-7 with dilute hydrochloric acid. The reaction solution was directly concentrated under reduced pressure, and then the obtained crude product was separated and purified by preparative HPLC to obtain compound 13 (25 mg, 11%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% ammonium acetate); b. gradient elution: the content of mobile phase A: 5%-50%; c. flow rate: 12 mL/min; d. elution time: 20 min.

Retention time for compound 13: 12.2 min;
¹H NMR (400 MHz, DMSO-d6) δ 12.17 (s, 1H), 8.45 (dd, 1H), 8.28 (d, 1H), 8.20 (s, 1H), 7.82-7.80 (m, 1H), 6.94 (d, 1H), 6.52 (d, 1H), 4.78 (t, 1H), 4.06 (s, 2H), 3.33 (s, 2H), 2.75 (d, 1H), 2.36 (s, 3H), 2.02 (s, 2H), 1.86 - 1.70 (m, 3H), 1.64-1.56 (m, 3H), 1.44-1.41 (m, 2H).

LC-MS (ESI): m/z =464.2[M +H]+.

### Example 14: (1R,2S,3S,4R)-3-((7-cyclopropyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 14)

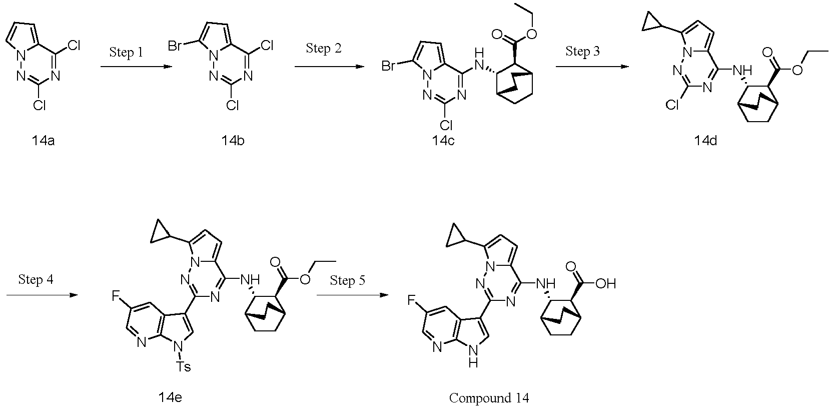

### Step 1:

### 7-bromo-2,4-dichloropyrrolo[2,1-f] [1,2,4]triazine (14b)

(**14a**) (1 g, 5.32 mmol) was added to acetonitrile (15 ml). At 0°C, N-bromosuccinimide (1.04 g, 2.85 mmol) was dissolved in acetonitrile (5 ml) and added dropwise to the reaction system, and the mixture was reacted at room temperature for 16 h. The reaction solution was directly concentrated under reduced pressure, and then the residue was separated and purified by silica gel column chromatography (petroleum ether: ethyl acetate (v/v) = 10 : 1-1 : 9) to obtain compound (**14b**) (1.2 g, yield: 84%).

### Step 2:

### (1R,2S,3S,4R)-ethyl3-((7-bromo-2-chloropyrrolo[2,1-f][1,2,4]triazin-4-yl)amino)bicyclo[2.2.2]octane-2-carboxylate (14c)

(**14b**) (1.2 g, 4.49 mmol) and ethyl (1R,2S,3S,4R)-3-aminobicyclo[2.2.2]octane-2-carboxylate hydrochloride (1.05 g, 4.49 mmol) were added to DCM (10 mL). DIPEA (1.74 g, 13.47 mmol) was added, and the mixture was reacted at room temperature for 1 h. Water (20 mL) was added, and the mixture was extracted with DCM (15 ml*2). The organic phases were combined, washed with an aqueous hydrochloric acid solution (1 mol/L) and then a saturated sodium chloride solution, dried over anhydrous sodium sulphate and filtered. The filtrate was concentrated, and then the residue was separated and purified by silica gel column chromatography (petroleum ether: ethyl acetate (v/v) = 10 : 1-1 : 9) to obtain compound (**14c**) (1.8 g, yield: 94%).

LC-MS (ESI): m/z =427.1 [M+H]+,429.1 [M+H]+.

### Step 3:

### (1R,2S,3S,4R)-ethyl3-((2-chloro-7-cyclopropylpyrrolo[2,1-f][1,2,4]triazin-4-yl)amino)bicyclo[2.2.2]octane-2-carboxylate (14d)

(**14c**) (500 mg, 1.17 mmol), cyclopropylboronic acid (252 mg, 2.93 mmol), Pd(dppf)Cl₂ (190 mg, 0.23 mmol) and potassium carbonate (485 mg, 3.5 mmol) were added to dioxane (10 mL). Then water (0.5 ml) was added, and the mixture was subjected to N₂ replacement, then warmed to 100°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 5) to obtain compound (**14d**) (340 mg, yield: 75%).

LC-MS (ESI): m/z =389.2[M +H]+.

### Step 4:

### (1R,2S,3 S,4R)-ethyl3-((7-cyclopropyl-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (14e)

(**14d**) (300 mg, 0.77 mmol), intermediate 1 (354 mg, 0.85 mmol), Pd(dppf)Cl₂ (122 mg, 0.15 mmol) and K₃PO₄·7H₂O (760 mg, 2.3 mmol) were added to dioxane (10 mL). Then water (0.5 ml) was added, and the mixture was subjected to N₂ replacement, then warmed to 100°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 5) to obtain compound (1R,2S,3S,4R)-ethyl3-((7-cyclopropyl-2-(5-fluoro-1-tosyl-1H-pyrrolo[2,3-b]pyridin-3-yl)pyrrolo [2,1-f][1,2,4]triazin-4-yl)amino)bicyclo[2.2.2]octane-2-carboxylate (**14e**) (350 mg, yield: 71%).

### Step 5: (1R,2S,3S,4R)-3-((7-cyclopropyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 14)

At room temperature, (**14e**) (350 mg, 0.545 mmol) and LiOH·H2O (230 mg, 5.5 mmol) were added to THF (5 ml). Then ethanol (5 ml) and water (2 ml) were added, and the mixture was warmed to 60°C and reacted for 2 h. The mixture was cooled to room temperature and adjusted to pH = 6-7 with dilute hydrochloric acid. The reaction solution was directly concentrated under reduced pressure, and then the obtained crude product was separated and purified by preparative HPLC to obtain compound 14 (110 mg, 44%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for compound 14: 14.5 min;
¹H NMR (400 MHz, DMSO-d6) δ 12.18 (s, 2H), 8.52 (dd, 1H), 8.29 (d, 1H), 8.19 (d, 1H), 7.78 (d, 1H), 6.91 (d, 1H), 6.24 (d, 1H), 4.78 (t, 1H), 2.77 (d, 1H), 2.43-2.37 (m, 1H), 2.02 (s, 2H), 1.81-1.71 (m, 3H), 1.67 - 1.51 (m, 3H), 1.48-1.38 (m, 2H), 1.10 - 1.03 (m, 2H), 0.83-0.79 (m, 2H).

LC-MS (ESI): m/z =461.2[M +H]+.

### Example 15: (1R,2S,3S,4R)-3-((2-(5-fluoro-1H-pyrrolo[2,3-b]pyridin-3-yl)-7-(methylcarbamoyl)pyrrolo[2,1-f][1,2,4]triazin-4-yl)amino)bicyclo[2.2.2]octane-2-carboxylic acid (compound 15)

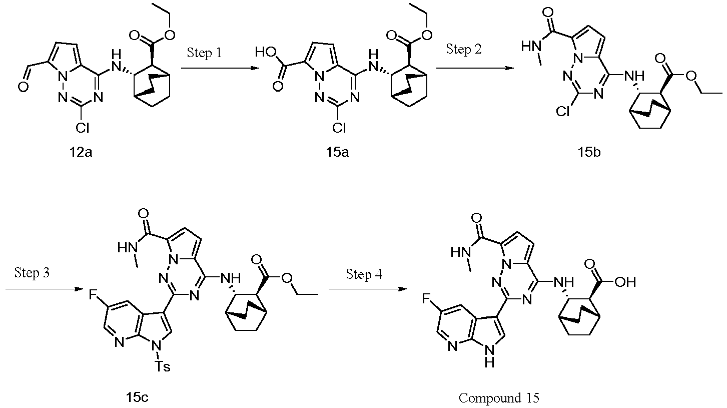

### Step 1:

### 2-chloro-4-(((1R,2S,3S,4R)-3-(ethoxycarbonyl)bicyclo[2.2.2]octan-2-yl)amino)pyrrolo[2,1-f][1,2,4]triazine-7-carboxylic acid (15a)

(**12a**) (900 mg, 2.39 mmol) and 2-methylbut-2-ene (838 mg, 12 mmol) were dissolved in THF (10 ml). Then sodium chlorite (860 mg, 9.56 mmol) and sodium dihydrogen phosphate dihydrate (1.5 g, 9.56 mmol) were dissolved in water (2 ml) and added dropwise to the reaction system, and the mixture was reacted at room temperature for 1 h. An aqueous ammonium chloride solution was added, and the mixture was extracted with EA (20 ml*3). The organic phases were combined, washed with a saturated sodium chloride solution, dried over anhydrous sodium sulphate and filtered. The filtrate was concentrated to obtain crude compound (**15a**) (1 g).

LC-MS (ESI): m/z =392.1 [M+H]+.

### Step 2:

### (1R,2S,3 S,4R)-ethyl3-((2-chloro-7-(methylcarbamoyl)pyrrolo[2,1-f][1,2,4]triazin-4-yl)amino)bicyclo[2.2.2]octane-2-carboxylate (15b)

At room temperature, to (**15a**) (500 mg, 1.276 mmol) were successively added methylamine hydrochloride (258 mg, 3.83 mmol), DCM (10 mL), HATU (970 mg, 2.55 mmol) and N,N-diisopropylethylamine (823 mg, 6.38 mmol). The mixture was stirred at room temperature for 2 hours, diluted with water and extracted with DCM. The organic phase was washed with saturated brine (20 mL), dried over anhydrous sodium sulphate and concentrated under reduced pressure. The obtained crude product was then separated and purified by silica gel column chromatography (petroleum ether: ethyl acetate (v/v) = 10 : 1-1 : 4) to obtain (**15b**) (400 mg, yield: 77%).

LC-MS (ESI): m/z =406.2 [M+H]+.

### Step 3:

### (1R,2S,3S,4R)-ethyl3-((2-(5-fluoro-1-tosyl-1H-pyrrolo[2,3-b]pyridin-3-yl)-7-(methylcarbamoyl)pyrrolo[2,1-f][1,2,4]triazin-4-yl)amino)bicyclo[2.2.2]octane-2-carboxylate (15c)

(**15b**) (400 mg, 0.99 mmol), intermediate 1 (453 mg, 1.1 mmol), Pd(dppf)Cl₂ (163 mg, 0.2 mmol) and potassium carbonate (414 mg, 3 mmol) were added to dioxane (10 mL). Then water (0.5 ml) was added, and the mixture was subjected to N₂ replacement, then warmed to 100°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**15c**) (300 mg, yield: 46%).

### Step 4: (1R,2S,3S,4R)-3-((2-(5-fluoro-1H-pyrrolo[2,3-b]pyridin-3-yl)-7-(methylcarbamoyl)pyrrolo[2,1-f][1,2,4]triazin-4-yl)amino)bicyclo[2.2.2]octane-2-carboxylic acid (compound 15)

At room temperature, (**15c**) (300 mg, 0.46 mmol) and LiOH·H2O (193 mg, 4.6 mmol) were added to THF (5 ml). Then ethanol (5 ml) and water (2 ml) were added, and the mixture was warmed to 60°C and reacted for 2 h. The mixture was cooled to room temperature and adjusted to pH = 6-7 with dilute hydrochloric acid. The reaction solution was directly concentrated under reduced pressure, and then the obtained crude product was separated and purified by preparative HPLC to obtain **compound 15** (40 mg, 18%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for **compound 15**: 14.2 min;
¹H NMR (400 MHz, DMSO-d6) δ 12.39 (d, 2H), 8.94 (q, 1H), 8.45 (m, 2H), 8.32 (m, 1H), 8.25 (d, 1H), 7.15 (d, 1H), 7.09 (d, 1H), 4.89 (t, 1H), 3.01 (d, 3H), 2.83 (d, 1H), 2.06 (s, 2H), 1.89 - 1.72 (m, 3H), 1.70 - 1.54 (m, 3H), 1.52-1.44 (m, 2H).

LC-MS (ESI): m/z =478.2[M +H]+.

### Example 16:

### (1R,2S,3S,4R)-3-((2-(5-fluoro-1H-pyrrolo[2,3-b]pyridin-3-yl)-5-vinylpyrrolo[2,1-f][1,2,4]triazin-4-yl)amino)bicyclo[2.2.2]octane-2-carboxylic acid (compound 16)

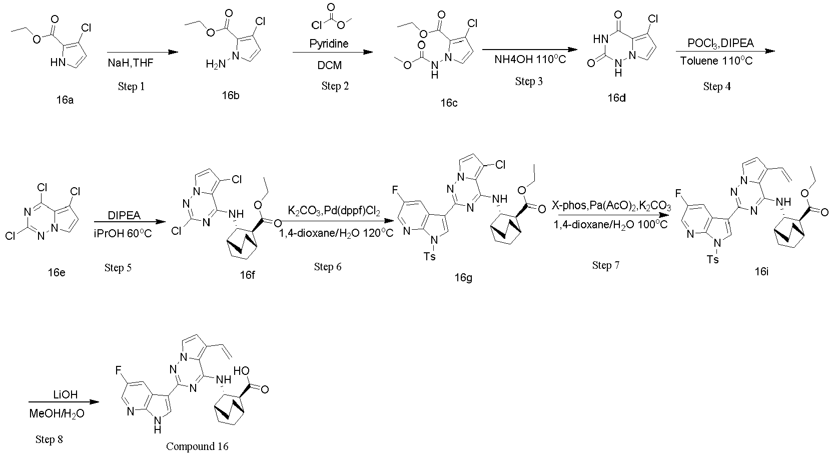

### Step 1:

### ethyl 1-amino-3-chloro-1H-pyrrole-2-carboxylate (16b)

Under nitrogen protection, compound **16a** (5.0 g, 28.8 mmol) was dissolved in tetrahydrofuran (100 ml), and the mixture was cooled to 0-5°C in an ice bath. Sodium hydride (1.4 g, 34.5 mmol, content: 60%) was added in portions and after the addition, the mixture was stirred for 30 minutes while the temperature was controlled at 0-5°C. 2,4-dinitrophenylhydroxylamine (8.6 g, 43.2 mmol) was then added and after the addition, the mixture was warmed to room temperature and stirred for 15 hours. After completion of the reaction, the reaction solution was slowly poured into ice water (150 ml) and extracted with ethyl acetate (150 ml*4). The ethyl acetate phases were combined, washed with saturated brine, dried over anhydrous sodium sulphate and filtered. The filtrate was concentrated under reduced pressure, and then the residue was separated and purified by column chromatography (petroleum ether: ethyl acetate (v/v) = 10 : 1-2 : 1) to obtain compound **16b** (4.1 g, 75%).

LC-MS (ESI): m/z =189.1[M+H]⁺.

### Step 2:

### ethyl 3-chloro-1-((methoxycarbonyl)amino)-1H-pyrrole-2-carboxylate (16c)

Compound **16b** (4.1 g, 21.7 mmol) was dissolved in dichloromethane (100 ml), and pyridine (2.5 g, 32.5 mmol) was added to the mixture. Methyl chloroformate (2.2 g, 23.9 mmol) was added dropwise while the temperature was controlled at 0-5°C and after the addition, the mixture was naturally warmed to room temperature and reacted for 3 hours. After completion of the reaction, ice water (100 ml) was added, and the mixture was subjected to liquid-liquid separation. The aqueous phase was extracted twice with dichloromethane (100 ml*2). The organic phases were combined, washed with saturated brine, dried over anhydrous sodium sulphate and filtered. The filtrate was concentrated under reduced pressure, and then the residue was separated and purified by column chromatography (petroleum ether: ethyl acetate (v/v) = 10 : 1-2 : 1) to obtain compound **16c** (3.5 g, 65%).

LC-MS (ESI): m/z =247.1[M+H]⁺.

### Step 3:

### 5-chloropyrrolo[2,1-f][1,2,4]triazine-2,4(1H,3H)-dione (16d)

To a 120 ml sealed tube were added compound **16c** (3.5 g, 14.2 mmol), isopropanol (20 ml) and ammonia water (60 ml, 28%-30%). After the tube was sealed, the mixture was warmed to 110°C and reacted for 15 hours. After completion of the reaction, the mixture was cooled to room temperature and then concentrated under reduced pressure. The concentrated residue was slurried with a mixed system of diethyl ether and methanol (10 : 1, 20 ml, v/v) and filtered. The filter cake was rinsed with diethyl ether (10 ml) and dried to obtain compound **16d** (1.5 g, 57%).

LC-MS (ESI): m/z =184.1[M-H]⁻.

### Step 4:

### 2,4,5-trichloropyrrolo[2,1-f][1,2,4]triazine (16e)

To a 48 ml sealed tube were added compound **16d** (1.5 g, 8.1 mmol), DIPEA (2.1 g, 16.2 mmol), phosphorus oxychloride (10 ml) and toluene (20 ml). After the addition and the tube was sealed, the mixture was warmed to 110°C and reacted for 23 h. After completion of the reaction, the mixture was cooled to room temperature and concentrated under reduced pressure. The residue was separated and purified by column chromatography (petroleum ether: ethyl acetate (v/v) = 10 : 1-5 : 1) to obtain compound **16e** (810 mg, 45%).

### Step 5:

### ethyl (1R,2S,3S,4R)-3-((2,5-dichloropyrrolo[2,1-f][1,2,4]triazin-4-yl)amino)bicyclo[2.2.2]octane-2-carboxylate (16f)

Compound **16e** (810 mg, 3.6 mmol) was dissolved in isopropanol (15 ml). DIPEA (0.9 g, 7.2 mmol) and ethyl-(2S,3S)-3-aminobicyclo[2.2.2]octane-2-carboxylate hydrochloric acid (950.0 mg, 4.0 mmol) were added and after the addition, the mixture was warmed to 60°C and reacted for 6 hours. After completion of the reaction, the mixture was directly concentrated under reduced pressure, and the residue was separated and purified by column chromatography (petroleum ether: ethyl acetate (v/v) = 10 : 1-3 : 1) to obtain compound **16f** (1.05 g, 80%).

LC-MS (ESI): m/z =383.1[M+H]⁺.

### Step 6:

### ethyl (1R,2S,3S,4R)-3-((5-chloro-2-(5-fluoro-1-tosyl-1H-pyrrolo[2,3-b]pyridine-3-yl)pyrrolo[2,1-f][1,2,4]triazin-4-yl)amino)bicyclo[2.2.2]octane-2-carboxylate(16g)

Compound 16f (800.0 mg, 2.1 mmol) was dissolved in a mixed solvent of 1,4-dioxane (15 ml) and water (1 ml). Intermediate 1 (1050.0 mg, 2.5 mmol), potassium carbonate (870.0 mg, 6.3 mmol) and Pd(dppf)Cl₂ (146.0 mg, 0.2 mmol) were added and after the addition, the mixture was reacted in a microwave reactor at 120°C for 2 hours. After completion of the reaction, the mixture was filtered, and the filtrate was concentrated. The residue was separated and purified by column chromatography (petroleum ether: ethyl acetate (v/v) = 10 : 1-5 : 1) to obtain compound **16g** (880 mg, 67%).

LC-MS (ESI): m/z =636.1[M+H]⁺.

### Step 7: Ethyl (1R,2S,3S,4R)-3-((2-(5-fluoro-1-tosyl-1H-pyrrolo[2,3-b]pyridin-3-yl)-5-vinylpyrrolo[2,1-f][1,2,4]triazin-4-yl)amino)bicyclo[2.2.2]octane-2-carboxylate(16i)

Compound **16g** (350.0 mg, 0.56 mmol) was dissolved in a mixed solvent of 1,4-dioxane (10 ml) and water (1 ml). Ethenyl boronic acid pinacol ester (172.5 mg, 1.12 mmol), X-phos (95.5 mg, 0.2 mmol), palladium acetate (22.5 mg, 0.1 mmol) and potassium carbonate (232.0 mg, 1.7 mmol) were added and after the addition, the mixture was subjected to nitrogen replacement 3 times, heated to 100°C and reacted for 15 hours. After completion of the reaction, the mixture was filtered, and the filtrate was concentrated. The residue was separated and purified by column chromatography (petroleum ether: ethyl acetate (v/v) = 10 : 1-5 : 1) to obtain compound **16i** (180 mg, 50%).

LC-MS (ESI): m/z =629.2[M+H]⁺.

### Step 8: (1R,2S,3S,4R)-3-((2-(5-fluoro-1H-pyrrolo[2,3-b]pyridin-3-yl)-5-vinylpyrrolo[2,1-f][1,2,4]triazin-4-yl)amino)bicyclo[2.2.2]octane-2-carboxylic acid (compound 16)

Compound **16i** (180 mg, 0.28 mmol) was dissolved in methanol (2 ml). Lithium hydroxide (117 mg, 2.8 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound **16** (76.0 mg, 60%).

LC-MS (ESI): m/z =447.2[M+H]⁺.

¹HNMR (400 MHz, DMSO-d6) δ 12.15 (s, 2H), 8.50 (d, 1H), 8.26(s,1H),8.25-8.24 (m, 1H), 8.18 (d, 1H),7.69(d, 1H), 7.27-7.20(m, 1H) , 6.85(d, 1H), 6.61(d, 1H), 5.65(d, 1H), 5.28(d, 1H), 4.83-4.83(m, 1H), 2.90(d, 1H), 2.02(s, 1H), 1.78-1.46(m, 8H).

### Example 17:

### (1R,2S,3 S,4R)-3-((5-cyclopropyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 17)

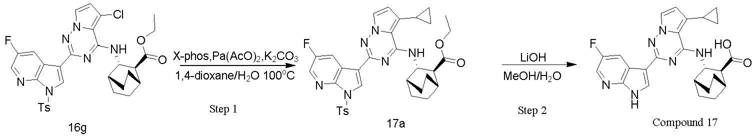

### Step 1: ethyl (1R,2S,3S,4R)-3-((5-cyclopropyl-2-(5-fluoro-1-tosyl-1H-pyrrolo[2,3-b]Pyridine-3-yl)pyrrolo[2,1-f][1,2,4]triazin-4-yl)amino)bicyclo[2.2.2]octane-2-carboxylate (17a)

Compound **16g** (300.0 mg, 0.47 mmol) was dissolved in a mixed solvent of 1,4-dioxane (10 ml) and water (1 ml). Cyclopropylboronic acid (81.5 mg, 0.94 mmol), X-phos (95.5 mg, 0.2 mmol), palladium acetate (22.5 mg, 0.1 mmol) and potassium carbonate (195.0 mg, 1.4 mmol) were added and after the addition, the mixture was subjected to nitrogen replacement 3 times, heated to 100°C and reacted for 15 hours. After completion of the reaction, the mixture was filtered, and the filtrate was concentrated. The residue was separated and purified by column chromatography (petroleum ether: ethyl acetate (v/v) = 10 : 1-5 : 1) to obtain compound **17a** (130 mg, 43%).

LC-MS (ESI): m/z =643.2[M+H]⁺.

### Step 2: (1R,2S,3S,4R)-3-((5-cyclopropyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 17)

Compound **17a** (130 mg, 0.20 mmol) was dissolved in methanol (5 ml). Lithium hydroxide (84.0 mg, 2.0 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound **17** (38.0 mg, 41%).

LC-MS (ESI): m/z =461.2[M+H]⁺.

¹H NMR (400 MHz, DMSO-d6) δ 12.15 (s, 2H), 8.50 (d, 1H), 8.26(s,1H),8.25-8.24 (m, 1H), 8.18 (d, 1H),7.69(d, 1H), 7.27-7.20(m, 1H) , 6.85(d, 1H), 6.61(d, 1H), 5.65(d, 1H), 5.28(d, 1H), 4.83-4.83(m, 1H), 2.90(d, 1H), 2.02(s, 1H), 1.78-1.46(m, 8H).

### Example 18:

### (1R,2S,3 S,4R)-3-((2-(5-fluoro-1H-pyrrolo[2,3-b]pyridin-3-yl)-7-(trifluoromethyl)pyrrolo[2,1-f][1,2,4]triazin-4-yl)amino)bicyclo[2.2.2]octane-2-carboxylic acid (compound 18)

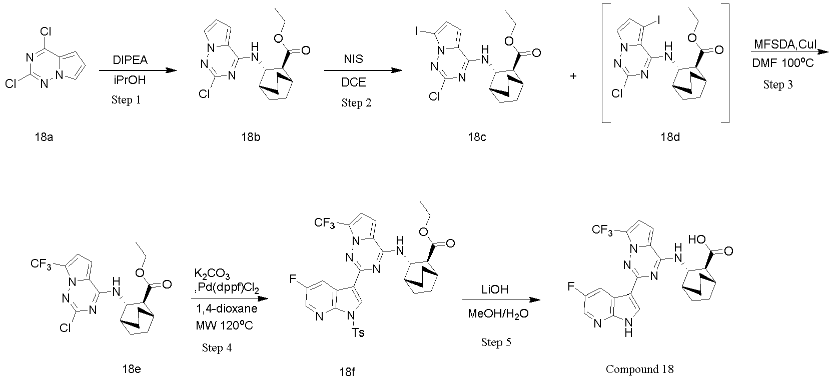

### Step 1:

### ethyl (2S,3S)-3-((2-chloropyrrolo[2,1-f][1,2,4]triazin-4-yl)amino)bicyclo[2.2.2]octane-2-carboxylate (18b)

Compound **18a** (1.0 g, 5.4 mmol) was dissolved in isopropanol (10 ml). DIPEA (2.1 g, 16.2 mmol) and ethyl (2S,3S)-3-aminobicyclo[2.2.2]octane-2-carboxylate hydrochloric acid (1.38 g, 5.9 mmol) were added, and the mixture was warmed to 60°C and reacted for 3 hours. After completion of the reaction, the mixture was directly concentrated, and the concentrated residue was separated and purified by column chromatography (petroleum ether: ethyl acetate (v/v) = 10 : 1-5 : 1) to obtain compound **18b** (1.6 g, 85%).

LC-MS (ESI): m/z =349.2[M+H]⁺.

### Step 2:

### ethyl (1R,2S,3S,4R)-3-((2-chloro-7-iodopyrrolo[2,1-f][1,2,4]triazin-4-yl)amino)bicyclo[2.2.2]octane-2-carboxylate(18c)

### ethyl (1R,2S,3S,4R)-3-((2-chloro-5-iodopyrrolo[2,1-f][1,2,4]triazin-4-yl)amino)bicyclo[2.2.2]octane-2-carboxylate(18d)

Under nitrogen protection, compound **18b** (1.6 g, 4.6 mmol) was dissolved in DCE (20 ml), and the mixture was cooled to 0°C. NIS (1.14 g, 5.1 mmol) was added in portions and after the addition, the mixture was naturally warmed to room temperature and reacted for 3 hours. After completion of the reaction, water (20 ml) was added, and the mixture was extracted with dichloromethane (50 ml*2). The organic phases were combined, washed with saturated brine (20 ml), dried over anhydrous sodium sulphate, filtered and concentrated under reduced pressure. The residue was separated and purified by column chromatography (petroleum ether: ethyl acetate (v/v) = 10 : 1-1 : 1) to obtain compound **18c** (620 mg, 28%) and compound **18d** (1.25 g, 59%).

Compound **18c:** LC-MS (ESI): m/z =475.1[M+H]⁺.

Compound **18c:** ¹H NMR (400 MHz, CDCl₃) δ 6.81 (s, 1H),6.70 (s, 1H), 5.47(s, 1H), 4.68(s,1H), 4.24-4.17 (m, 2H), 2.45 (s, 1H),2.05-2.04(m, 1H) , 1.96-1.95(m, 1H), 1.83-1.58(m, 7H), 1.48-1.43(m, 1H), 1.27-1.24(m, 3H).

Compound **18d:** LC-MS (ESI): m/z =475.1[M+H]⁺.

Compound **18d:** ¹H NMR (400 MHz, CDCl₃) δ 7.42 (d, 1H),7.04 (s, 1H), 6.70(d, 1H), 4.67-4.65(m,1H), 4.26-4.21 (m, 2H), 2.47-2.45 (m, 1H), 2.04-2.01(m, 1H) , 1.96-1.94(m, 1H), 1.85-1.62(m, 7H), 1.48-1.43(m, 1H), 1.30-1.26(m, 3H).

### Step 3:

### ethyl (1R,2S,3S,4R)-3-((2-chloro-7-(trifluoromethyl)pyrrolo[2,1-f][1,2,4]triazin-4-yl)amino)bicyclo[2.2.2]octane-2-carboxylate (18e)

Under nitrogen protection, compound **18c** (620 mg, 1.3 mmol) was dissolved in DMF (10 ml). Cuprous iodide (380 mg, 2.0 mmol) and methyl fluorosulfonyldifluoroacetate (1.25 g, 6.5 mmol) were added and after the addition, the mixture was warmed to 100°C and reacted for 5 hours. After completion of the reaction, the mixture was filtered. To filtrate was added water (30 ml), and the mixture was extracted with ethyl acetate (100 ml*2). The organic phases were combined, washed with saturated brine (20 ml), dried over anhydrous sodium sulphate, filtered and concentrated under reduced pressure. The residue was separated and purified by column chromatography (petroleum ether: ethyl acetate (v/v) = 10 : 1-3 : 1) to obtain compound **18e** (420 mg, 77%).

LC-MS (ESI): m/z =417.2[M+H]⁺.

### Step 4:

### ethyl (1R,2S,3S,4R)-3-((2-(5-fluoro-1-tosyl-1H-pyrrolo[2,3-b]pyridin-3-yl)-7-(trifluoromethyl)pyrrolo[2,1-f][1,2,4]triazin-4-yl)amino)bicyclo[2.2.2]octane-2-carboxylate (18f)

Under nitrogen protection, compound **18e** (420 mg, 1.0 mmol) was dissolved in a mixed solvent of 1,4-dioxane and water (10 ml/0.5 ml, v/v). Then potassium carbonate (414 mg, 3.0 mmol), Pd(dppf)Cl₂ (73 mg, 0.1 mmol) and intermediate 1 (625.0 mg, 1.5 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **18f** (180 mg, 29%).

### Step 5:

### (1R,2S,3 S,4R)-3-((2-(5-fluoro-1H-pyrrolo[2,3-b]pyridin-3-yl)-7-(trifluoromethyl)pyrrolo[2,1-f][1,2,4]triazin-4-yl)amino)bicyclo[2.2.2]octane-2-carboxylic acid (compound 18)

Compound 18f (180 mg, 0.27 mmol) was dissolved in methanol (5 ml). Lithium hydroxide (113.0 mg, 2.7 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound 18 (53.0 mg, 40%).

LC-MS (ESI): m/z =489.2[M+H]⁺.

¹H NMR (400 MHz, DMSO-d6) δ 12.32 (d, 2H), 8.41-8.36 (m, 1H), 8.35(d,1H),8.32-8.31 (m, 1H), 8.24 (d, 1H),7.45(d, 1H), 7.10(d, 1H), 4.81-4.78(m, 1H), 2.79(d, 1H), 2.02(s, 2H), 1.82-1.42 (m, 8H).

### Example 19:

### (1R,2S,3 S,4R)-3-((2-(5-fluoro-1H-pyrrolo[2,3-b]pyridin-3-yl)-5-(trifluoromethyl)pyrrolo[2,1-f][1,2,4]triazin-4-yl)amino)bicyclo[2.2.2]octane-2-carboxylic acid (compound 19)

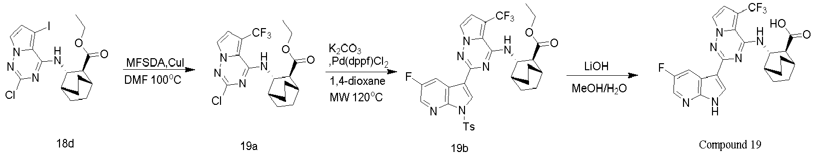

### Step 1:

### ethyl (1R,2S,3 S,4R)-3-((2-chloro-5-(trifluoromethyl)pyrrolo[2,1-f][1,2,4]triazin-4-yl)amino)bicyclo[2.2.2]octane-2-carboxylate (19a)

Under nitrogen protection, compound **18d** (530 mg, 1.11 mmol) was dissolved in DMF (10 ml). Cuprous iodide (315 mg, 1.65 mmol) and methyl fluorosulfonyldifluoroacetate (1.05 g, 5.55 mmol) were added and after the addition, the mixture was warmed to 100°C and reacted for 5 hours. After completion of the reaction, the mixture was filtered. To filtrate was added water (30 ml), and the mixture was extracted with ethyl acetate (100 ml*2). The organic phases were combined, washed with saturated brine (20 ml), dried over anhydrous sodium sulphate, filtered and concentrated under reduced pressure. The residue was separated and purified by column chromatography (petroleum ether: ethyl acetate (v/v) = 10 : 1-3 : 1) to obtain compound **19a** (380 mg, 82%).

LC-MS (ESI): m/z =417.2[M+H]⁺.

### Step 2:

### ethyl (1R,2S,3S,4R)-3-((2-(5-fluoro-1-tosyl-1H-pyrrolo[2,3-b]pyridin-3-yl)-5-(trifluoromethyl)pyrrolo[2,1-f][1,2,4]triazin-4-yl)amino)bicyclo[2.2.2]octane-2-carboxylate (19b)

Under nitrogen protection, compound 19a (380 mg, 0.91 mmol) was dissolved in a mixed solvent of 1,4-dioxane and water (10 ml/0.5 ml, v/v). Then potassium carbonate (377 mg, 2.73 mmol), Pd(dppf)Cl₂ (73 mg, 0.1 mmol) and intermediate **int-1** (570.0 mg, 1.37 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **19b** (160 mg, 26%).

### Step 3: (1R,2S,3S,4R)-3-((2-(5-fluoro-1H-pyrrolo[2,3-b]pyridin-3-yl)-5-(trifluoromethyl)pyrrolo[2,1-f][1,2,4]triazin-4-yl)amino)bicyclo[2.2.2]octane-2-carboxylic acid (compound 19)

Compound **19b** (160 mg, 0.24 mmol) was dissolved in methanol (5 ml). Lithium hydroxide (101.0 mg, 2.4 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound **19** (45.0 mg, 38%).

LC-MS (ESI): m/z =489.2[M+H]⁺.

¹H NMR (400 MHz, DMSO-d6) δ 12.37 (d, 2H), 8.53-8.50 (m, 1H), 8.33-8.31(m,2H), 7.98 (d, 1H), 7.09(d, 1H), 6.10-6.08 (m, 1H), 4.81(s,1H), 2.48(d, 1H), 1.99(s, 2H), 1.68-1.46(m, 8H).

### Example 20:

### (1R,5S,6S,7S)-7-((2-(5-fluoro-1H-pyrrolo[2,3-b]pyridin-3-yl)-7-(methoxymethyl)pyrrolo[2,1-f][1,2,4]triazin-4-yl)amino)tricyclo[3.2.2.0^{2,4}]nonane-6-carboxylic acid (compound 20)

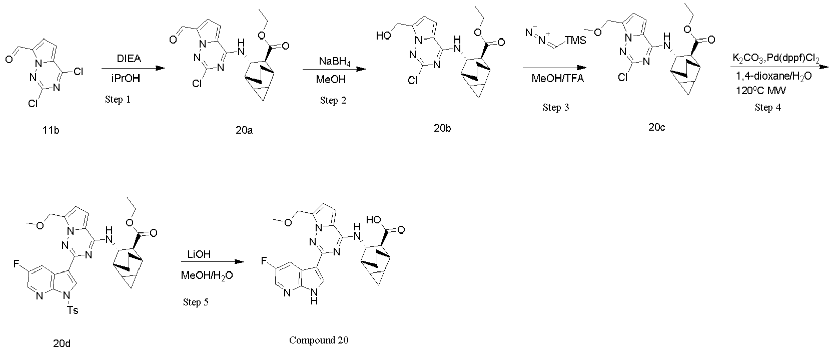

### Step 1:

### ethyl (1R,5S,6S,7S)-7-((2-chloro-7-formylpyrrolo[2,1-f][1,2,4]triazin-4-yl)amino)tricyclo[3.2.2.0^{2,4}]nonane-6-carboxylate (20a)

Compound **11b** (1.0 g, 4.6 mmol) was dissolved in isopropanol (10 ml). DIPEA (1.78 g, 13.8 mmol) and ethyl (1R,5S,6S,7S)-7-aminotricyclo[3.2.2.0^{2,4}]nonane-6-carboxylate (1.14 g, 4.6 mmol) were added, and the mixture was warmed to 60°C and reacted for 3 hours. After completion of the reaction, the mixture was directly concentrated, and the concentrated residue was separated and purified by column chromatography (petroleum ether: ethyl acetate (v/v) = 10 : 1-3 : 1) to obtain compound **20a** (1.3 g, 71%).

LC-MS (ESI): m/z =389.2[M+H]⁺.

### Step 2:

### ethyl (1R,5S,6S,7S)-7-((2-chloro-7-(hydroxymethyl)pyrrolo[2,1-f][1,2,4]triazin-4-yl)amino)tricyclo[3.2.2.0^{2,4}]nonane-6-carboxylate (20b)

Compound **20a** (1.3 g, 3.3 mmol) was dissolved in methanol (10 ml), and the mixture was cooled to 0°C. Sodium borohydride (166 mg, 4.0 mmol) was added and after the addition, the mixture was warmed to room temperature and reacted for 30 minutes. After completion of the reaction, water (20 ml) was added, and the mixture was extracted twice with dichloromethane (100 ml*2). The organic layers were combined, dried over anhydrous sodium sulphate, filtered and concentrated under reduced pressure to obtain compound **20b** (1.1 g, 84%).

LC-MS (ESI): m/z =391.2[M+H]⁺.

### Step 3:

### ethyl (1R,5S,6S,7S)-7-((2-chloro-7-(methoxymethyl)pyrrolo[2,1-f][1,2,4]triazin-4-yl)amino)tricyclo[3.2.2.0^{2,4}]nonane-6-carboxylate (20c)

Compound **20b** (1.1 g, 2.8 mmol) was dissolved in methanol (10 ml) and trifluoroacetic acid (2 ml). At room temperature, trimethylsilyldiazomethane (14 ml, 28 mmol, 2.0 mol/L n-hexane solution) was slowly added dropwise and after the dropwise addition, the mixture was reacted for another 30 minutes. After completion of the reaction, the reaction solution was added to a saturated sodium bicarbonate solution (50 ml), and the mixture was extracted twice with ethyl acetate (100 ml*2). The organic layers were combined, dried over anhydrous sodium sulphate, filtered and concentrated. The residue was separated and purified by column chromatography (petroleum ether: ethyl acetate (v/v) = 10 : 1-3 : 1) to obtain compound **20c** (850 mg, 75%).

LC-MS (ESI): m/z =405.2[M+H]⁺.

### Step 4:

### ethyl (1R,5S,6S,7S)-7-((2-(5-fluoro-1-tosyl-1H-pyrrolo[2,3-b]pyridin-3-yl)-7-(methoxymethyl)pyrrolo[2,1-f][1,2,4]triazin-4-yl)amino)tricyclo[3.2.2.0^{2,4}]nonane-6-carboxylate (20d)

Under nitrogen protection, compound **20c** (125 mg, 0.31 mmol) was dissolved in a mixed solvent of 1,4-dioxane and water (10 ml/0.5 ml, v/v). Then potassium carbonate (128 mg, 0.92 mmol), Pd(dppf)Cl₂ (36 mg, 0.05 mmol) and intermediate 1 (119.0 mg, 0.47 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **20d** (120 mg, 59%).

### Step 5:

### (1R,5S,6S,7S)-7-((2-(5-fluoro-1H-pyrrolo[2,3-b]pyridin-3-yl)-7-(methoxymethyl)pyrrolo[2,1-f][1,2,4]triazin-4-yl)amino)tricyclo[3.2.2.0^{2,4}]nonane-6-carboxylic acid (compound 20)

Compound **20d** (120 mg, 0.18 mmol) was dissolved in methanol (5 ml). Lithium hydroxide (75.0 mg, 1.8 mmol, dissolved in 2 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound 20 (50.0 mg, 58%).

LC-MS (ESI): m/z =477.2[M+H]⁺.

¹H NMR (400 MHz, DMSO-d6) δ 12.17 (d, 2H), 8.51-8.48 (m, 1H), 8.31-8.30(m,1H), 8.23 (d, 1H), 7.70(d, 1H), 7.01(d, 1H), 6.64(d, 1H), 4.79 (s, 2H), 4.44(s,1H), 3.35(s, 3H), 2.65-2.64(m, 2H), 2.38(s, 1H), 1.72-1.56(m,4H), 1.07-1.03(m,2H), 0.87-0.84(m,1H), 0.40-0.34(m,1H).

### Example 21:

### (1R,2S,3 S,4R)-3-((2-(5-fluoro-1H-pyrazolo[3,4-b]pyridin-3-yl)-7-(methoxymethyl)pyrrolo[2,1-f][1,2,4]triazin-4-yl)amino)bicyclo[2.2.2]octane-2-carboxylic acid (compound 21)

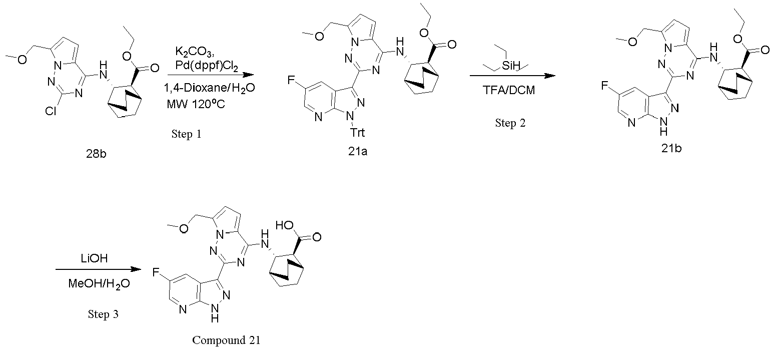

### Step 1:

### ethyl (1R,2S,3S,4R)-3-((2-(5-fluoro-1-trityl-1H-pyrazolo[3,4-b]pyridin-3-yl)-7-(methoxymethyl)pyrrolo[2,1-f][1,2,4]triazin-4-yl)amino)bicyclo[2.2.2]octane-2-carboxylate (21a)

Under nitrogen protection, compound **28b** (200 mg, 0.51 mmol) was dissolved in a mixed solvent of 1,4-dioxane and water (5 ml/0.2 ml, v/v). Then potassium carbonate (207 mg, 1.5 mmol), Pd(dppf)Cl₂ (36 mg, 0.05 mmol) and int-2 (505.0 mg, 1.0 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **21a** (120 mg, 31%).

### Step 2:

### ethyl (1R,2S,3S,4R)-3-((2-(5-fluoro-1H-pyrazolo[3,4-b]pyridin-3-yl)-7-(methoxymethyl)pyrrolo[2,1-f][1,2,4]triazin-4-yl)amino)bicyclo[2.2.2]octane-2-carboxylate (21b)

Compound **21a** (120 mg, 0.16 mmol) was dissolved in dichloromethane (5 ml). Trifluoroacetic acid (1 ml) and triethylsilane (370.0 mg, 3.2 mmol) were added and after the addition, the mixture was reacted at room temperature for 5 hours. After completion of the reaction, the reaction solution was added to a saturated sodium bicarbonate solution (10 ml), and the mixture was extracted twice with dichloromethane (50 ml*2). The organic layers were combined, dried over anhydrous sodium sulphate, filtered and concentrated. The residue was separated and purified by column chromatography (petroleum ether: ethyl acetate (v/v) = 10 : 1-1 : 2) to obtain compound **21b** (27 mg, 34%).

LC-MS (ESI): m/z =494.2[M+H]⁺.

### Step 3:

### (1R,2S,3 S,4R)-3-((2-(5-fluoro-1H-pyrazolo[3,4-b]pyridin-3-yl)-7-(methoxymethyl)pyrrolo[2,1-f][1,2,4]triazin-4-yl)amino)bicyclo[2.2.2]octane-2-carboxylic acid (compound 21)

Compound **21b** (27 mg, 0.054 mmol) was dissolved in methanol (2 ml). Lithium hydroxide (23.0 mg, 0.54 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 5) to obtain compound **21** (12.0 mg, 48%).

LC-MS (ESI): m/z =466.2[M+H]⁺.

¹H NMR (400 MHz, DMSO-d6) δ 14.09 (s, 1H), 12.38 (s, 1H), 8.64 (s, 1H), 8.56(d,1H), 8.09(d, 1H), 7.04(d, 1H), 6.71(d, 1H), 4.92(s, 1H), 4.78 (s, 2H), 3.33(s,3H), 2.81(d, 1H), 2.05(d, 2H), 1.78-1.42(m,8H).

### Example 22:

### (1S,2S,3R,4S)-5,5-difluoro-3-((2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 22)

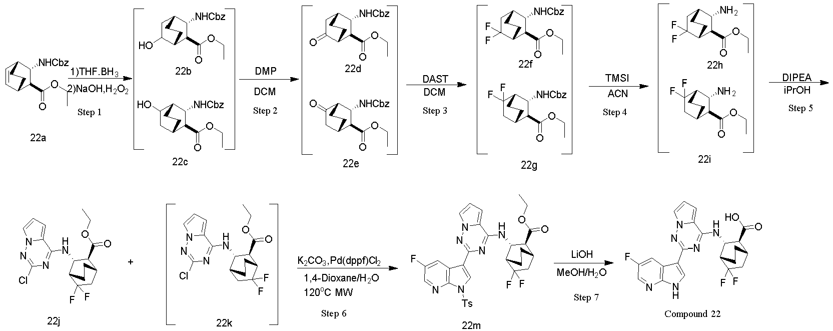

### Step 1:

### ethyl (1R,2S,3 S,4R)-3-(((benzyloxy)carbonyl)amino)-6-hydroxybicyclo[2.2.2]octane-2-carboxylate (22b)

### ethyl (1S,2S,3S,4S)-3-(((benzyloxy)carbonyl)amino)-5-hydroxybicyclo[2.2.2]octane-2-carboxylate (22c)

Under nitrogen protection, compound **22a** (2.0 g, 6.1 mmol) was dissolved in tetrahydrofuran (20 ml), and the mixture was cooled to 0°C. A boranetetrahydrofuran solution (7.3 ml, 7.3 mmol, 1.0 mol/L solution) was slowly added dropwise and after the addition, the mixture was naturally warmed to room temperature and reacted for 2 hours. After completion of the reaction, the mixture was cooled to 0°C. A sodium hydroxide solution (306 mg, 7.3 mmol, 5 ml of water) was added dropwise to a reaction flask, and then hydrogen peroxide (1.38 g, 12.2 mmol, 30% aqueous solution) was added dropwise. After the addition, the mixture was naturally warmed to room temperature and reacted for 5 hours. After completion of the reaction, an aqueous sodium thiosulphate solution (10 ml) was added to quench the reaction, and then the mixture was extracted twice with ethyl acetate (100 ml*2). The organic phases were combined, washed with saturated brine, dried over anhydrous sodium sulphate and filtered. The filtrate was concentrated to obtain a mixture of compound **22b** and compound **22c** (2.0 g, 94%), which was directly used in the next reaction.

LC-MS (ESI): m/z =348.2[M+H]⁺.

### Step 2:

### ethyl (1R,2S,3S,4R)-3-(((benzyloxy)carbonyl)amino)-6-oxobicyclo[2.2.2]octane-2-carboxylate (22d)

### ethyl (1S,2S,3S,4S)-3-(((benzyloxy)carbonyl)amino)-5-oxobicyclo[2.2.2]octane-2-carboxylate (22e)

Under nitrogen protection, a mixture of compound 22b and compound 22c (2.0 g, 5.7 mmol) was dissolved in dichloromethane (20 ml). Dess-Martin reagent (3.6 g, 8.5 mmol) was added in portions while the temperature was controlled at 0°C and after the addition, the mixture was naturally warmed to room temperature and reacted for 5 hours. After completion of the reaction, an aqueous sodium thiosulphate solution (10 ml) was added to quench the reaction, and then the mixture was extracted twice with ethyl acetate (100 ml*2). The organic phases were combined, washed with saturated brine, dried over anhydrous sodium sulphate and filtered. The filtrate was concentrated under reduced pressure, and the residue was separated and purified by column chromatography (petroleum ether: ethyl acetate (v/v) = 10 : 1-1 : 1) to obtain a mixture of compound 22d and compound 22e (1.60 g, 80%), which was directly used in the next reaction.

LC-MS (ESI): m/z =346.2[M+H]⁺.

### Step 3:

### ethyl (1R,2S,3S,4R)-3-(((benzyloxy)carbonyl)amino)-6,6-difluorobicyclo[2.2.2]octane-2-carboxylate (22f)

### ethyl (1S,2S,3R,4S)-3-(((benzyloxy)carbonyl)amino)-5,5-difluorobicyclo[2.2.2]octane-2-carboxylate (22g)

Under nitrogen protection, a mixture of compound **22d** and compound **22e** (1.6 g, 4.65 mmol) was dissolved in dichloromethane (16 ml), and the mixture was cooled to 0-5°C. DAST (3.75 g, 23.23 mmol) was added dropwise and after the addition, the mixture was warmed to 40°C and reacted for 8 hours. After completion of the reaction, the reaction solution was slowly poured into a saturated sodium bicarbonate solution (100 ml) to quench the reaction. Then the obtained reaction solution was extracted with dichloromethane (100 ml*2), washed with saturated brine, dried over anhydrous sodium sulphate and filtered. The filtrate was concentrated under reduced pressure, and the residue was separated and purified by column chromatography (petroleum ether: ethyl acetate (v/v) = 10 : 1-1 : 1) to obtain a mixture of compound **22f** and compound **22g** (1.3 g, 76%), which was directly used in the next reaction.

LC-MS (ESI): m/z =368.2[M+H]⁺.

### Step 4:

### ethyl (1R,2S,3S,4R)-3-amino-6,6-difluorobicyclo[2.2.2]octane-2-carboxylate (22h)

### ethyl (1S,2S,3R,4S)-3-amino-5,5-difluorobicyclo[2.2.2]octane-2-carboxylate (22i)

Under nitrogen protection, a mixture of compound **22f** and compound **22g** (1.3 g, 3.54 mmol) was dissolved in acetonitrile (15 ml), and the mixture was cooled to 0°C. Iodotrimethylsilane (1.06 g, 5.31 mmol) was added dropwise and after the addition, the mixture was naturally warmed to room temperature and reacted. After completion of the reaction (monitored by TLC), triethylamine (1.07 g, 10.62 mmol) was added dropwise and after the addition, the reaction solution was slowly poured into ice water, and the mixture was extracted with dichloromethane (100 ml*2). The organic phases were combined, dried over anhydrous sodium sulphate and filtered. The filtrate was concentrated to obtain a mixture of compound **22h** and compound **22i** (720 mg, 87%), which product was directly used in the next step without purification.

LC-MS (ESI): m/z =234.2[M+H]⁺.

### Step 5:

### ethyl (1S,2S,3R,4S)-3-((2-chloropyrrolo[2,1-f][1,2,4]triazin-4-yl)amino)-5,5-difluorobicyclo[2.2.2]octane-2-carboxylate (22j)

### ethyl (1S,2S,3R,4S)-3-((2-chloropyrrolo[2,1-f][1,2,4]triazin-4-yl)amino)-6,6-difluorobicyclo[2.2.2]octane-2-carboxylate (22k)

A mixture of compound **22h** and compound **22i** (720.0 mg, 3.10 mmol), and compound **18a** (553.0 mg, 2.95 mmol) were dissolved in isopropanol (10 ml). DIPEA (1.20 g, 9.3 mmol) was added and after the addition, the mixture was warmed to 60°C and reacted. After completion of the reaction, the mixture was directly concentrated to remove solvent. The residue was purified by preparative liquid chromatography to obtain compound **22j** and compound **22k.** Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min. Retention time for compound 22j (140 mg, 12%): 11.3 min; Retention time for compound 22k (330 mg, 28%): 13.3 min; the specific configuration was not determined.

Compound 22j: LC-MS (ESI): m/z =385.1[M+H]⁺.

Compound 22j: ¹H NMR (400 MHz, CDCl₃) δ7.52(s, 1H),6.62-6.61(m, 1H), 6.56(s,1H), 4.95-4.90(m, 1H),4.27-4.18(m, 2H), 2.52-1.87(m, 10H), 1.28-1.24(m, 3H).

Compound 22k: LC-MS (ESI): m/z =385.1 [M+H]⁺.

Compound 22k: ¹H NMR (400 MHz, CDCl₃) δ7.53(s, 1H), 6.63(s, 1H), 5.41(s,1H), 4.76 (s, 1H),4.27-4.21(m, 2H), 2.79(d, 1H), 2.54-2.52(m, 1H), 2.40-2.38(m, 1H) , 2.22-1.95(m, 2H), 1.81-1.59(m, 4H) , 1.29-1.24(m, 4H).

### Step 6:

### ethyl (1S,2S,3R,4S)-5,5-difluoro-3-((2-(5-fluoro-1-tosyl-1H-pyrrolo[2,3-b]pyridine-3-yl)pyrrolo[2,1-f][1,2,4]triazin-4-yl)amino)bicyclo[2.2.2]octane-2-carboxylate (22m)

Under nitrogen protection, compound **22j** (140 mg, 0.36 mmol) was dissolved in a mixed solvent of 1,4-dioxane and water (6 ml/0.2 ml, v/v). Then potassium carbonate (150 mg, 1.08 mmol), Pd(dppf)Cl₂ (37 mg, 0.05 mmol) and **intermediate 1** (225.0 mg, 0.54 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **22m** (120 mg, 53%).

### Step 7:

### (1S,2S,3R,4S)-5,5-difluoro-3-((2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 22)

Compound 22m (120 mg, 0.19 mmol) was dissolved in methanol (3 ml). Lithium hydroxide (80.0 mg, 1.9 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 2) to obtain compound **22** (65.0 mg, 74%).

LC-MS (ESI): m/z =457.2[M+H]⁺.

¹H NMR (400 MHz, DMSO-d6) δ 12.21 (d, 2H), 8.50 (dd, 1H), 8.30-8.29 (m, 1H), 8.20(d,1H),7.82(d,1H), 7.73-7.72(m,1H), 6.95(dd, 1H), 6.61-6.60(m, 1H), 4.92-4.86(m, 1H), 2.98(d, 1H), 2.63 (s, 1H), 2.42(s,1H), 2.30-2.07(m, 2H), 1.91-1.76(m, 2H), 1.57-1.53(m, 2H).

### Example 23:

### (1R,2S,3S,4R)-6,6-difluoro-3-((2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 23)

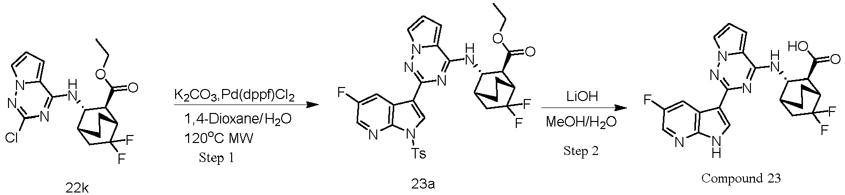

### Step 1:

### ethyl (1R,2S,3S,4R)-6,6-difluoro-3-((2-(5-fluoro-1-tosyl-1H-pyrrolo[2,3-b]pyridine-3-yl)pyrrolo[2,1-f][1,2,4]triazin-4-yl)amino)bicyclo[2.2.2]octane-2-carboxylate (23a)

Under nitrogen protection, compound **22k** (330 mg, 0.86 mmol) was dissolved in a mixed solvent of 1,4-dioxane and water (10 ml/0.5 ml, v/v). Then potassium carbonate (356 mg, 2.58 mmol), Pd(dppf)Cl₂ (73 mg, 0.1 mmol) and intermediate 1 (538.0 mg, 1.29 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **23a** (380 mg, 69%).

### Step 2:

### (1R,2S,3 S,4R)-6,6-difluoro-3-((2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 23)

Compound **23a** (380 mg, 0.69 mmol) was dissolved in methanol (5 ml). Lithium hydroxide (290.0 mg, 6.9 mmol, dissolved in 1 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 2) to obtain compound **23** (150.0 mg, 47%).

LC-MS (ESI): m/z =457.2[M+H]⁺.

¹H NMR (400 MHz, DMSO-d6) δ 12.80 (s, 1H), 12.20(d,1H), 8.50 (dd, 1H), 8.30-8.29 (m, 1H), 8.21(d,1H),8.01(d,1H), 7.73-7.72(m,1H), 6.97(dd, 1H), 6.62-6.60(m, 1H), 4.74-4.72(m, 1H), 3.05(d, 1H), 2.46-2.25(m, 3H), 2.07-1.54(m, 5H).

### Example 24: (1R,6S,7S,8S)-8-((2-(5-fluoro-1H-pyrazolo[3,4-b]pyridin-3-yl)pyrrolo[2,1-f][1,2,4]triazin-4-yl)amino)tricyclo[4.2.2.02,5]decane-7-carboxylic acid (compound 24)

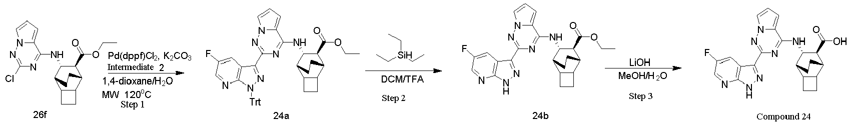

### Step 1: ethyl (1R,6S,7S,8S)-8-((2-(5-fluoro-1-trityl-1H-pyrazolo[3,4-b]pyridin-3-yl)pyrrolo[2,1-f][1,2,4]triazin-4-yl)amino)tricyclo[4.2.2.02,5]decane-7-carboxylate (24a)

Under nitrogen protection, compound **26f (200** mg, 0.53 mmol) was dissolved in a mixed solvent of 1,4-dioxane and water (5 ml/0.2 ml, v/v). Then potassium carbonate (220 mg, 1.6 mmol), Pd(dppf)Cl₂ (36 mg, 0.05 mmol) and int-2 (505.0 mg, 1.0 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **24a** (140 mg, 37%).

### Step 2: ethyl (1R,6S,7S,8S)-8-((2-(5-fluoro-1H-pyrazolo[3,4-b]pyridin-3-yl)pyrrolo[2,1-f][1,2,4]triazin-4-yl)amino)tricyclo[4.2.2.02,5]decane-7-carboxylate (24b)

Compound **24a** (140 mg, 0.19 mmol) was dissolved in dichloromethane (5 ml). Trifluoroacetic acid (1 ml) and triethylsilane (440.0 mg, 3.2 mmol) were added and after the addition, the mixture was reacted at room temperature for 5 hours. After completion of the reaction, the reaction solution was added to a saturated sodium bicarbonate solution (10 ml), and the mixture was extracted twice with dichloromethane (50 ml*2). The organic layers were combined, dried over anhydrous sodium sulphate, filtered and concentrated. The residue was separated and purified by column chromatography (petroleum ether: ethyl acetate (v/v) = 10 : 1-1 : 2) to obtain compound **24b** (24 mg, 26%).

LC-MS (ESI): m/z =476.2[M+H]⁺.

### Step 3: (1R,6S,7S,8S)-8-((2-(5-fluoro-1H-pyrazolo[3,4-b]pyridin-3-yl)pyrrolo[2,1-f][1,2,4]triazin-4-yl)amino)tricyclo[4.2.2.02,5]decane-7-carboxylic acid (compound 24)

Compound **24b** (24 mg, 0.05 mmol) was dissolved in methanol (2 ml). Lithium hydroxide (21.0 mg, 0.5 mmol, dissolved in 0.5 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 5) to obtain compound **24** (12.0 mg, 53%).

LC-MS (ESI): m/z =448.2[M+H]⁺.

¹H NMR (400 MHz, DMSO-d6) δ 14.09 (s, 1H), 12.38 (s, 1H), 8.64 (s, 1H), 8.53(d,1H), 8.14(d, 1H), 7.76(s, 1H), 7.06(d, 1H), 6.69(d, 1H), 4.76(s, 1H), 4.07 (s, 2H), 2.94-2.93(m,1H), 2.72-2.69(m,1H), 2.45-2.43(m,1H), 2.19-1.71(m,8H).

### Example 25 (1R,2S,3S,4R)-3-((6-acetamido-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 25)

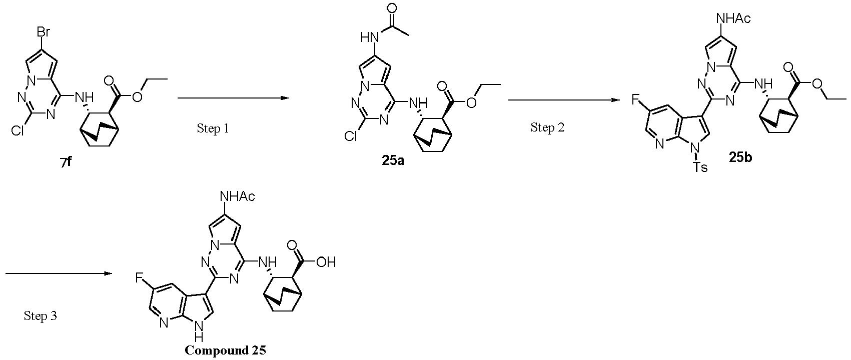

### Step 1:

### Ethyl-(1R,2S,3S,4R)-3-((6-acetamido-2-chloropyrrolo[2,1-f][1,2,4]triazin-4-yl)amino)bicyclo[2.2.2]octane-2-carboxylate (25a)

Compound **7f** (300 mg, 0.7 mmol), acetamide (62 mg, 1.05 mmol), CuI (13 mg, 0.07 mmol), K₃PO₄ (300 mg, 1.4 mmol) and N,N'-dimethyl-1,2-ethylenediamine (6.2 mg ,0.07 mmol) were mixed and dissolved in 5 mL of 1,4-dioxane, and the mixture was heated to 120°C under microwave and reacted for 2 hours.

After LC-MS detection showed that the reaction of the raw materials was completed, the reaction solution was concentrated, and the residue was separated by column chromatography (PE : EA = 2 : 1, v/v) to obtain 160 mg of compound **25a** as a brown solid (yield: 56%).

LC-MS (ESI): m/z =373.1 [M+H] +

### Step 2:

### ethyl (1R,2S,3S,4R)-3-((6-acetamido-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (25b)

**25a** (230 mg, 0.55 mmol), Pd(dppf)Cl₂ (34 mg, 0.046 mmol) and potassium carbonate (127 mg, 0.92 mmol) were mixed and dissolved in 5 mL of 1,4-dioxane and 0.1 mL of water. The mixture was heated to 120°C under microwave and reacted for 1 hour. After LC-MS detection showed that the reaction of the raw materials was completed, the reaction solution was concentrated, and the residue was separated by column chromatography (PE : EA = 2 : 1, v/v) to obtain 220 mg of compound **25b** as a brown solid (yield: 72.5%).

LC-MS (ESI): m/z =660.2 [M+H] +

### Step 3:

### (1R,2S,3 S,4R)-3-((6-acetamido-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 25)

**25b** (220 mg, 0.33 mmol) was dissolved in 10 mL of methanol. An aqueous lithium hydroxide solution (79 mg, 3.3 mmol) was added, and the mixture was heated to 60°C and reacted for 6 hours. After LC-MS detection showed that the reaction was completed, 2 N aqueous hydrochloric acid solution was added to adjust pH to 6. The reaction solution was concentrated, and the residue was separated by pre-HPLC (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain 90 mg of compound 25 as a yellow solid (retention time: 6.5 min, yield: 55%).

LC-MS (ESI): m/z =478.2 [M+H] +

¹H NMR (400 MHz, DMSO-d6) δ 12.15 (s, 1H), 10.15(s, 1H), 8.52-8.49 (m, 1H), 8.28-8.26 (m, 1H), 8.16-8.15(d, 1H), 7.88-7.86 (d, 1H), 7.79 (d, 1H), 7.02 (d, 1H), 4.80-4.77(m, 1H), 2.81-2.79(d, 1H), 2.06 (s, 2H), 2.02(s, 2H), 1.82 - 1.42 (m, 8H).

### Example 26 (1R,6S,7S,8S)-8-((2-(5-fluoro-1H-pyrrolo[2,3-b]pyridin-3-yl)pyrrolo[2,1-f][1,2,4]triazin-4-yl)amino)tricyclo[4.2.2.02,5]decane-7-carboxylic acid (compound 26)

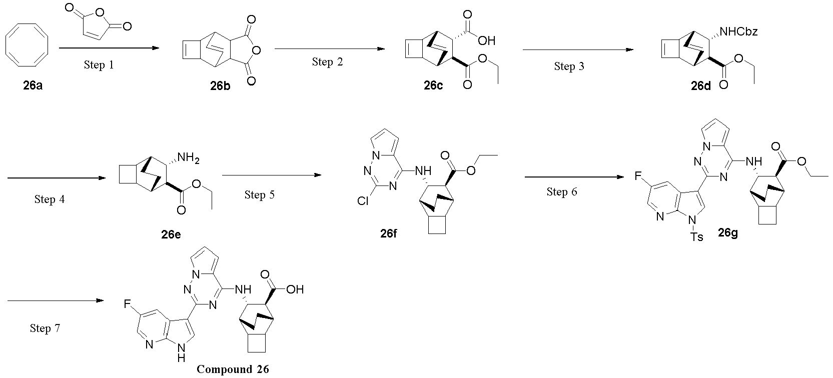

### Step 1:

### 3a,4,4a,6a,7,7a-hexahydro-4,7-ethenocyclobuta[f]isobenzofuran-1,3-dione (26-B)

Maleic anhydride (16.95 g, 172.83 mmol) was dissolved in 250 mL of chlorobenzene. Cycloocta-1,3,5,7-tetraene (18 g, 172.83 mmol) was added under an ice bath, and the mixture was warmed to 120°C and reacted overnight. The reaction solution was concentrated to dryness, and a yellow solid was precipitated out. 50 mL of methanol was added, and the mixture was slurried for 1 hour and filtered by suction. The filter cake was washed with a small amount of methanol and dried to obtain 22 g of compound 26b as a white solid (yield: 63%).

LC-MS (ESI): m/z =203.1 [M+H] +

### Step 2:

### (1S,6R,7S,8S)-8-(ethoxycarbonyl)tricyclo[4.2.2.0^{2.5}]deca-3,9-diene-7-carboxylic acid (26c)

**26b** (10 g, 49.45 mmol) was dissolved in 500 mL of toluene, and quinine (17.65 g, 54.40 mmol) was added to the mixture. Anhydrous ethanol (17 mL) was added at -20°C, and the mixture was reacted overnight at this temperature. After the reaction of the raw materials was completed, 8 mL of 6 N aqueous hydrochloric acid solution was added, and the mixture was stirred for half an hour and subjected to liquid-liquid separation. The organic phase was dried over anhydrous sodium sulphate for half an hour. Potassium tert-amylate (1.7 M, 58 mL) was added dropwise to the dried organic phase at -20°C, and the mixture was stirred for another half an hour at this temperature. 2 mL of acetic acid was added, followed by 80 mL of 2 N aqueous hydrochloric acid solution, and the mixture was stirred for 15 minutes, extracted and subjected to liquid-liquid separation. The organic phase was washed once with saturated brine, dried and concentrated to dryness. The residue was subjected to column chromatography (PE : EA = 5 : 1, v/v) to obtain 6.9 g of compound 26c as a colourless oil (yield: 56%).

LC-MS (ESI): m/z =249.1 [M+H] +

### Step 3:

### Ethyl-(1R,6S,7S,8S)-8-(((benzyloxy)carbonyl)amino)tricyclo[4.2.2.02,5]deca-3,9-diene-7-carboxylate (26d)

**26c** (0.55 g, 2.22 mmol) and triethylamine (0.62 mL, 0.45 mmol) were dissolved in 10 ml of toluene, and DPPA (0.73 g, 2.66 mmol) was added to the mixture while stirring. The mixture was warmed to 96°C and reacted for 1 hour. Benzyl alcohol (0.23 ml, 2.22 mmol) was added, and the mixture was reacted at 96°C for 5 hours. After the reaction of the raw materials was completed, 25 ml of EA and 10 ml of a saturated aqueous sodium bicarbonate solution were added, respectively. The organic phase was extracted, separated, dried, concentrated and passed through a column (PE : EA = 10 : 1, v/v) to obtain 645 mg of compound 26d as a colourless oil (yield: 82%).

LC-MS (ESI): m/z =354.2 [M+H] +

### Step 4:

### ethyl (1R,6S,7S,8S)-8-aminotricyclo[4.2.2.02,5]decane-7-carboxylate (26e)

**26d** (0.644 g, 1.82 mmol) was dissolved in 50 mL of methanol. 120 mg of 10% palladium on carbon was added, and the mixture was subjected to hydrogen replacement twice and reacted at room temperature under hydrogen condition for 2 hours. After the reaction of the raw materials was completed, the mixture was filtered by suction to remove palladium on carbon. The filter cake was washed once with methanol, and the mother liquor was concentrated to dryness to obtain 405 mg of compound **26e** as a colourless oil (yield: 99%).

LC-MS (ESI): m/z =224.2 [M+H] +

### Step 5:

### Ethyl-(1R,6S,7S,8S)-8-((2-chloropyrrolo[2,1-f][1,2,4]triazin-4-yl)amino)tricyclo[4.2.2.02,5]decane-7-carboxylate (26f)

**26e** (0.34 g, 1.79 mmol) and DIEA (0.59 mL, 3.58 mmol) were mixed and dissolved in 20 mL of isopropanol, and the mixture was warmed to 60°C and stirred for 2 hours. The reaction solution was concentrated, and the residue was separated by column chromatography (PE : EA = 4 : 1, v/v) to obtain (26f) as a colourless oil (316 mg, yield: 47%).

LC-MS (ESI): m/z =375.2 [M+H] +

### Step 6:

### Ethyl-(1R,6S,7S,8S)-8-((2-(5-fluoro-1-tosyl-1H-pyrrolo[2,3-b]pyridin-3-yl)pyrrolo[2,1-f][1,2,4]triazin-4-yl)amino)tricyclo[4.2.2.02,5]decane-7-carboxylate (26g)

**26f** (0.1 g, 0.27 mmol), 5-fluoro-1-(4-tosyl)-3-(tetramethyl-1,3,2-dioxaboran-2-yl)-1H-pyrrolo[2,3-b]pyridine (0.13 g, 0.32 mmol), potassium carbonate (75 mg, 0.54 mmol) and Pd(dppf)Cl₂ (20 mg, 0.027 mmol) were mixed and dissolved in 3 mL of 1,4-dioxane and 0.2 mL of water. The mixture was subjected to N₂ replacement, heated to 120°C under microwave and reacted for 1 hour. The reaction solution was concentrated, and the residue was separated by column chromatography (PE : EA = 3 : 1, v/v) to obtain (**26g**) as a white solid (114 mg, yield: 67%).

LC-MS (ESI): m/z =629.2 [M+H] +

### Step 7:

### (1R,6S,7S,8S)-8-((2-(5-fluoro-1H-pyrrolo[2,3-b]pyridin-3-yl)pyrrolo[2,1-f][1,2,4]triazin-4-yl)amino)tricyclo[4.2.2.02,5]decane-7-carboxylic acid (compound 26)

**26g** (114 mg, 0.18 mmol) was dissolved in 10 mL of methanol. An aqueous lithium hydroxide solution (86 mg, 3.6 mmol) was added, and the mixture was heated to 60°C and reacted for 5 hours. After LC-MS detection showed that the reaction was completed, 2 N aqueous hydrochloric acid solution was added to adjust pH to 6. The reaction solution was concentrated, and the residue was separated by pre-HPLC (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain 72 mg of compound **26** as a yellow solid (retention time: 7.5 min, yield: 89%).

LC-MS (ESI): m/z =447.2 [M+H] +

¹H NMR (400 MHz, DMSO-d6) δ 12.16 (s, 1H), 8.50-8.47 (m, 1H), 8.28-8.27 (m, 1H), 8.15-8.14(d, 1H), 7.92-7.90 (d, 1H), 7.67 (d, 1H), 6.96 (d, 1H), 6.60-6.58(m, 1H), 4.67-4.63(m, 1H), 2.90-2.87(d, 1H), 2.73(s, 1H), 2.45-2.40(m, 1H), 2.22-2.08 (m, 4H), 2.03-2.00(m, 3H), 1.89-1.63 (m, 4H).

### Example 27:

### (25,3 S)-3-((2-(5-fluoro-1H-pyrrolo[2,3-b]pyridin-3-yl)imidazo[5,1-f][1,2,4]triazin-4-yl)amino)bicyclo[2.2.2]octane-2-carboxylic acid (compound 27)

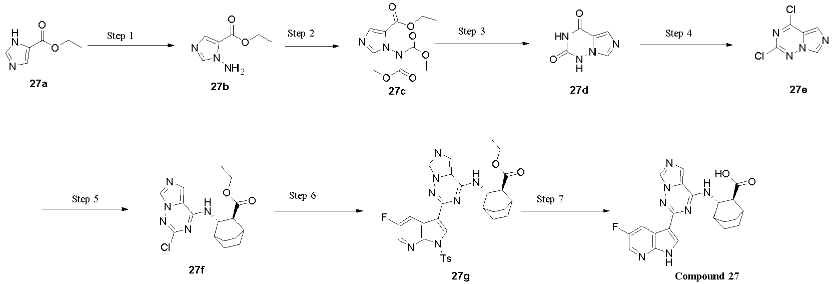

### Step 1:

### ethyl 1-amino-1H-imidazole-5-carboxylate (27b)

**27a** (1.4 g, 10 mmol) was dissolved in N,N-dimethylformamide (50 mL). Under nitrogen protection, lithium bistrimethylsilylamide (2.5 M, 4 mL) was added at -10°C, followed by diphenylphosphinyl hydroxylamine (2.35 g, 10 mmol) after 30 min, and the mixture was naturally warmed to room temperature and reacted for 4 hours. The mixture was concentrated under reduced pressure, and then washed with dichloromethane (100 mL × 3). The washing solution was concentrated, and then the residue was separated and purified by silica gel column chromatography (petroleum ether: ethyl acetate (v/v) = 0.1 : 1-0.3 : 1) to obtain title compound (**27b**) as a yellow solid (600 mg, yield: 38%).

LCMS m/z =156.07 [M+1]

### Step 2:

### ethyl 1-(bis(methoxycarbonyl)amino)-1H-imidazole-5-carboxylate (27c)

**27b** (600 mg, 3.87 mmol) was dissolved in DMF (20 mL). Methyl chloroformate (1.1 g, 11.61 mmol) and pyridine (930 mg, 11.61 mmol) were added, and under nitrogen protection, the mixture was reacted at room temperature for 1 hour. Water (30 mL) was added, and the mixture was extracted with dichloromethane (30 mL × 3), washed with dilute hydrochloric acid (1 N, 30 mL × 1), and dried over anhydrous sodium sulphate. The residue was separated and purified by silica gel column chromatography (petroleum ether: ethyl acetate (v/v) = 0.1 : 1-0.5 : 1) to obtain title compound 27c as a light yellow solid (500 mg, yield: 48%).

LC-MS m/z =272.08 [M+1]

### Step 3:

### imidazo[5,1-f][1,2,4]triazine-2,4(1H,3H)-dione (27d)

**27c** (500 mg, 1.85 mmol) was placed in a sealed tube (100 mL), to which isopropanol (10 mL) was added, followed by ammonia water (10 mL), and the mixture was reacted at 110°C for 12 hours and concentrated to obtain title compound (**27d**) as a yellow solid (300 mg, yield: 100%).

LC-MS m/z =153.03 [M+1]

### Step 4:

### 2,4-dichloroimidazo[5,1-f][1,2,4]triazine (27e)

Imidazo[5,1-f] [1,2,4]triazine-2,4(1H,3H)-dione (**27d**) (300 mg, 1.85 mmol) was placed in a sealed tube (100 mL), to which phosphorus oxychloride (20 mL) and triethylamine hydrochloride (754 mg, 5.55 mmol) were added, and the mixture was reacted at 110°C for 12 hours and concentrated. Then the residue was separated and purified by silica gel column chromatography (petroleum ether: ethyl acetate (v/v) = 0.1 : 1-0.5 : 1) to obtain title compound 2,4-dichloroimidazo[5,1-f] [1,2,4]triazine (**27e**) as a yellow solid (150 mg, yield: 43%).

LCMS m/z =189.01 [M+1]

### Step 5:

### ethyl (2S,3S)-3-((2-chloroimidazo[5,1-f][1,2,4]triazin-4-yl)amino)bicyclo[2.2.2]octane-2-carboxylate (27f)

**27e** (150 mg, 0.79 mmol) was dissolved in isopropanol (10 mL). Ethyl (2S,3S)-3-aminobicyclo[2.2.2]octane-2-carboxylate hydrochloride (233 mg, 1.0 mmol) and N,N-diisopropylethylamine (516 mg, 4.0 mmol) were added, and the mixture was reacted at 60°C for 1 hour and concentrated. Then the residue was separated and purified by silica gel column chromatography (petroleum ether: ethyl acetate (v/v) = 0.1 : 1-0.3 : 1) to obtain title compound **27f** as a yellow solid (100 mg, yield: 36%).

LC-MS m/z =350.13 [M+1]

### Step 6:

### ethyl (25,3 S)-3-((2-(5-fluoro-1-tosyl-1H-pyrrolo[2,3-b]pyridin-3-yl)imidazo[5,1-f][1,2,4]triazin-4-yl)amino)bicyclo[2.2.2]octane-2-carboxylate (27g)

**27f** (80 mg, 0.43 mmol) was dissolved in dioxane (10 mL), and 5-fluoro-3-(4,4,5,5-tetramethyl-1,3,2-dioxaboran-2-yl)-1-tosyl-1H-pyrrolo [2,3-b]pyridine (247 mg, 0.60 mmol), potassium carbonate (166 mg, 1.2 mmol) and [1,1'-bis(diphenylphosphino)ferrocene]palladium dichloride (50 mg, 0.07 mmol) were added to the mixture, followed by water (1 mL). Under nitrogen protection, the mixture was reacted under microwave at 120°C for 1 hour and concentrated. Then the residue was separated and purified by silica gel column chromatography (petroleum ether: ethyl acetate (v/v) = 1 : 5-1 : 1) to obtain title compound 27 g as a yellow solid (80 mg, yield: 46%).

LC-MS m/z =604.21 [M+1]

### Step 7:

### (2S,3S)-3-((2-(5-fluoro-1H-pyrrolo[2,3-b]pyridin-3-yl)imidazo[5,1-f][1,2,4]triazin-4-yl)amino)bicyclo[2.2.2]octane-2-carboxylic acid (compound 27)

**27g** (80 mg, 0.13 mmol) was dissolved in methanol (10 mL). Water (5 mL) and lithium hydroxide (29 mg, 1.2 mmol) were added, and the mixture was reacted at 60°C for 2 hours. 1 N hydrochloric acid was added to adjust pH to 5-6. Water (20 mL) was added, and the mixture was extracted with dichloromethane (50 mL × 3). The organic phases were combined, washed with a saturated aqueous sodium chloride solution (50 mL), dried over anhydrous sodium sulphate and concentrated. The obtained crude compound was separated and purified by a preparative liquid chromatography column (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain title compound (**compound 27**) (19 mg, 32%, retention time: about 6.3 min).

¹H NMR (400 MHz, DMSO-d6) δ 12.35 (d, 2H), 8.54 - 8.44 (m, 3H), 8.27 (d, 2H), 7.83 (s, 1H), 4.94 - 4.66 (m, 1H), 2.72 (d, 1H), 2.05 - 2.02 (m, 2H), 1.80 - 1.46 (m, 8H).

MS M/Z (ESI): m/z =422.17 (M+1)

### Example 28 (1R,2S,3S,4R)-3-((2-(5-fluoro-1H-pyrrolo[2,3-b]pyridin-3-yl)-7-(methoxymethyl)pyrrolo[2,1-f][1,2,4]triazin-4-yl)amino)bicyclo[2.2.2]octane-2-carboxylic acid (compound 28)

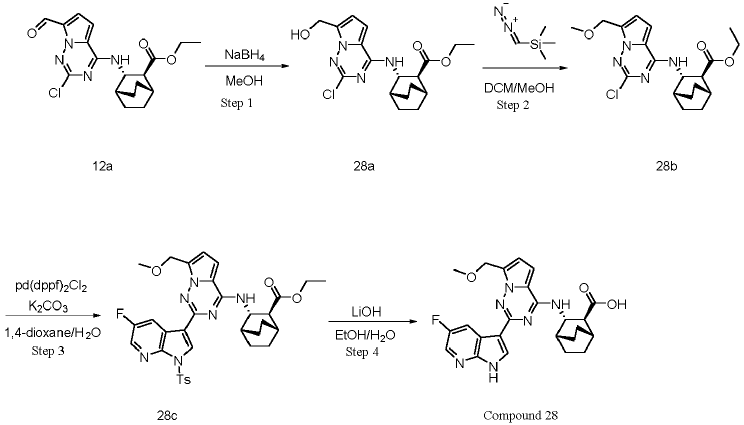

### Step 1:

### ethyl-(1R,2S,3 S,4R)-3-((2-chloro-7-(hydroxymethyl)pyrrolo[2,1-f][1,2,4]triazin-4-yl)amino)bicyclo[2.2.2]octane-2-carboxylate (28a)

Compound **12a** (700 mg, 1.86 mmol) was dissolved in methanol (7 mL). Sodium borohydride (70 mg, 1.86 mmol) was added, and the mixture was reacted at room temperature for 30 minutes. Water was added, and the mixture was extracted with ethyl acetate. The ethyl acetate phase was concentrated under reduced pressure to dryness, and the residue was separated and purified by column chromatography (petroleum ether: ethyl acetate (v/v) = 10 : 1-2 : 1) to obtain compound 28a (400 mg, 56.9%).

LC-MS (ESI): m/z =379.2[M+H]⁺.

¹H NMR (400 MHz, CDCl₃) δ 6.55 (s, 2H), 5.58 (s, 1H), 4.90 (s, 2H), 4.67 (s, 1H), 4.24-4.19 (m, 2H), 2.83 (s, 1H), 2.45-2.43 (m, 1H), 2.04-2.03 (m, 1H), 1.96-1.95 (m, 1H), 1.84-1.80 (m, 1H), 1.73 - 1.58 (m, 6H), 1.48-1.42 (m, 1H), 1.28-1.25 (t, 3H).

### Step 2:

### ethyl-(1R,2S,3 S,4R)-3-((2-chloro-7-(methoxymethyl)pyrrolo[2,1-f][1,2,4]triazin-4-yl)amino)bicyclo[2.2.2]octane-2-carboxylate (28b)

Compound **28a** (120 mg, 0.32 mmol) was dissolved in a mixed solvent of dichloromethane (5 mL) and methanol (5 mL). A 2 mol/L solution of trimethylsilyldiazomethane in n-hexane (1.3 mL, 3.2 mmol) was added, and the mixture was reacted at room temperature for 4 hours. The 2 mol/L solution of trimethylsilyldiazomethane in n-hexane (1.3 mL*3, 3.2 mmol) was repeatedly added at intervals of 2 hours, and the mixture was stirred at room temperature overnight. The mixture was extracted with dichloromethane and water. The dichloromethane phase was concentrated under reduced pressure, and the residue was separated and purified by column chromatography (petroleum ether: ethyl acetate (v/v) = 10 : 1-3 : 1) to obtain compound **28b** (48 mg, 38.7%).

LC-MS (ESI): m/z =393.2[M+H]⁺.

¹H NMR (400 MHz, CDCl₃) δ 6.62 (m, 2H), 5.51 (s, 1H), 4.74 (s, 2H), 4.67 (s, 1H), 4.24-4.19 (m, 2H), 3.41 (s, 3H), 2.43-2.42 (m, 1H), 2.03-2.02 (m, 1H), 1.95-1.94 (m, 1H), 1.84-1.81 (m, 1H), 1.75 - 1.56 (m, 6H), 1.47-1.42 (m, 1H), 1.28-1.24 (t, 3H).

### Step 3:

### ethyl-(1R,2S,3S,4R)-3-((2-(5-fluoro-1-tosyl-1H-pyrrolo[2,3-b]pyridin-3-yl)-7-(methoxymethyl)pyrrolo[2,1-f][1,2,4]triazin-4-yl)amino)bicyclo[2.2.2]octane-2-carboxylate (28c)

Compound **28b** (48 mg, 0.122 mmol) was dissolved in a mixed solvent of 1,4-dioxane (2 mL) and water (0.2 mL). Intermediate **2** (77 mg, 0.183 mmol), potassium carbonate (36 mg, 0.244 mmol) and Pd(dppf)Cl₂ (22 mg, 0.025 mmol) were added, and under nitrogen protection, the mixture was reacted in a microwave reactor at 120°C for 1 hour and extracted with ethyl acetate and water. The ethyl acetate phase was concentrated under reduced pressure, and the residue was separated and purified by column chromatography (petroleum ether: ethyl acetate (v/v) = 10 : 1-2 : 1) to obtain compound **28c** (70 mg, 88.6%).

LC-MS (ESI): m/z =647.2[M-H]+.

### Step 4:

### (1R,2S,3S,4R)-3-((7-ethynyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 28)

Compound **28c** (70 mg, 0.108 mmol) was dissolved in methanol (5 mL) and water (1 mL). Lithium hydroxide (91 mg, 2.16 mmol) was added and after the addition, the mixture was warmed to 60°C and reacted for 2 hours. After completion of the reaction, 1 N hydrochloric acid was added to adjust pH to 5-6. Then the mixture was extracted twice with ethyl acetate (50 mL*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound **28** (25 mg, 50%).

LC-MS (ESI): m/z =465.2[M+H]⁺.

¹H NMR (400 MHz, CDCl₃,small MeOD) δ 8.68-8.66 (d, 1H), 8.36 (s, 1H), 8.13 (s, 1H), 6.66-6.63 (d, 2H), 4.90 (s, 2H), 4.76 (s, 1H), 3.52 (s, 3H), 2.59 (s, 1H), 2.13 (s, 1H), 1.99 (s, 1H), 1.84-1.73 (m, 7H), 1.53-1.48 (m, 1H).

### Example 29:

### (1R,2S,3S,4R)-3-((7-ethynyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 29)

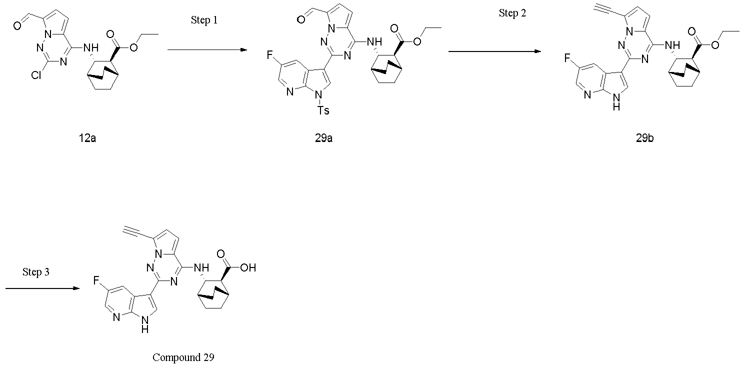

### Step 1:

### ethyl-(1R,2S,3 S,4R)-3-((2-(5-fluoro-1-tosyl-1H-pyrrolo[2,3-b]pyridin-3-yl)-7-formylpyrrolo[2,1-f][1,2,4]triazin-4-yl)amino)bicyclo[2.2.2]octane-2-carboxylate (29a)

Compound **12a** (500 mg, 1.33 mmol) was dissolved in a mixed solvent of 1,4-dioxane (5 mL) and water (0.5 mL). Intermediate 2 (830 mg, 1.99 mmol), potassium carbonate (367 mg, 2.66 mmol) and Pd(dppf)Cl₂ (217 mg, 0.26 mmol) were added, and under nitrogen protection, the mixture was reacted in a microwave reactor at 120°C for 1 hour and extracted with ethyl acetate and water. The ethyl acetate phase was concentrated under reduced pressure, and the residue was separated and purified by column chromatography (petroleum ether: ethyl acetate (v/v) = 10 : 1-3 : 1) to obtain compound **29a** (600 mg, 71.7%).

LC-MS (ESI): m/z =631.2[M+H]⁺.

### Step 3:

### ethyl-(1R,2S,3S,4R)-3-((7-ethynyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylate (29b)

Compound **29a** (300 mg, 0.476 mmol) was added to methanol (3 mL) and tetrahydrofuran (3 mL), followed by potassium carbonate (132 mg, 0.952 mmol). The mixture was cooled to 0°C, and dimethyl (1-diazo-2-oxopropyl)phosphonate (138 mg, 0.714 mmol) was added dropwise. After completion of the dropwise addition, the mixture was transferred to room temperature, stirred overnight and extracted with ethyl acetate and water. The ethyl acetate phase was concentrated under reduced pressure, and the residue was separated and purified by column chromatography (petroleum ether: ethyl acetate (v/v) = 10 : 1-2 : 1) to obtain compound **29b** (60 mg, 26.7%).

LC-MS (ESI): m/z =473.2[M-H]⁺.

### Step 4:

### (1R,2S,3S,4R)-3-((7-ethynyl-2-(5-fluoro-1H-pyrrolo[2,3-b]pyridin-3-yl)pyrrolo[2,1-f][1,2,4]triazin-4-yl)amino)bicyclo[2.2.2]octane-2-carboxylic acid (compound 29)

Compound **29b** (60 mg, 0.127 mmol) was dissolved in methanol (5 mL) and water (1 mL). Lithium hydroxide (106 mg, 2.54 mmol) was added and after the addition, the mixture was warmed to 60°C and reacted for 2 hours. After completion of the reaction, 1 N hydrochloric acid was added to adjust pH to 5-6. Then the mixture was extracted twice with ethyl acetate (50 mL*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 1) to obtain compound **29** (20 mg, 35.7%).

LC-MS (ESI): m/z =445.2[M+H]⁺.

¹H NMR (400 MHz, CDCl₃,small MeOD) δ 8.73 (d, 1H), 8.32 (s, 1H), 8.14 (s, 1H), 6.86-6.84 (d, 1H), 6.60-6.58 (d, 1H), 4.73-4.71 (m, 1H), 3.75 (s, 1H), 2.59-2.57 (m, 1H), 2.12-2.11 (m, 1H), 1.90-1.82 (m, 4H), 1.76-1.70 (m, 4H), 1.52-1.51 (m, 1H).

### Example 30:

### (1R,5S,6S,7S)-7-((2-(5-fluoro-1H-pyrrolo[2,3-b]pyridin-3-yl)-7-(trifluoromethyl)pyrrolo[2,1-f][1,2,4]triazin-4-yl)amino)tricyclo[3.2.2.0^{2,4}]nonane-6-carboxylic acid (compound 30)

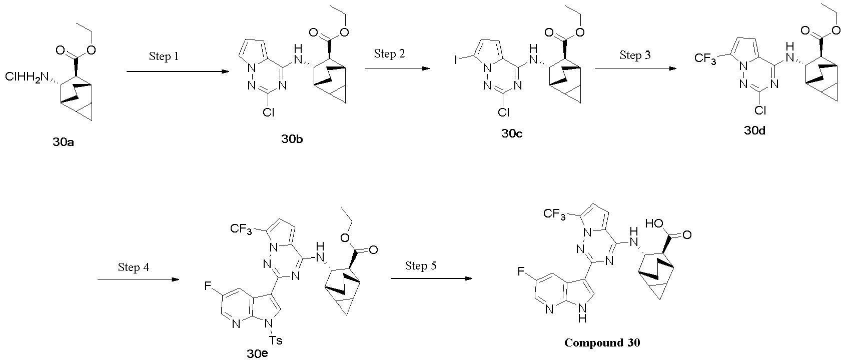

### Step 1:

### ethyl (1R,5S,6S,7S)-7-((2-chloropyrrolo[2,1-f][1,2,4]triazin-4-yl)amino)tricyclo[3.2.2.0^{2,4}]nonane-6-carboxylate (30b)

(**30a**) (800 mg, 3.26 mmol) was dissolved in isopropanol (30 mL). 2,4-dichloropyrrolo[2,1-f] [1,2,4]triazine (610 mg, 3.26 mmol) and N,N-diisopropylethylamine (1.2 g, 9.78 mmol) were added, and the mixture was reacted at 60°C for 2 hours and concentrated under reduced pressure. Then the residue was separated and purified by silica gel column chromatography (petroleum ether: ethyl acetate (v/v) = 0.1 : 1-0.4 : 1) to obtain title compound (30b) as a yellow solid (1.0 g, yield: 85%).

LCMS m/z =361.14 [M+1]

### Step 2:

### ethyl (1R,5S,6S,7S)-7-((2-chloro-7-iodopyrrolo[2,1-f][1,2,4]triazin-4-yl)amino)tricyclo[3.2.2.0^{2,4}]nonane-6-carboxylate (30c)

**30b** (1.0 g, 2.7 mmol) was dissolved in DCE (50 mL). N-iodosuccinimide (608 mg, 2.7 mmol) was added under an ice bath, and the mixture was naturally warmed to room temperature and reacted for 2 hours. The residue was separated and purified by silica gel column chromatography (petroleum ether: ethyl acetate (v/v) = 0.1 : 1-0.4 : 1) to obtain title compound (30c) as a light yellow solid (500 mg, yield: 38%).

LCMS m/z =487.03 [M+1]

### Step 3:

### ethyl (1R,5S,6S,7S)-7-((2-chloro-7-(trifluoromethyl)pyrrolo[2,1-f][1,2,4]triazin-4-yl)amino)tricyclo[3.2.2.0^{2,4}]nonane-6-carboxylate (30d)

**30c** (500 mg, 1.03 mmol) was dissolved in DMF (40 mL), and cuprous iodide (391 mg, 2.06 mmol) and methyl 2,2-difluoro-2-(fluorosulfonyl)acetate (1056 mg, 5.5 mmol) were added to the mixture. Under nitrogen protection, the mixture was reacted at 110°C for 4 hours. Water (30 mL) was added, and the mixture was extracted with ethyl acetate (30 mL × 3), washed with water (30 mL × 3), dried over anhydrous sodium sulphate, and separated and purified by silica gel column chromatography (petroleum ether: ethyl acetate (v/v) = 0.1 : 1-0.4 : 1) to obtain title compound (**30d**) as a light yellow solid (320 mg, yield: 72%).

LCMS m/z =429.12 [M+1]

### Step 4:

### ethyl (1R,SS,6S,7S)-7-((2-(5-fluoro-1-tosyl-1H-pyrrolo[2,3-b]pyridin-3-yl)-7-(trifluoromethyl)pyrrolo[2,1-f][1,2,4]triazin-4-yl)amino)tricyclo[3.2.2.0^{2,4}]nonane-6-carboxylate (30e)

**30d** (120 mg, 0.28 mmol) was dissolved in dioxane (10 mL), and 5-fluoro-3-(4,4,5,5-tetramethyl-1,3,2-dioxaboran-2-yl)-1-tosyl-1H-pyrrolo [2,3-b]pyridine (165 mg, 0.4 mmol), potassium carbonate (116 mg, 0.84 mmol) and [1,1'-bis(diphenylphosphino)ferrocene]palladium dichloride (50 mg, 0.07 mmol) were added to the mixture, followed by water (1 mL). Under nitrogen protection, the mixture was reacted under microwave at 120°C for 1 hour and concentrated. Then the residue was separated and purified by silica gel column chromatography (petroleum ether: ethyl acetate (v/v) = 1:5-1 : 1) to obtain title compound **30e** as a yellow solid (90 mg, yield: 47%).

LCMS m/z =683.20 [M+1]

### Step 7:

### (1R,5S,6S,7S)-7-((2-(5-fluoro-1H-pyrrolo[2,3-b]pyridin-3-yl)-7-(trifluoromethyl)pyrrolo[2,1-f][1,2,4]triazin-4-yl)amino)tricyclo[3.2.2.0^{2,4}]nonane-6-carboxylic acid (compound 30)

(**30e**) (90 mg, 0.13 mmol) was dissolved in methanol (10 mL). Water (5 mL) and lithium hydroxide (29 mg, 1.2 mmol) were added, and the mixture was reacted at 60°C for 2 hours. 1 N hydrochloric acid was added to adjust pH to 5-6. Water (20 mL) was added, and the mixture was extracted with dichloromethane (50 mL × 3). The organic phases were combined, washed with a saturated aqueous sodium chloride solution (50 mL), dried over anhydrous sodium sulphate and concentrated. The obtained crude compound was separated and purified by a preparative liquid chromatography column (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain title compound (**compound 30**) (10 mg, 15%, retention time: about 6.5 min).

¹H NMR (400 MHz, DMSO-d6) δ 12.28 (d, 2H), 8.38 (d, 2.9 Hz, 1H), 8.31 (d, 1H), 8.27 (d, 1H), 8.15 (d, 1H), 7.16 (d, 1H), 7.09 (d, 1H), 4.45 - 4.39 (m, 1H), 2.64 - 2.59 (m, 2H), 2.39 - 2.31 (m, 1H), 1.76 - 1.65 (m, 2H), 1.62 - 1.48 (m, 2H), 1.13-1.02 (m, 1H), 0.97 - 0.84 (m, 2H), 0.44 - 0.33 (m, 1H).

MS M/Z (ESI): m/z =501.16 (M+1)

### Example 31 (1R,SS,6S,7S)-7-((2-(5-fluoro-1H-pyrazolo[3,4-b]pyridin-3-yl)-7-(trifluoromethyl)pyrrolo[2,1-f][1,2,4]triazin-4-yl)amino)tricyclo[3.2.2.0^{2,4}]nonane-6-carboxylic acid (compound 31)

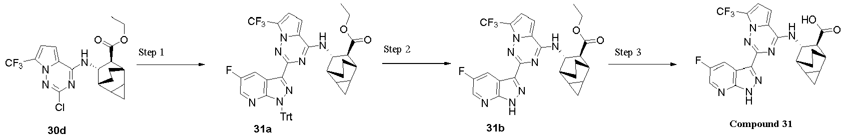

### Step 1:

### ethyl (1R,SS,6S,7S)-7-((2-(5-fluoro-1-trityl-1H-pyrazolo[3,4-b]pyridin-3-yl)-7-(trifluoromethyl)pyrrolo[2,1-f][1,2,4]triazin-4-yl)amino)tricyclo[3.2.2.0^{2,4}]nonane-6-carboxylate (31a)

**30d** (200 mg, 0.47 mmol) was dissolved in dioxane (10 mL), and 5-fluoro-3-(4,4, 5, 5-tetramethyl-1,3,2-dioxaboran-2-yl)-1-trityl-1H-pyrazolo[3,4-b]pyridine (intermediate int-2) (712 mg, 1.4 mmol), potassium carbonate (194 mg, 1.41 mmol) and [1,1'-bis(diphenylphosphino)ferrocene]palladium dichloride (50 mg, 0.07 mmol) were added to the mixture, followed by water (1 mL). Under nitrogen protection, the mixture was reacted under microwave at 120°C for 1 hour and concentrated. Then the residue was separated and purified by silica gel column chromatography (petroleum ether: ethyl acetate (v/v) = 1 : 5-1 : 1) to obtain title compound (**31a**) as a yellow solid (110 mg, yield: 30%).

LCMS m/z =772.29 [M+1]

### Step 2:

### ethyl (1R,5S,6S,7S)-7-((2-(5-fluoro-1H-pyrazolo[3,4-b]pyridin-3-yl)-7-(trifluoromethyl)pyrrolo[2,1-f][1,2,4]triazin-4-yl)amino)tricyclo[3.2.2.0^{2,4}]nonane-6-carboxylate (31b)

**31a** (110 mg, 0.14 mmol) was dissolved in dichloromethane (20 mL). Trifluoroacetic acid (1 mL) and triethylsilane (0.5 mL) were added, and the mixture was reacted at room temperature for 1 hour. Water (30 mL) was added, and the mixture was extracted with dichloromethane (30 mL × 3) and dried over anhydrous sodium sulphate. The residue was separated and purified by silica gel column chromatography (petroleum ether: ethyl acetate (v/v) = 0.1 : 1-1 : 1) to obtain title compound (31b) as a light yellow solid (50 mg, yield: 67%).

LCMS m/z =530.18 [M+1]

### Step 3:

### (1R,5S,6S,7S)-7-((2-(5-fluoro-1H-pyrazolo[3,4-b]pyridin-3-yl)-7-(trifluoromethyl)pyrrolo[2,1-f][1,2,4]triazin-4-yl)amino)tricyclo[3.2.2.0^{2,4}]nonane-6-carboxylic acid (compound 31)

**31b** (50 mg, 0.10 mmol) was dissolved in methanol (10 mL). Water (5 mL) and lithium hydroxide (29 mg, 1.2 mmol) were added, and the mixture was reacted at 60°C for 2 hours. 1 N hydrochloric acid was added to adjust pH to 5-6. Water (20 mL) was added, and the mixture was extracted with dichloromethane (50 mL × 3). The organic phases were combined, washed with a saturated aqueous sodium chloride solution (50 mL), dried over anhydrous sodium sulphate and concentrated. The obtained crude compound was separated and purified by a preparative liquid chromatography column (conditions for preparative liquid chromatography: C18 reverse-phase preparative column, mobile phase: deionized water containing 0.1% trifluoroacetic acid (A) and acetonitrile (B), gradient elution, B content = 5%-50%, elution time: 15 min, flow rate: 12 mL/min, and column temperature: 30°C) to obtain title compound (**compound 31**) (20 mg, 39%, retention time: about 6.0 min).

¹H NMR (400 MHz, DMSO-d6) δ 13.92 (d, 2H), 8.65 (s, 1H), 8.43 (d, 1H), 8.20 (s, 1H), 7.33 - 6.94 (m, 2H), 4.89 - 4.51 (m, 1H), 2.80 - 2.58 (m, 2H), 2.48 - 2.43 (m, 1H), 1.71 - 1.62 (m, 2H), 1.54 - 1.41 (m, 1H), 1.27 - 1.22 (m, 1H), 1.11 - 0.95 (m, 2H), 0.86 - 0.70 (m, 1H), 0.39 - 0.19 (m, 1H).

MS M/Z (ESI): m/z =502.15 (M+1)

### Example 32: (1R,SS,6S,7S)-7-((2-(5-fluoro-1H-pyrazolo[3,4-b]pyridin-3-yl)-7-(methoxymethyl)pyrrolo[2,1-f][1,2,4]triazin-4-yl)amino)tricyclo[3.2.2.0^{2,4}]nonane-6-carboxylic acid (compound 32)

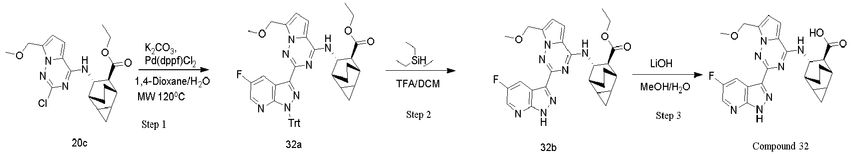

### Step 1:

### Ethyl (1R,SS,6S,7S)-7-((2-(5-fluoro-1-trityl-1H-pyrazolo[3,4-b]pyridin-3-yl)-7-(methoxymethyl)pyrrolo[2,1-f][1,2,4]triazin-4-yl)amino)tricyclo[3.2.2.0^{2,4}]nonane-6-carboxylate(32a)

Under nitrogen protection, compound 20c (1.0 g, 2.47 mmol) was dissolved in a mixed solvent of 1,4-dioxane and water (15 ml/0.3 ml, v/v). Then potassium carbonate (1.0 g, 7.42 mmol), Pd(dppf)Cl₂ (146 mg, 0.2 mmol) and intermediate 2 (2.5 g, 4.94 mmol) were successively added and after the addition, the mixture was reacted under microwave at 120°C for 1 hour. After completion of the reaction, the mixture was directly concentrated, and the concentrated residue was separated and purified by column chromatography (petroleum ether: ethyl acetate (v/v) = 10 : 1-3 : 1) to obtain compound **32a** (1.1 g, 61%).

### Step 2:

### ethyl (1R,5S,6S,7S)-7-((2-(5-fluoro-1H-pyrazolo[3,4-b]pyridin-3-yl)-7-(methoxymethyl)pyrrolo[2,1-f][1,2,4]triazin-4-yl)amino)tricyclo[3.2.2.0^{2,4}]nonane-6-carboxylate (32b)

Compound **32a** (1.1 g, 1.5 mmol) was dissolved in dichloromethane (15 ml). Trifluoroacetic acid (1 ml) and triethylsilane (1.7 g, 15.0 mmol) were added and after the addition, the mixture was reacted at room temperature for 5 hours. After completion of the reaction, the reaction solution was added to a saturated sodium bicarbonate solution (50 ml), and the mixture was extracted twice with dichloromethane (100 ml^{∗}2). The organic layers were combined, dried over anhydrous sodium sulphate, filtered and concentrated. The residue was separated and purified by column chromatography (petroleum ether: ethyl acetate (v/v) = 10 : 1-1 : 2) to obtain compound 32b (310.0 mg, 41%).

LC-MS (ESI): m/z =506.2[M+H]+.

### Step 3:

### (1R,5S,6S,7S)-7-((2-(5-fluoro-1H-pyrazolo[3,4-b]pyridin-3-yl)-7-(methoxymethyl)pyrrolo[2,1-f][1,2,4]triazin-4-yl)amino)tricyclo[3.2.2.0^{2,4}]nonane-6-carboxylic acid (compound 32)

Compound **32b** (310.0 mg, 0.61 mmol) was dissolved in methanol (5 ml). Lithium hydroxide (256.0 mg, 6.1 mmol, dissolved in 1 ml of water) was added and after the addition, the mixture was warmed to 60°C and reacted for 5 hours. After completion of the reaction, 1 N hydrochloric acid was added to adjust pH to 5-6. Then the mixture was extracted twice with dichloromethane (50 ml*2). The organic layers were combined, dried over anhydrous sodium sulphate, filtered and concentrated under reduced pressure. The residue was separated and purified by column chromatography (petroleum ether: ethyl acetate (v/v) = 10 : 1-1 : 5) to obtain compound **32** (220.0 mg, 75%).

LC-MS (ESI): m/z =478.2[M+H]+.

¹H NMR (400 MHz, DMSO-d6) δ 14.12 (s, 1H), 12.52 (s, 1H), 8.65 (s, 1H), 8.53(dd,1H), 7.43(d, 1H), 7.06(d, 1H), 6.73(d, 1H), 4.79 (s, 2H), 4.61(s,1H), 3.34(s,3H), 2.85-2.76(m, 2H), 2.49-2.47(m, 1H), 1.68-1.53(m,4H) , 1.14-1.03(m,2H), 0.82-0.80 (m,1H), 0.35-0.30(m,1H).

### Example 33:

### (1R,2S,3S,4R)-3-((7-(dimethylamino)-2-(5-fluoro-1H-pyrrolo[2,3-b]pyridin-3-yl)pyrolo[2,1-f][1,2,4]triazin-4-yl)amino)bicyclo[2.2.2]octane-2-carboxylic acid (compound 33)

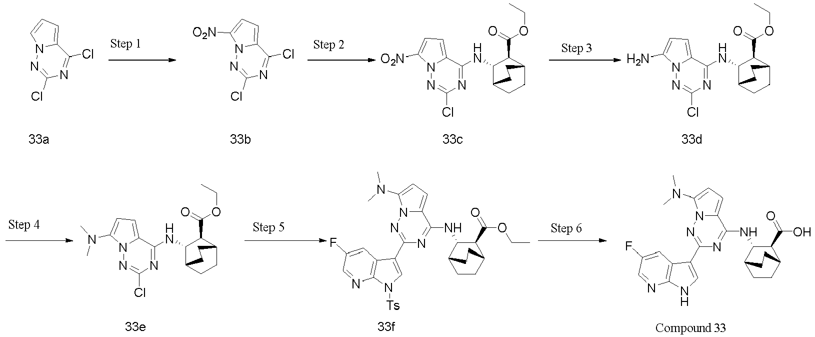

### Step 1:

### 2,4-dichloro-7-nitropyrrolo[2,1-f][1,2,4]triazine (33b)

HNO₃ (3.35 g, 53 mmol) was added dropwise to acetic anhydride (5 ml). The obtained mixed solution was added dropwise to a solution of 2,4-dichloropyrrolo[2,1-f] [1,2,4]triazine (33a) (2 g, 10.64 mmol) in acetic anhydride (30 ml) at 0°C, and the mixture was slowly warmed to room temperature and reacted for 1 h. The obtained reaction solution was poured into water (150 ml), and the mixture was filtered. The solid was washed with water and dried to obtain crude compound 2,4-dichloro-7-nitropyrrolo[2,1-f] [1,2,4]triazine (33b) (2 g).

### Step 2:

### (1R,2S,3S,4R)-ethyl3-((2-chloro-7-nitropyrrolo[2,1-f][1,2,4]triazin-4-yl)amino)bicyclo[2.2.2]octane-2-carboxylate (33c)

33b (2 g, 8.56 mmol) and ethyl (1R,2S,3S,4R)-3-aminobicyclo[2.2.2]octane-2-carboxylate hydrochloride (1.995 g, 8.56 mmol) were added to DCM (20 mL). DIPEA (3.3 g, 25.68 mmol) was added, and the mixture was reacted at room temperature for 1 h. Water (50 mL) was added, and the mixture was extracted with DCM (30 ml*2). The organic phases were combined, washed with a saturated sodium chloride solution, dried over anhydrous sodium sulphate and filtered. The filtrate was concentrated, and then the residue was separated and purified by silica gel column chromatography (petroleum ether: ethyl acetate (v/v) = 10 : 1-1 : 8) to obtain (33c) (1.9 g).

LC-MS (ESI): m/z =394.1 [M+H]+.

### Step 3:

### (1R,2S,3S,4R)-ethyl3-((7-amino-2-chloropyrrolo[2,1-f][1,2,4]triazin-4-yl)amino)bicyclo[2.2.2]octane-2-carboxylate (33d)

**33c** (1.2 g, 3 mmol) and acetic acid (1.8 g, 30 mmol) were added to methanol (15 ml). Then zinc powder (1.95 g, 30 mmol) was added in portions, and the mixture was reacted at room temperature for 1 h and filtered. The filtrate was concentrated to obtain crude compound (1R,2S,3S,4R)-ethyl3-((7-amino-2-chloropyrrolo[2,1-f][1,2,4]triazin-4-yl)amino)bicyclo[2.2.2]octane-2-carboxylate (**33d**) (1.2 g).

LC-MS (ESI): m/z =364.2 [M+H]+.

### Step 4:

### (1R,2S,3 S,4R)-ethyl3-((2-chloro-7-(dimethylamino)pyrrolo[2,1-f][1,2,4]triazin-4-yl)amino)bicyclo[2.2.2]octane-2-carboxylate (33e)

**33d** (500 mg, 1.38 mmol), potassium carbonate (571 mg, 4.14 mmol) and iodomethane (583 mg, 4.14 mmol) were added to DMF (5 ml), and the mixture was reacted at room temperature for 2 h. Water (50 ml) was added, and the mixture was extracted with ethyl acetate, washed with a saturated sodium chloride solution, dried over anhydrous sodium sulphate and filtered. The filtrate was concentrated under reduced pressure, and then the residue was separated and purified by silica gel column chromatography (petroleum ether: ethyl acetate (v/v) = 10 : 1-1 : 4) to obtain compound (**33e**) (360 mg, 67%).

LC-MS (ESI): m/z =392.2 [M+H]+.

### Step 5:

### ethyl (1R,2S,3S,4R)-3-((7-(dimethylamino)-2-(5-fluoro-1-tosyl-1H-pyrrolo[2,3-b]pyridin-3-yl)pyrrolo[2,1-f][1,2,4]triazin-4-yl)amino)bicyclo[2.2.2]octane-2-carboxylate (33f)

**33e** (360 mg, 0.92 mmol), intermediate 1 (500 mg, 1.2 mmol), Pd(dppf)Cl₂ (150 mg, 0.18 mmol) and potassium carbonate (380 mg, 2.76 mmol) were added to dioxane (8 mL). Then water (0.5 ml) was added, and the mixture was subjected to N₂ replacement, then warmed to 110°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**33f**) (400 mg, yield: 60.6%).

LC-MS (ESI): m/z =646.2 [M+H]+.

### Step 6:

### (1R,2S,3 S,4R)-3-((7-(dimethylamino)-2-(5-fluoro-1H-pyrrolo[2,3-b]pyridin-3-yl)pyrolo[2,1-f][1,2,4]triazin-4-yl)amino)bicyclo[2.2.2]octane-2-carboxylic acid (compound 33)

At room temperature, **33f** (330 mg, 0.51 mmol) and LiOH·H2O (210 mg, 5.1 mmol) were added to THF (5 ml). Then ethanol (5 ml) and water (2 ml) were added, and the mixture was warmed to 60°C and reacted for 2 h. The mixture was cooled to room temperature, adjusted to pH = 5-6 with dilute hydrochloric acid and extracted with EA (15 ml*3). The organic phases were combined, washed with a saturated sodium chloride solution, dried over anhydrous sodium sulphate and filtered. The filtrate was concentrated under reduced pressure, and then the obtained crude product was separated and purified by preparative HPLC to obtain compound 33 (200 mg, 75.8%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for compound **33**: 14.1 min;
¹H NMR (400 MHz, DMSO-d6) δ 12.30 (s, 1H), 12.18 (d, 1H), 8.48 (dd, 1H), 8.28 (dd, 1H), 8.19 (d, 1H), 7.58-7.53 (t, 2H), 6.58 (d, 1H), 4.72-4.68 (m, 1H), 2.70 (s, 6H), 2.49 (m, 1H), 1.98 (s, 2H), 1.78 - 1.50 (m, 7H), 1.48-1.45 (m, 1H).

LC-MS (ESI): m/z =464.2[M +H]+.

### Example 34:

### (1R,2S,3 S,4R)-3-((2-(5-fluoro-1H-pyrrolo[2,3-b]pyridin-3-yl)-7-methoxypyrrolo[2,1-f][1,2,4]triazin-4-yl)amino)bicyclo[2.2.2]octane-2-carboxylic acid (compound 34)

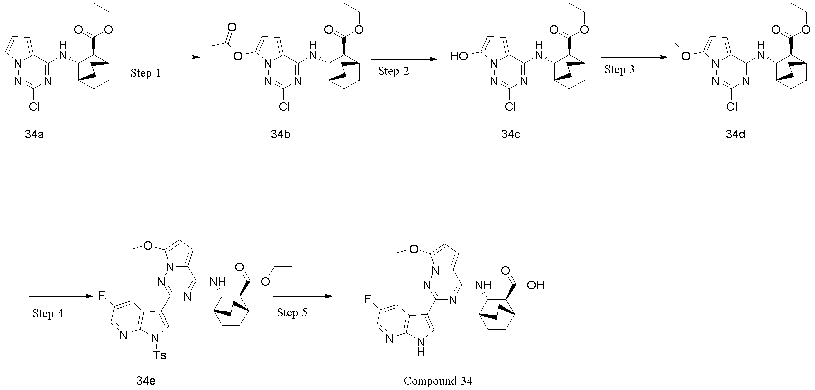

### Step 1: ethyl(1R,2S,3S,4R)-3-((7-acetoxy-2-chloropyrrolo[2,1-f][1,2,4]triazin-4-yl)amino)bicyclo[2.2.2]octane-2-carboxylate (34b)

**34a** (same as compound **18b**) (3.56 g, 10.2 mmol) and iodobenzene diacetate (3.9 g, 12.3 mmol) were added to acetic acid (30 ml), and the mixture was reacted at room temperature for 2 h. Then palladium acetate (456 mg, 2 mmol) was added, and the mixture was subjected to N₂ replacement, and then reacted at room temperature overnight. Water (150 ml) was added to the reaction system, and the mixture was extracted with EA, washed with a saturated sodium chloride solution, dried over anhydrous sodium sulphate and filtered. The filtrate was concentrated under reduced pressure, and then the residue was separated and purified by silica gel column chromatography (petroleum ether: ethyl acetate (v/v) = 10 : 1-1 : 6) to obtain (**34b**) (1.2 g, 29%).

LC-MS (ESI): m/z =407.1 [M+H]+.

### Step 2: ethyl(1R,2S,3S,4R)-3-((2-chloro-7-hydroxypyrrolo[2,1-f][1,2,4]triazin-4-yl)amino)bicyclo[2.2.2]octane-2-carboxylate (34c)

**34b** (1.2 g, 3 mmol), sodium acetate (2.4 g, 30 mmol) and water (10 ml) were added to ethanol (15 ml), and the mixture was reacted at an outside temperature of 85°C for 1 h. The reaction solution was cooled to room temperature and concentrated under reduced pressure to remove part of ethanol. Water (20 ml) was added, and the mixture was extracted with EA, washed with a saturated sodium chloride solution, dried over anhydrous sodium sulphate and filtered. The filtrate was concentrated under reduced pressure to obtain compound (**34c**) (1.05 g, 96%).

LC-MS (ESI): m/z =365.1 [M+H]+.

### Step 3:

### ethyl (1R,2S,3S,4R)-3-((2-chloro-7-methoxypyrrolo[2,1-f][1,2,4]triazin-4-yl)amino)bicyclo[2.2.2]octane-2-carboxylate (34d)

**34c** (500 mg, 1.37 mmol) was added to methanol (10 ml), and then trimethylsilyldiazomethane (7 mmol) was added dropwise to the mixture. The mixture was reacted at room temperature for 2 h. The reaction solution was directly concentrated under reduced pressure, and then the residue was separated and purified by silica gel column chromatography (petroleum ether: ethyl acetate (v/v) = 10 : 1-1 : 6) to obtain (**34d**) (400 mg, 77%).

LC-MS (ESI): m/z =379.2 [M+H]+.

### Step 4:

### ethyl (1R,2S,3S,4R)-3-((2-(5-fluoro-1-tosyl-1H-pyrrolo[2,3-b]pyridin-3-yl)-7-methoxypyrrolo[2,1-f][1,2,4]triazin-4-yl)amino)bicyclo[2.2.2]octane-2-carboxylate (34e)

**34d** (300 mg, 0.79 mmol), intermediate 1 (400 mg, 0.95 mmol), Pd(dppf)Cl₂ (129 mg, 0.16 mmol) and potassium carbonate (327 mg, 2.37 mmol) were added to dioxane (8 mL), followed by water (0.5 ml). The mixture was subjected to N₂ replacement, then warmed to 110°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**34e**) (400 mg, 80%).

LC-MS (ESI): m/z =633.2 [M+H]+.

### Step 5:

### (1R,2S,3S,4R)-3-((2-(5-fluoro-1H-pyrrolo[2,3-b]pyridin-3-yl)-7-methoxypyrrolo[2,1-f][1,2,4]triazin-4-yl)amino)bicyclo[2.2.2]octane-2-carboxylic acid (compound 34)

At room temperature, **34e** (400 mg, 0.63 mmol) and (265 mg, 6.3 mmol) were added to THF (5 ml). Then ethanol (5 ml) and water (2 ml) were added, and the mixture was warmed to 60°C and reacted for 2 h. The mixture was cooled to room temperature, adjusted to pH = 5-6 with dilute hydrochloric acid and extracted with EA (15 ml*3). The organic phases were combined, washed with a saturated sodium chloride solution, dried over anhydrous sodium sulphate and filtered. The filtrate was concentrated under reduced pressure, and then the obtained crude product was separated and purified by preparative HPLC to obtain compound 34 (100 mg, 35%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for **compound 34**: 14.3 min;
¹H NMR (400 MHz, DMSO-d6) δ 12.17 (m, 2H), 8.48 (dd, 1H), 8.27 (dd, 1H), 8.17 (d, 1H), 7.54 (d, 1H), 6.39 (d, 1H), 6.35 (d, 1H), 4.72 (t, 1H), 3.90 (s, 3H), 2.62 (d, 1H), 1.98 (s, 2H), 1.73-1.68(m, 3H), 1.64-1.58 (m, 3H), 1.52 - 1.38 (m, 2H).

LC-MS (ESI): m/z =450.2[M +H]+.

### Example 35:

### (1R,5S,6S,7S)-7-((2-(5-fluoro-1H-pyrrolo[2,3-b]pyridin-3-yl)-7-(methylcarbamoyl)pyrrolo[2,1-f][1,2,4]triazin-4-yl)amino)tricyclo[3.2.2.0^{2,4}]nonane-6-carboxylic acid (compound 35)

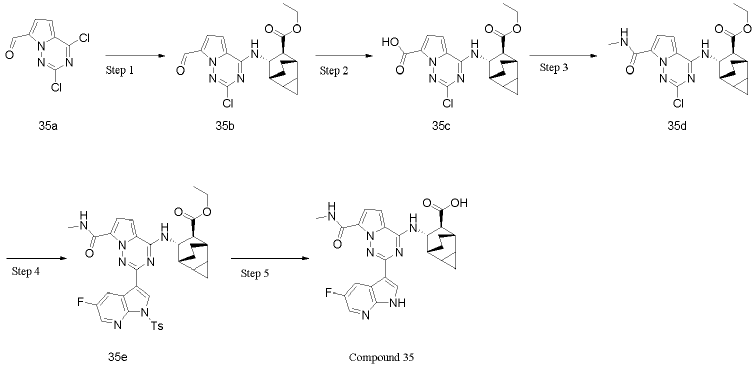

### Step 1:

### ethyl (1R,5S,6S,7S)-7-((2-chloro-7-formylpyrrolo[2,1-f][1,2,4]triazin-4-yl)amino)tricyclo[3.2.2.0^{2,4}]nonane-6-carboxylate (35b)

2,4-dichloropyrrolo[2,1-f] [1,2,4]triazine-7-carbaldehyde (1.06 g, 4.9 mmol) **35a** (same as compound **11b**) and DIPEA (1.6 g, 12.2 mmol) were added to isopropanol, and the mixture was warmed to an outside temperature of 60°C. Then ethyl (1R,5S,6S,7S)-7-aminotricyclo[3.2.2.0^{2,4}]nonane-6-carboxylate hydrochloride (1 g, 4.08 mmol) was added, and the mixture was reacted at 60°C for 1 h. The mixture was cooled to room temperature. The reaction solution was directly concentrated under reduced pressure, and then the residue was separated and purified by silica gel column chromatography (petroleum ether: ethyl acetate (v/v) = 0 : 1-1 : 8) to obtain compound (**35b**) (1.3 g, 82%).

LC-MS (ESI): m/z =389.1 [M+H]+.

### Step 2:

### 2-chloro-4-(((1S,SR,6S,7S)-7-(ethoxycarbonyl)tricyclo[3.2.2.0^{2,4}]nonan-6-yl)amino)pyrrolo[2,1-f][1,2,4]triazine-7-carboxylic acid (35c)

**35b** (1.3 g, 3.35 mmol) and 2-methylbut-2-ene (1.17 g, 16.7 mmol) were dissolved in THF (15 ml). Then sodium chlorite (1.2 g, 13.4 mmol) and sodium dihydrogen phosphate dihydrate (2.09 g, 13.4 mmol) were dissolved in water (4 ml) and added dropwise to the reaction system, and the mixture was reacted at room temperature for 1 h. An aqueous ammonium chloride solution was added, and the mixture was extracted with EA (20 ml*3). The organic phases were combined, washed with a saturated sodium chloride solution, dried over anhydrous sodium sulphate and filtered. The filtrate was concentrated, and then the residue was separated and purified by silica gel column chromatography (petroleum ether: ethyl acetate (v/v) = 10 : 1-1 : 3) to obtain compound (**35c**) (1 g, 74%).

LC-MS (ESI): m/z =405.1 [M+H]+.

### Step 3:

### ethyl (1R,5S,6S,7S)-7-((2-chloro-7-(methylcarbamoyl)pyrrolo[2,1-f][1,2,4]triazin-4-yl)amino)tricyclo[3.2.2.0^{2,4}]nonane-6-carboxylate (35d)

**35c** (350 mg, 0.87 mmol), methylamine hydrochloride (175 mg, 2.6 mmol), HATU (660 mg, 1.73 mmol) and DIPEA (671 mg, 5.2 mmol) were successively added to DCM (10 ml), and the mixture was reacted at room temperature for 2 h. Water (20 ml) was added, and the mixture was extracted with DCM. The organic phase was adjusted to pH = 6 with dilute hydrochloric acid, washed with a saturated sodium chloride solution, dried over anhydrous sodium sulphate and filtered. The filtrate was concentrated under reduced pressure to obtain crude compound ethyl (1R,5S,6S,7S)-7-(((2-chloro-7-(methylcarbamoyl)pyrrolo[2,1-f] [1,2,4]triazin-4-yl)amino)tricyclo[3.2.2.0^{2,4}]nonane-6-carboxylate (**35d**) (400 mg).

LC-MS (ESI): m/z =418.2 [M+H]+.

### Step 4:

### ethyl (1R,SS,6S,7S)-7-((2-(5-fluoro-1-tosyl-1H-pyrrolo[2,3-b]pyridin-3-yl)-7-(methylcarbamoyl)pyrrolo[2,1-f][1,2,4]triazin-4-yl)amino)tricyclo[3.2.2.0^{2,4}]nonane-6-carboxylate (35e)

**35d** (400 mg, 0.96 mmol), intermediate 1 (520 mg, 1.25 mmol), Pd(dppf)Cl₂ (156 mg, 0.19 mmol) and potassium carbonate (397 mg, 2.88 mmol) were added to dioxane (8 mL). Then water (0.5 ml) was added, and the mixture was subjected to N₂ replacement, then warmed to 110°C and reacted under microwave for 1 h. The mixture was cooled to room temperature and filtered. The filtrate was concentrated under reduced pressure, and then the residue was separated and purified by silica gel column chromatography (petroleum ether: ethyl acetate (v/v) = 10 : 1-1 : 4) to obtain compound (**35e**) (500 mg, 77%).

### Step 5:

### (1R,5S,6S,7S)-7-((2-(5-fluoro-1H-pyrrolo[2,3-b]pyridin-3-yl)-7-(methylcarbamoyl)pyrrolo[2,1-f][1,2,4]triazin-4-yl)amino)tricyclo[3.2.2.0^{2,4}]nonane-6-carboxylic acid (compound 35)

At room temperature, **35e** (500 mg, 0.74 mmol) and LiOH·H₂O (310 mg, 7.4 mmol) were added to THF (5 ml). Then ethanol (5 ml) and water (2 ml) were added, and the mixture was warmed to 60°C and reacted for 2 h. The mixture was cooled to room temperature, adjusted to pH = 5-6 with dilute hydrochloric acid and extracted with EA (15 ml^{∗}3). The organic phases were combined, washed with a saturated sodium chloride solution, dried over anhydrous sodium sulphate and filtered. The filtrate was concentrated under reduced pressure, and then the obtained crude product was separated and purified by preparative HPLC to obtain compound 35 (100 mg, 28%). Preparative HPLC separation methods: 1. Instrument: waters 2767 (preparative liquid phase chromatographic instrument); chromatographic column: SunFire@ Prep C18 (19 mm × 250 mm) 2. The sample was dissolved in DMF and filtered with a 0.45 µm filter to prepare a sample solution. 3. Preparative chromatography conditions: a. composition of mobile phases A and B: mobile phase A: acetonitrile; mobile phase B: water (containing 0.1% TFA); b. gradient elution: the content of mobile phase A: 5%-50%; c. flow rate: 12 mL/min; d. elution time: 20 min.

Retention time for compound **35**: 14.2 min;
¹H NMR (400 MHz, DMSO-d6) δ 12.38 (m, 2H), 8.91 (t, 1H), 8.44 (d, 1H), 8.40 (dd, 1H), 8.33 (m, 1H), 7.97 (d, 1H), 7.14 (q, 2H), 4.54 (t, 1H), 3.01 (d, 3H), 2.75 (m, 1H), 2.70 (m, 1H), 2.43 (m, 1H), 1.83 - 1.66 (m, 2H), 1.60-1.54(m, 2H), 1.09-1.01 (m, 2H), 0.90 - 0.81 (m, 1H), 0.37 (q, 1H).

LC-MS (ESI): m/z =490.2[M +H]+.

### Example 36:

### (1R,2S,3S,4R)-3-((2-(5-fluoro-1H-pyrrolo[2,3-b]pyridin-3-yl)-6-(methoxymethyl)pyrrolo[2,1-f][1,2,4]triazin-4-yl)amino)bicyclo[2.2.2]octane-2-carboxylic acid (compound 36)

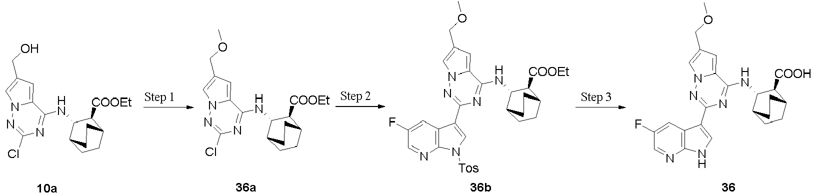

### Step 1:

### (1R,2S,3 S,4R)-ethyl-3-((2-chloro-6-(methoxymethyl)pyrrolo[2,1-f][1,2,4]triazin-4-yl)amino)bicyclo[2.2.2]octane-2-carboxylate (36a)

At room temperature, compound **10a** (0.3 g, 0.79 mmol) was added to methanol (5 mL), followed by trimethylsilyldiazomethane (40 ml, 0.04 mol). Trifluoroacetic acid (11.8 mL, 158 mmol) was added dropwise, and the mixture was reacted for 2 hours. The reaction solution was poured into water (100 mL) and extracted with EA (50 mL × 1). The organic phase was washed once with an aqueous sodium bicarbonate solution and concentrated to dryness. The residue was purified by column chromatography (PE/EA = 10/1-1/1, v/v) to obtain compound **36a** (150 mg, yield: 48%).

LC-MS (ESI): m/z =393.1 [M+H]+

### Step 2:

### (1R,2S,3 S,4R)-ethyl-3-((2-(5-fluoro-1-tosyl-1H-pyrrolo[2,3-b]pyridin-3-yl)-6-(methoxymethyl)pyrrolo[2,1-f][1,2,4]triazin-4-yl)amino)bicyclo[2.2.2]octane-2-carboxylate (36b)

At room temperature, compound **36a** (150 mg, 0.38 mmol) was added to a mixed solution of dioxane (10 mL) and water (1 mL), and 5-fluoro-3-(4,4,5,5-tetramethyl-1,3,2-dioxaborolan-2-yl)-1-tosyl-1H-pyrrolo[2,3-b]pyridine (240 mg, 0.57 mmol), potassium carbonate (160 mg, 1.14 mmol) and Pd(dppf)Cl₂ (28 mg, 0.038 mmol) were successively added to the mixture. The mixture was subjected to nitrogen replacement and protection, heated to 120°C under microwave and reacted for 1 hour. The reaction solution was concentrated to dryness, and the residue was purified by column chromatography (PE/EA = 10/1-1/1, v/v) to obtain compound 36b (60 mg, yield: 24%).

### Step 3:

### (1R,2S,3S,4R)-3-((2-(5-fluoro-1H-pyrrolo[2,3-b]pyridin-3-yl)-6-(methoxymethyl)pyrrolo[2,1-f][1,2,4]triazin-4-yl)amino)bicyclo[2.2.2]octane-2-carboxylic acid (compound 36)

At room temperature, to compound **36b** (60 mg, 0.093 mmol) were successively added ethanol (1 mL), water (2 mL) and sodium hydroxide (74 mg, 1.86 mmol). The mixture was reacted at room temperature for 18 hours. The reaction solution was adjusted to pH 5-6 by adding 6 N hydrochloric acid and extracted with ethyl acetate (30 mL × 1). The organic phase was concentrated to dryness, and the residue was separated and purified by a preparative plate (DCM/MeOH = 10/1, v/v) to obtain compound **36** (20 mg, yield: 46%).

LC-MS (ESI): m/z =465.2[M+H]+.

¹H NMR (400 MHz,DMSO-d6) δ12.18 (s,1H), 8.52-8.49 (m,1H), 8.28-8.27 (m,1H), 8.18-8.17 (m,1H),7.87-7.85 (m,1H), 7.66-7.65 (m,1H), 6.97-6.96 (m,1H), 4.81-4.78 (m,1H), 4.46 (s,2H), 3.29 (s,3H), 2.76-2.75 (m,1H), 2.03-1.99 (m,2H), 1.81-1.73 (m,3H), 1.62-1.57 (m,3H), 1.49-1.40 (m,2H).

### Example 37:

### (1R,2S,3S,4R)-3-((7-cyclopropyl-2-(5-fluoro-1H-pyrazolo[3,4-b]pyridin-3-yl)pyrrolo[2,1-f][1,2,4]triazin-4-yl)amino)bicyclo[2.2.2]octane-2-carboxylic acid (compound 37)

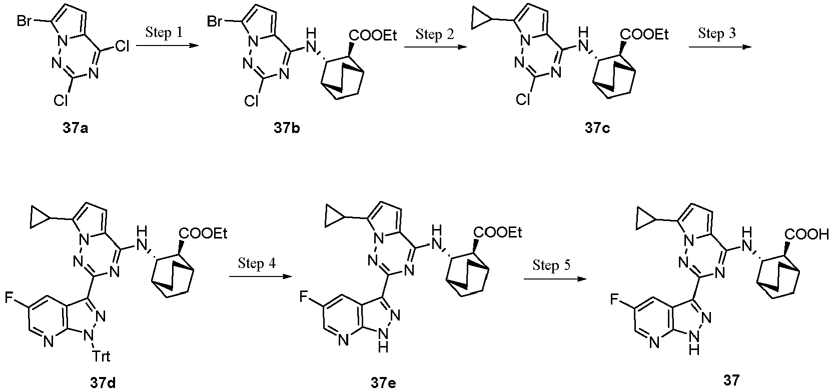

### Step 1:

### (1R,2S,3S,4R)-ethyl-3-((7-bromo-2-chloropyrrolo[2,1-f][1,2,4]triazin-4-yl)amino)bicyclo[2.2.2]octane-2-carboxylate (37b)

At room temperature, compound **37a** (0.5 g, 1.9 mmol) was added to DCM (20 mL), and ethyl (1R,2S,3S,4R)-3-aminobicyclo[2.2.2]octane-2-carboxylate hydrochloride (0.6 g, 2.4 mmol) and DIPEA (0.5 g, 3.8 mmol) were successively added to the mixture. The mixture was reacted at room temperature for 2 hours. The reaction solution was poured into water (50 mL) and extracted with DCM (50 mL × 1). The organic phase was concentrated to dryness, and the residue was purified by column chromatography (PE/EA, 20/1-10/1, v/v) to obtain compound **37b** (300 mg, yield: 37%).

LC-MS (ESI): m/z =427.1 [M+H]+.

### Step 2:

### (1R,2S,3S,4R)-ethyl-3-((2-chloro-7-cyclopropylpyrrolo[2,1-f][1,2,4]triazin-4-yl)amino)bicyclo[2.2.2]octane-2-carboxylate (37c)

At room temperature, compound **37b** (300 mg, 0.7 mmol) was added to a mixed solution of dioxane (5 mL) and water (0.5 mL), and cyclopropylboronic acid (120 mg, 1.4 mmol), potassium carbonate (240 mg, 1.7 mmol) and Pd(dppf)Cl₂ (30 mg, 0.04 mmol) were successively added to the mixture. The mixture was reacted under microwave at 120°C for 1 hour. The reaction solution was poured into water (30 mL) and extracted with ethyl acetate (30 mL × 1). The organic phase was concentrated to dryness, and the residue was purified by column chromatography (PE/EA, 20/1-10/1, v/v) to obtain compound 37c (200 mg, yield: 72%).

LC-MS (ESI): m/z =389.1 [M+H]+.

### Step 3:

### (1R,2S,3S,4R)-ethyl-3-((7-cyclopropyl-2-(5-fluoro-1-trityl-1H-pyrazolo[3,4-b]pyridin-3-yl)pyrrolo[2,1-f][1,2,4]triazin-4-yl)amino)bicyclo[2.2.2]octane-2-carboxylate (37d)

At room temperature, compound **37c** (200 mg, 0.5 mmol) was added to a mixed solution of dioxane (5 mL) and water (0.5 mL), and intermediate Int-2 (515 mg, 1.0 mmol), potassium carbonate (170 mg, 1.2 mmol) and Pd(dppf)Cl₂ (30 mg, 0.04 mmol) were successively added to the mixture. The mixture was reacted under microwave at 120°C for 1 hour. The reaction solution was poured into water (30 mL) and extracted with ethyl acetate (30 mL × 1). The organic phase was concentrated to dryness, and the residue was purified by column chromatography (PE/EA, 20/1-10/1, v/v) to obtain compound **37d** (30 mg, yield: 8%).

### Step 4:

### (1R,2S,3 S,4R)-ethyl-3-((7-cyclopropyl-2-(5-fluoro-1H-pyrazolo[3,4-b]pyridin-3-yl)pyrrolo[2,1-f][1,2,4]triazin-4-yl)amino)bicyclo[2.2.2]octane-2-carboxylate (37e)

At room temperature, compound **37d** (30 mg, 0.04 mmol) was added to DCM (10 mL), and trifluoroacetic acid (90 mg, 0.8 mmol) and triethylsilane (93 mg, 0.8 mmol) were successively added to the mixture. The mixture was reacted at room temperature for 5 hours. The reaction solution was poured into water (50 mL) and extracted with DCM (50 mL × 1). The organic phase was concentrated to dryness, and the residue was purified by a preparative plate (PE/EA, 1/1, v/v) to obtain compound 37e (10 mg, yield: 50%).

LC-MS (ESI): m/z =490.2 [M+H]+.

### Step 5:

### (1R,2S,3S,4R)-3-((7-cyclopropyl-2-(5-fluoro-1H-pyrazolo[3,4-b]pyridin-3-yl)pyrrolo[2,1-f][1,2,4]triazin-4-yl)amino)bicyclo[2.2.2]octane-2-carboxylic acid (compound 37)

At room temperature, to compound **37e** (10 mg, 0.02 mmol) were successively added ethanol (1 mL), water (2 mL) and sodium hydroxide (16 mg, 0.4 mmol). The mixture was reacted at room temperature for 18 hours. The reaction solution was adjusted to pH 5-6 by adding 6 N hydrochloric acid and extracted with ethyl acetate (30 mL × 1). The organic phase was concentrated to dryness, and the residue was separated and purified by a preparative plate (DCM/MeOH = 10/1, v/v) to obtain compound **37** (2.5 mg, yield: 25%).

LC-MS (ESI): m/z =462.1[M+H]+.

¹H NMR (400 MHz,DMSO-d6) δ8.64-8.63 (m,1H), 8.58-8.55 (m,1H), 7.96-7.94 (m,1H), 6.96-6.95 (m,1H), 6.32-6.31 (m,1H), 4.91-4.88 (m,1H), 2.84-2.82 (m,1H), 2.42-2.39 (m,1H), 2.05-2.03 (m,2H), 1.78-1.74 (m,3H), 1.61-1.41 (m,5H), 1.08-1.04 (m,2H), 0.80-0.77 (m,2H).

### Example 38:

### (1R,5S,6S,7S)-7-((7-cyclopropyl-2-(5-fluoro-1H-pyrazolo[3,4-b]pyridin-3-yl)pyrrolo[2,1-f][1,2,4]triazin-4-yl)amino)tricyclo[3.2.2.0^{2,4}]nonane-6-carboxylic acid (compound 38)

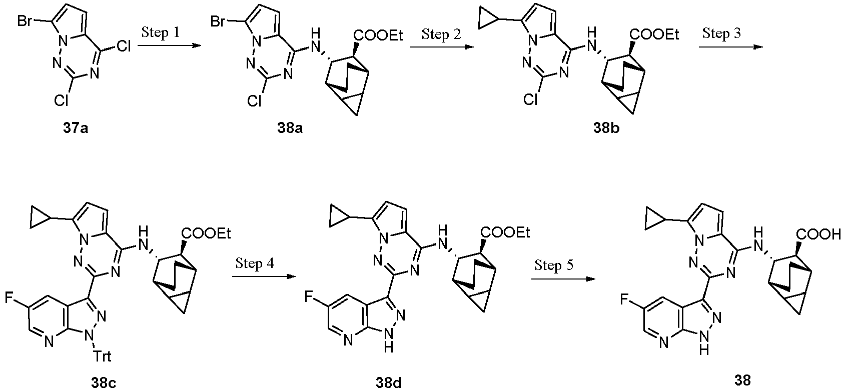

### Step 1:

### (1R,5S,6S,7S)-ethyl-7-((7-bromo-2-chloropyrrolo[2,1-f][1,2,4]triazin-4-yl)amino)tricyclo[3.2.2.0^{2,4}]nonane-6-carboxylate (38a)

At room temperature, compound **37a** (1.5 g, 5.7 mmol) was added to DCM (30 mL), and ethyl (1R,SS,6S,7S)-7-aminotricyclo[3.2.2.0^{2,4}]nonane-6-carboxylate hydrochloride (1.7 g, 6.8 mmol) and DIPEA (1.5 g, 11.4 mmol) were successively added to the mixture. The mixture was reacted at room temperature for 2 hours. The reaction solution was poured into water (50 mL) and extracted with DCM (50 mL × 1). The organic phase was concentrated to dryness, and the residue was purified by column chromatography (PE/EA, 20/1-10/1, v/v) to obtain compound 38a (800 mg, yield: 31%).

LC-MS (ESI): m/z =438.1 [M+H]+.

### Step 2:

### (1R,5S,6S,7S)-ethyl-7-((2-chloro-7-cyclopropylpyrrolo[2,1-f][1,2,4]triazin-4-yl)amino)tricyclo[3.2.2.0^{2,4}]nonane-6-carboxylate (38b)

At room temperature, compound **38a** (800 mg, 1.8 mmol) was added to a mixed solution of dioxane (10 mL) and water (1 mL), and cyclopropylboronic acid (310 mg, 3.6 mmol), potassium carbonate (620 mg, 4.5 mmol) and Pd(dppf)Cl₂ (140 mg, 0.2 mmol) were successively added to the mixture. The mixture was reacted under microwave at 120°C for 1 hour. The reaction solution was poured into water (50 mL) and extracted with ethyl acetate (5 mL × 1). The organic phase was concentrated to dryness, and the residue was purified by column chromatography (PE/EA, 20/1-10/1, v/v) to obtain compound **38b** (600 mg, yield: 83%).

LC-MS (ESI): m/z =401.1 [M+H]+.

### Step 3:

### (1R,5S,6S,7S)-ethyl-7-((7-cyclopropyl-2-(5-fluoro-1-trityl-1H-pyrazolo[3,4-b]pyridin-3-yl)pyrrolo[2,1-f][1,2,4]triazin-4-yl)amino)tricyclo[3.2.2.0^{2,4}]nonane-6-carboxylate (38c)

At room temperature, compound 38b (600 mg, 1.5 mmol) was added to a mixed solution of dioxane (10 mL) and water (1 mL), and intermediate Int-2 (1.5 g, 3.0 mmol), potassium carbonate (510 mg, 3.8 mmol) and Pd(dppf)Cl₂ (140 mg, 0.2 mmol) were successively added to the mixture. The mixture was reacted under microwave at 120°C for 1 hour. The reaction solution was poured into water (50 mL) and extracted with ethyl acetate (50 mL × 1). The organic phase was concentrated to dryness, and the residue was purified by column chromatography (PE/EA, 20/1-10/1, v/v) to obtain crude compound **38c** (700 mg).

### Step 4:

### (1R,5S,6S,7S)-ethyl-7-((7-cyclopropyl-2-(5-fluoro-1H-pyrazolo[3,4-b]pyridin-3-yl)pyrrolo[2,1-f][1,2,4]triazin-4-yl)amino)tricyclo[3.2.2.0^{2,4}]nonane-6-carboxylate (38d)

At room temperature, crude compound **38c** (700 mg, 0.9 mmol) was added to DCM (30 mL), and trifluoroacetic acid (2 g, 18 mmol) and triethylsilane (2.1 mg, 18 mmol) were successively added to the mixture. The mixture was reacted at room temperature for 5 hours. The reaction solution was poured into water (50 mL) and extracted with DCM (50 mL × 1). The organic phase was concentrated to dryness, and the residue was purified by a preparative plate (PE/EA, 20/1-3/1, v/v) to obtain compound **38d** (120 mg).

LC-MS (ESI): m/z =502.2 [M+H]+.

### Step 5:

### (1R,5S,6S,7S)-7-((7-cyclopropyl-2-(5-fluoro-1H-pyrazolo[3,4-b]pyridin-3-yl)pyrrolo[2,1-f][1,2,4]triazin-4-yl)amino)tricyclo[3.2.2.0^{2,4}]nonane-6-carboxylic acid (compound 38)

At room temperature, to compound **38d** (120 mg, 0.24 mmol) were successively added ethanol (3 mL), water (6 mL) and sodium hydroxide (190 mg, 4.8 mmol). The mixture was reacted at room temperature for 5 hours. The reaction solution was adjusted to pH 5-6 by adding 6 N hydrochloric acid. A solid was precipitated out and filtered. The obtained solid was rinsed with water (50 mL) and dried to obtain compound **38** (70 mg, yield: 62%).

LC-MS (ESI): m/z =474.2[M+H]+.

¹H NMR (400 MHz,DMSO-d6) δ8.64-8.63 (m,1H), 8.55-8.53 (m,1H), 7.63-7.62 (m,1H), 6.98-6.97 (m,1H), 6.28-6.27 (m,1H), 4.62-4.59 (m,1H), 2.83-2.75 (m,2H), 2.46-2.39 (m,2H), 1.71-1.54 (m,4H), 1.09-1.04 (m,4H), 0.79-0.73 (m,3H), 0.33-0.27 (m,1H).

### Example 39:

### (1R,6S,7S,8S)-8-((2-(5-fluoro-1H-pyrazolo[3,4-b]pyridin-3-yl)-7-(methoxymethyl)pyrrolo[2,1-f][1,2,4]triazin-4-yl)amino)tricyclo[4.2.2.02,5]decane-7-carboxylic acid (compound 39)

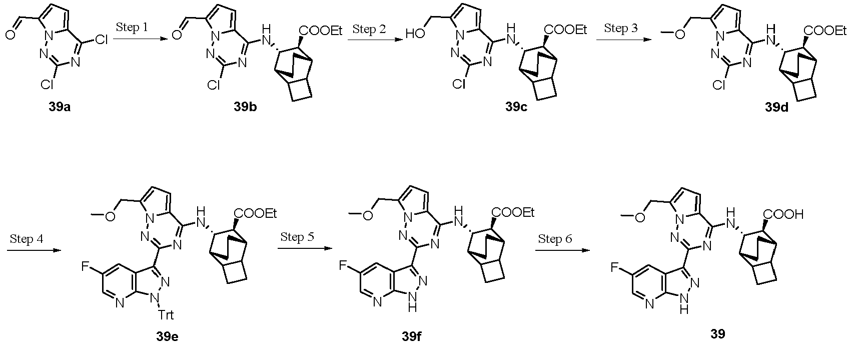

### Step 1:

### (1R,6S,7S,8S)-ethyl-8-((2-chloro-7-formylpyrrolo[2,1-f][1,2,4]triazin-4-yl)amino)tricyclo[4.2.2.02,5]decane-7-carboxylate (39b)

At room temperature, compound **39a** (same as compound **11b**) (3 g, 13.9 mmol) and DIPEA (4.5 g, 35 mmol) were added to a mixed solution of DCM (30 mL) and isopropanol (60 mL), and the mixture was reacted at 60°C for 10 minutes. Ethyl (1R,6S,7S,8S)-8-aminotricyclo[4.2.2.02,5]decan-7-carboxylate (2.5 g, 11.2 mmol) was added to the reaction solution, and the mixture was reacted for 50 minutes with the temperature maintained. The reaction solution was concentrated to dryness. Water (50 mL) was added, and the mixture was extracted with EA (50 mL × 1). The organic phase was concentrated to dryness, and the residue was purified by column chromatography (PE/EA, 20/1-10/1, v/v) to obtain compound 39b (3 g, yield: 53%).

LC-MS (ESI): m/z =403.2 [M+H]+.

### Step 2:

### (1R,6S,7S,8S)-ethyl-8-((2-chloro-7-(hydroxymethyl)pyrrolo[2,1-f][1,2,4]triazin-4-yl)amino)tricyclo[4.2.2.02,5]decane-7-carboxylate (39c)

At room temperature, compound **39b** (3 g, 7.5 mmol) was added to methanol (50 mL), followed by sodium borohydride (340 mg, 9.0 mmol). The mixture was reacted at room temperature for 1 hour. The reaction solution was poured into water (100 mL) and extracted with EA (50 mL × 1). The organic phase was concentrated to dryness to obtain compound **39c** (2.8 g, yield: 93%).

LC-MS (ESI): m/z =405.2 [M+H]+.

### Step 3:

### (1R,6S,7S,8S)-ethyl-8-((2-chloro-7-(methoxymethyl)pyrrolo[2,1-f][1,2,4]triazin-4-yl)amino)tricyclo[4.2.2.02,5]decane-7-carboxylate (39d)

At room temperature, compound **39c** (2.8 g, 6.9 mmol) was added to methanol (20 mL). Trimethylsilyldiazomethane (50 mL, 100 mmol) was added and after the addition, trifluoroacetic acid (11.4 g, 100 mmol) was added dropwise to the reaction solution. The mixture was reacted at room temperature for 2 hours. The reaction solution was poured into water (100 mL) and extracted with EA (100 mL × 1). The organic phase was concentrated to dryness, and the residue was purified by column chromatography (PE/EA, 10/1-5/1, v/v) to obtain compound **39d** (1.2 g, yield: 41%).

LC-MS (ESI): m/z =419.2 [M+H]+.

### Step 4:

### (1R,6S,7S,8S)-ethyl-8-((2-(5-fluoro-1-trityl-1H-pyrazolo[3,4-b]pyridin-3-yl)-7-(methoxymethyl)pyrrolo[2,1-f][1,2,4]triazin-4-yl)amino)tricyclo[4.2.2.02,5]decane-7-carboxylate (39e)

At room temperature, compound 39d (1.2 g, 2.86 mmol) was added to a mixed solution of dioxane (15 mL) and water (1.5 mL), and intermediate Int-2 (2.9 g, 5.7 mmol), potassium carbonate (1 g, 7.2 mmol) and Pd(dppf)Cl₂ (210 mg, 0.29 mmol) were successively added to the mixture. The mixture was reacted under microwave at 120°C for 1 hour. The reaction solution was concentrated to dryness and directly purified by column chromatography ((PE/EA, 10/1-5/1, v/v) to obtain crude compound **39e** (400 mg).

### Step 5:

### (1R,6S,7S,8S)-ethyl-8-((2-(5-fluoro-1H-pyrazolo[3,4-b]pyridin-3-yl)-7-(methoxymethyl)pyrrolo[2,1-f][1,2,4]triazin-4-yl)amino)tricyclo[4.2.2.02,5]decane-7-carboxylate (39f)

At room temperature, crude compound 39e (400 mg, 0.53 mmol) was added to DCM (20 mL), and trifluoroacetic acid (1.2 g, 10.6 mmol) and triethylsilane (1.2 g, 10.6 mmol) were successively added to the mixture. The mixture was reacted at room temperature for 16 hours. The reaction solution was concentrated to dryness, and the residue was purified by column chromatography (PE/EA, 10/1-1/1, v/v) to obtain compound **39f** (100 mg, yield: 36%).

LC-MS (ESI): m/z =520.2 [M+H]+.

### Step 6:

### (1R,6S,7S,8S)-8-((2-(5-fluoro-1H-pyrazolo[3,4-b]pyridin-3-yl)-7-(methoxymethyl)pyrrolo[2,1-f][1,2,4]triazin-4-yl)amino)tricyclo[4.2.2.02,5]decane-7-carboxylic acid (compound 39)

At room temperature, to compound **39f** (100 mg, 0.19 mmol) were successively added ethanol (2 mL), water (4 mL) and sodium hydroxide (150 mg, 3.8 mmol). The mixture was reacted at room temperature for 16 hours. Water (10 mL) was added to the reaction solution, and the mixture was adjusted to pH 5-6 by adding 6 N hydrochloric acid. A solid was precipitated out and filtered. The obtained solid was rinsed with water (5 mL) and dried to obtain compound **39** (30 mg, yield: 31.5%).

LC-MS (ESI): m/z =492.2[M+H]+.

¹H NMR (400 MHz,DMSO-d6) δ8.65-8.64 (m,1H), 8.52-8.50 (m,1H), 8.13-8.12 (m,1H), 7.05-7.04 (m,1H), 6.74-6.73 (m,1H), 4.78 (s,2H), 3.33 (s,3H), 2.96-2.94 (m,1H), 2.69-2.41 (m,2H), 2.21-1.98 (m,10H), 1.85-1.71 (m,2H).

### Biological test

Experimental method: the cytopathic effect (CPE) method was used to test the antiviral activity of the compounds of the present invention against influenza viruses, and the MDCK cytotoxicity was also measured. The compounds were tested for 8 concentrations (double replicate wells). CCK-8 reagent was used to detect cell viability. MDCK cells were seeded at a certain density in a microplate and cultured overnight at 37°C and 5% CO₂. The compounds and viruses were added the next day. Cell control (without compound treatment or virus infection) and virus infection control (cells infected with virus and without compound treatment) were provided. The final concentration of DMSO in the cell culture medium was 0.5%. The cells were cultured at 37°C and 5% CO₂ for 5 days until the rate at which a cytopathic effect appears in virus control wells reached 80%-95%. CCK-8 reagent was used to detect cell viability, and the raw data was used to calculate the antiviral activity of the compounds. GraphPad Prism software was used to analyse dose response curves of the compounds and calculate EC50 values.

Test results: the compounds of the present invention exhibit an anti-proliferative activity against virus IFV A/PR/8/34. The IC50 values of the example compounds against virus IFV A/PR/8/34 were in the range of 0.01-3 nM. The test results of some examples were shown in Table 1:

**Table 1 Anti-proliferative activity results of compounds of the present invention against virus IFV A/PR/8/34**

| Examples | EC50 (IFV A/PR/8/34) nM |
|---|---|
| 8 | 0.8 |
| 18 | 0.3 |
| 20 | 0.3 |
| 30 | 0.37 |
| 31 | 0.08 |
| 32 | 0.24 |
| 38 | 0.09 |

Conclusion: the compounds of the present invention exhibit a high anti-proliferative activity against virus IFV A/PR/8/34.

## Claims

1. A compound of formula (I), or a stereoisomer, a tautomer, a nitrogen oxide, a solvate, a metabolite, a pharmaceutically acceptable salt, a co-crystal or a prodrug thereof,
C_{y1}-L₁-C_{y2}-L₂-C_{y3} (I)
wherein C_{y1} is selected from 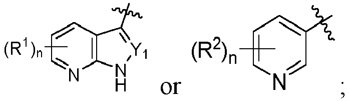
Y₁ is selected from -CH- or -N-;
n is selected from 1, 2 or 3;
R¹ and R² are each independently selected from F, Cl, Br, NH₂, C₁₋₆ alkyl or C₃₋₆ monocyclic cycloalkyl, and the R¹ and R² are optionally further substituted with 1-5 groups selected from halogen, OH or deuterium;
C_{y2} is selected from a bond, C₃₋₁₂ carbocycle or 3-12-membered heterocycle, and the C_{y2} is optionally further substituted with 1-5 groups selected from R^{A};
each R^{A} is independently selected from halogen, C₁₋₆ alkyl, C₂₋₆ alkenyl, C₂₋₆ alkynyl, 5-12-membered heteroaryl, phenyl, -NR^{B}C(O)C₁₋₆ alkyl, -C(O)NR^{B}C₁₋₆ alkyl, -C(O)C₁₋₆ alkyl, CN, =O, C₁₋₆ alkoxy C(O)-, -COOH, OH, -NR^{B}R^{C}, deuterium, -SH, -S(C₁₋₆ alkyl), -S(O)₂(C₁₋₆ alkyl) or C₃₋₆ monocyclic cycloalkyl, and the R^{A} is optionally further substituted with 1-5 groups selected from halogen, C₁₋₆ alkyl, C₁₋₆ alkoxy, C₁₋₆ alkylamino, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) or deuterium;
C_{y3} is selected from 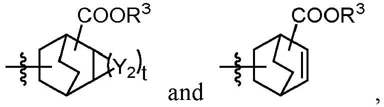 and the C_{y3} is optionally substituted with 1-5 groups selected from R_{cy3};
t is selected from 0, 1, 2, 3 or 4;
each Y₂ is independently selected from -CH₂-, -N-, -NH-, O, S, S(O) and S(O)₂, and each H atom in the Y₂ can be independently substituted with R_{cy3} group;
each R_{cy3} is independently selected from F, Cl, Br, OH, CN, C₁₋₆ alkyl, C₁₋₆ alkoxy, halo C₁₋₆ alkyl and hydroxy C₁₋₆ alkyl;
each R³ is independently selected from H and C₁₋₆ alkyl;
L₁ is selected from a bond, C₂₋₆ alkynylene, C₂₋₆ alkenylene or -C(O)-;
L₂ is selected from a bond, -NR^{B}- or -C(O)NR^{B}-;
and R^{B} and R^{C} are each independently selected from H, deuterium, C₁₋₆ alkyl, halo C₁₋₆ alkyl, -OC(O)CH₃ or deuterated C₁₋₆ alkyl.

2. The compound of formula (I), or the stereoisomer, the tautomer, the nitrogen oxide, the solvate, the metabolite, the pharmaceutically acceptable salt, the co-crystal or the prodrug thereof according to claim 1, having a structure of formula (II), (III), (IV) or (V):
C_{y1}-C_{y2a}-NH-C_{y3} (II)
C_{y1}-C_{y2b}-C_{y3} (III)
C_{y1}-L₁ₐ-C_{y2c}-L₂ₐ-C_{y3} (IV)
C_{y1}-L_{1b}-L_{2b}-C_{y3} (V)
wherein
C_{y1} is selected from 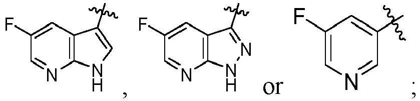
C_{y3} is selected from 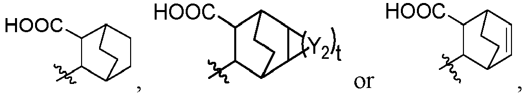 and the C_{y3} is optionally substituted with 1-5 groups selected from R_{cy3};
each Y₂ is independently selected from -CH₂-, -N-, -NH-, O, S, S(O) and S(O)₂, and each H atom in the Y₂ can be independently substituted with R_{cy3} group;
t is selected from 1, 2, 3 or 4;
each R_{cy3} is independently selected from F, Cl, Br, OH, CN, C₁₋₄ alkyl, C₁₋₄ alkoxy, halo C₁₋₄ alkyl or hydroxy C₁₋₄ alkyl;
C_{y2a} is selected from the following groups, wherein ^{∗} represents the coupling site of C_{y2a} with NH:
(a) 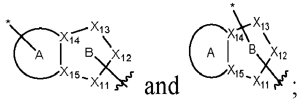
(b) 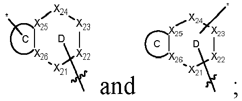
(c) 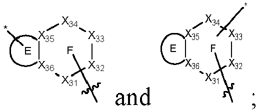
(d) 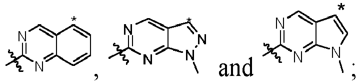
(e) 5-membered heteroaryl, 5-membered unsaturated monocyclic heterocycloalkyl and 6-membered saturated monocyclic heterocycloalkyl;
(f) 6-membered unsaturated monocyclic heterocycloalkyl;
(g) 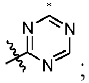
and (h) 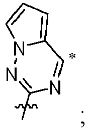
wherein the groups of (a) - (f) in C_{y2a} are optionally further substituted with 1-5 groups selected from R^{A}, and the group of (g) in C_{y2a} is substituted with 1 group selected from R^{D};
when C_{y1} is selected from 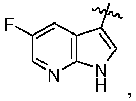 the group of (h) in C_{y2a} is substituted with 1-3 groups selected from R^{E}; or
when C_{y1} is selected from 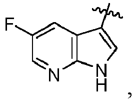 and C_{y2a} is (h), C_{y3} is substituted with 1-5 groups selected from R_{cy3}; or
when C_{y1} is selected from 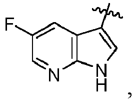 and C_{y2a} is (h), C_{y3} is selected from 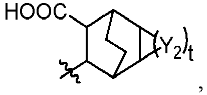 and t = 2, 3 or 4;
when C_{y1} is selected from 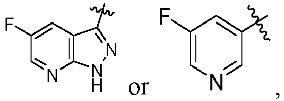 the group of (h) in C_{y2a} is optionally further substituted with 1-3 groups selected from R^{A};
each ring A is independently selected from 4-6-membered monocyclic cycloalkyl, 4-6-membered monocyclic heterocycloalkyl, 5-6-membered heteroaryl or phenyl;
each ring C is independently selected from 5-membered monocyclic cycloalkyl, 5-membered monocyclic heterocycloalkyl or 5-membered heteroaryl;
each ring E is independently selected from 6-membered monocyclic cycloalkyl, 6-membered monocyclic heterocycloalkyl, 6-membered heteroaryl or phenyl;
X₁₁, X₁₂ and X₁₃ are each independently selected from -CH-, -CR^{A}-, -CH₂-,-CHR^{A}-, -C(R^{A})₂-, -N-, -NH-, -NR^{A}-, O, S, S(O) or S(O)₂, X₁₄ and X₁₅ are each independently selected from -C-, -CH-, -CR^{A}- or -N-, and ring B is a saturated ring, a partially unsaturated ring or a heteroaryl ring;
X₂₁, X₂₂, X₂₃ and X₂₄ are each independently selected from -CH-, -CR^{A}-, -CH₂-, -CHR^{A}-, -C(R^{A})₂-, -N-, -NH-, -NR^{A}-, O, S, S(O) or S(O)₂, X₂₅ and X₂₆ are each independently selected from -C-, -CH-, -CR^{A}- or -N-, and ring D is a saturated ring, a partially unsaturated ring, a benzene ring or a heteroaryl ring, provided that D-fused ring C does not form substituted or unsubstituted 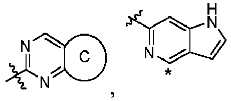 and 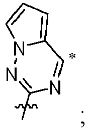
X₃₁, X₃₂, X₃₃ and X₃₄ are each independently selected from -CH-, -CR^{A}-, -CH₂-, -CHR^{A}-, -C(R^{A})₂-, -N-, -NH-, -NR^{A}-, O, S, S(O) or S(O)₂, X₃₅ and X₃₆ are each independently selected from -C-, -CH-, -CR^{A}- or -N-, and ring F is a saturated ring, a partially unsaturated ring, a benzene ring or a heteroaryl ring, provided that F-fused ring E does not form substituted or unsubstituted 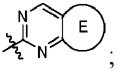
each R^{A} is independently selected from halogen, C₁₋₆ alkyl, C₁₋₆ alkoxy, C₂₋₆ alkenyl, C₂₋₆ alkynyl, 5-12-membered heteroaryl, phenyl, -NR^{B}C(O)C₁₋₆ alkyl, - C(O)NR^{B}C₁₋₆ alkyl, -C(O)C₁₋₆ alkyl, CN, =O, C₁₋₆ alkoxy C(O)-, -COOH, OH, - NR^{B}R^{C}, deuterium, -SH, -S(C₁₋₆ alkyl), -S(O)₂(C₁₋₆ alkyl) and C₃₋₆ monocyclic cycloalkyl, and the R^{A} is optionally further substituted with 1-5 groups selected from halogen, C₁₋₆ alkyl, C₁₋₆ alkoxy, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) and deuterium;
each R^{D} is selected from C₁₋₆ alkyl, 5-6-membered heteroaryl and phenyl, and the heteroaryl and phenyl are substituted with 1-2 groups selected from -Si(C₁₋₆ alkyl)₃;
each R^{E} is selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-12-membered heteroaryl, phenyl,-NR^{B}C(O)C₁₋₆ alkyl, -C(O)NR^{B}C₁₋₆ alkyl, -C(O)C₁₋₆ alkyl, =O, C₁₋₆ alkoxy C(O)-,-COOH, OH, -NR^{B}R^{C}, deuterium, -SH, -S(C₁₋₆ alkyl), -S(O)₂(C₁₋₆ alkyl), C₃₋₆ monocyclic cycloalkyl and C₃₋₆ monocyclic heterocyclic cycloalkyl, and the R^{E} is optionally further substituted with 1-5 groups selected from halogen, C₁₋₆ alkyl, C₁₋₆ alkoxy, C₁₋₆ alkylamino, halo C₁₋₆ alkyl, halo C₁₋₆ alkoxy, -Si(C₁₋₆ alkyl)₃, OH, 5-6-membered heteroaryl, C₃₋₆ monocyclic cycloalkyl, -S(C₁₋₆ alkyl) and deuterium;
C_{y2b} is selected from 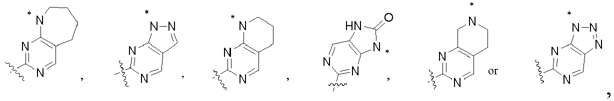 wherein ^{∗} represents the coupling site of C_{y2b} with 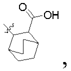 and the C_{y2b} is optionally substituted with 1-5 groups selected from R^{A};
C_{y2c} is selected from C₃₋₁₂ carbocycle and 5-12-membered heterocycle, and the C_{y2c} is optionally further substituted with 1-5 groups selected from R^{A};
L₁ₐ is selected from a bond, C₂₋₄ alkynylene, C₂₋₄ alkenylene or -C(O)-; L₂ₐ is selected from -NR^{B}- and -^{∗}C(O)NR^{B}-, wherein ^{∗} represents the coupling site of L₂ₐ with C_{y2c}, provided that when L₁ₐ is selected from a bond, L₂ₐ is selected from-^{∗}C(O)NR^{B}-;
L_{1b} is selected from C₂₋₄ alkynylene or C₂₋₄ alkenylene, and L_{2b} is selected from -NR^{B}- or -^{∗}C(O)NR^{B}-, wherein ^{∗} represents the coupling site of L_{2b} with L_{1b};
R^{B} and R^{C} are each independently selected from H, deuterium, C₁₋₆ alkyl,-OC(O)CH₃, halo C₁₋₆ alkyl and deuterated C₁₋₆ alkyl;
provided that C_{y2a} is not selected from substituted or unsubstituted 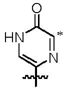 and substituted or unsubstituted

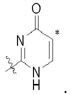

3. The compound of formula (I), or the stereoisomer, the tautomer, the nitrogen oxide, the solvate, the metabolite, the pharmaceutically acceptable salt, the co-crystal or the prodrug thereof according to claim 2, wherein
C_{y1} is selected from 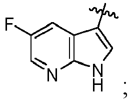 or
C_{y1} is selected from 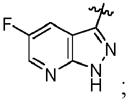 or
C_{y1} is selected from

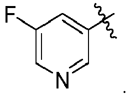

4. The compound of formula (I), or the stereoisomer, the tautomer, the nitrogen oxide, the solvate, the metabolite, the pharmaceutically acceptable salt, the co-crystal or the prodrug thereof according to claim 2 or 3, wherein
C_{y3} is selected from 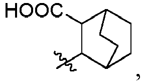 and the C_{y3} is optionally substituted with 1-3 groups selected from R_{cy3}; or
C_{y3} is selected from 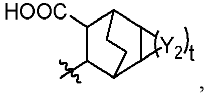 and the C_{y3} is optionally substituted with 1-3 groups selected from R_{cy3}, wherein t is selected from 1, 2 or 3, each Y₂ is independently selected from -CH₂-, -NH-, O and S, and each H atom in the Y₂ can be independently substituted with R_{cy3} group; or
C_{y3} is selected from 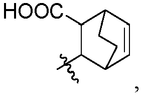 and the C_{y3} is optionally substituted with 1-3 groups selected from R_{cy3}.

5. The compound of formula (I), or the stereoisomer, the tautomer, the nitrogen oxide, the solvate, the metabolite, the pharmaceutically acceptable salt, the co-crystal or the prodrug thereof according to claim 4, wherein
C_{y3} is selected from

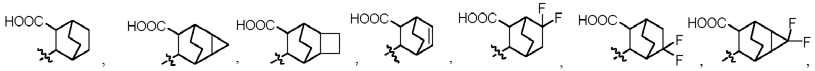

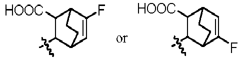

6. The compound of formula (I), or the stereoisomer, the tautomer, the nitrogen oxide, the solvate, the metabolite, the pharmaceutically acceptable salt, the co-crystal or the prodrug thereof according to claim 2, wherein
each R_{cy3} is independently selected from F, Cl and CN.

7. The compound of formula (I), or the stereoisomer, the tautomer, the nitrogen oxide, the solvate, the metabolite, the pharmaceutically acceptable salt, the co-crystal or the prodrug thereof according to claim 2, wherein
C_{y3} is selected from 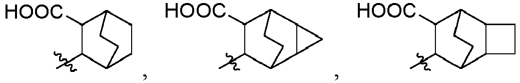 or 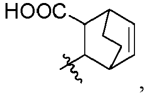 and the C_{y3} is optionally substituted with 1-3 groups selected from R_{cy3}; and C_{y1} is selected from

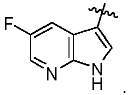

8. The compound of formula (I), or the stereoisomer, the tautomer, the nitrogen oxide, the solvate, the metabolite, the pharmaceutically acceptable salt, the co-crystal or the prodrug thereof according to claim 2, wherein
C_{y2a} is selected from 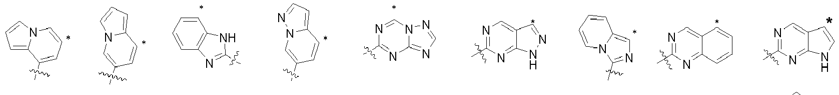 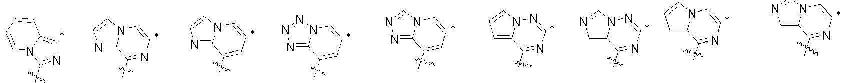 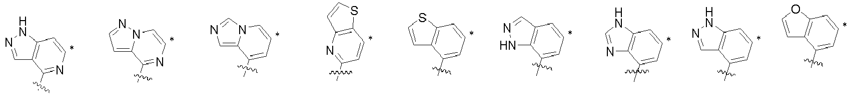 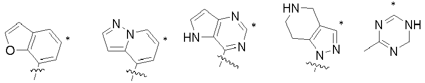 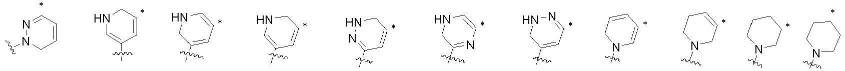 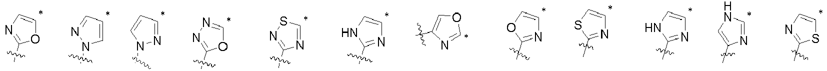 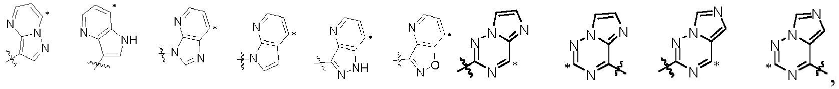 and the C_{y2a} is optionally substituted with 1-3 groups selected from R^{A}; or
C_{y2a} is selected from 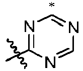 and is substituted with 1 group selected from methyl or 5-membered heteroaryl, and the 5-membered heteroaryl is substituted with 1 trimethylsilyl group; or
C_{y1} is selected from 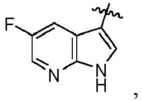 C_{y2a} is selected from 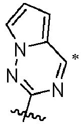 and is substituted with 1 group selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, - COOH, OH, -NR^{B}R^{C}, deuterium, C₃₋₆ monocyclic cycloalkyl or C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium; or
C_{y1} is selected from 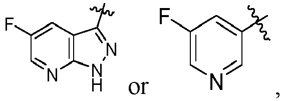 C_{y2a} is selected from 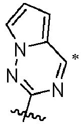 and is optionally substituted with 1-2 groups selected from hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, -COOH, OH, -NR^{B}R^{C}, deuterium, C₃₋₆ monocyclic cycloalkyl and C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium.

9. The compound of formula (I), or the stereoisomer, the tautomer, the nitrogen oxide, the solvate, the metabolite, the pharmaceutically acceptable salt, the co-crystal or the prodrug thereof according to claim 2, wherein
C_{y1} is selected from 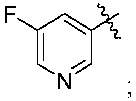
C_{y2a} is selected from a five-membered ring fused five-membered ring, a five-membered ring fused six-membered ring, a six-membered ring fused five-membered ring and a six-membered ring fused six-membered ring, and the C_{y2a} is optionally further substituted with 1-5 groups selected from R^{A}; or
C_{y2a} is selected from a five-membered heteroaryl fused six-membered heteroaryl, a six-membered heteroaryl fused six-membered heteroaryl, a five-membered heteroaryl fused five-membered heteroaryl and a six-membered heteroaryl fused five-membered heteroaryl, the C_{y2a} is optionally further substituted with 1-3 groups selected from halogen, hydroxy C₁₋₆ alkyl, halo C₁₋₆ alkyl, C₁₋₆ alkoxy, C₁₋₆ alkoxy-C₁₋₆ alkyl, C₂₋₆ alkenyl, C₂₋₆ alkynyl, 5-6-membered heteroaryl, -NR^{B}C(O)C₁₋₆ alkyl, -C(O)NR^{B}C₁₋₆ alkyl, -C(O)C₁₋₆ alkyl, C₁₋₆ alkoxy C(O)-, - COOH, OH, -NR^{B}R^{C}, deuterium, C₃₋₆ monocyclic cycloalkyl and C₃₋₆ monocyclic heterocyclic cycloalkyl, and the heteroaryl is optionally further substituted with 1-3 groups selected from halogen, C₁₋₆ alkyl, C₁₋₆ alkoxy, halo C₁₋₆ alkyl, halo C₁₋₆ alkoxy, -Si(C₁₋₃ alkyl)₃, OH, 5-6-membered heteroaryl, C₃₋₆ monocyclic cycloalkyl, -S(C₁₋₆ alkyl) and deuterium; or
C_{y2a} is selected from a five-membered heteroaryl fused six-membered heteroaryl, a six-membered heteroaryl fused six-membered heteroaryl, a five-membered heteroaryl fused five-membered heteroaryl and a six-membered heteroaryl fused five-membered heteroaryl, and the C_{y2a} is optionally further substituted with 1-3 groups selected from halogen and -NR^{B}R^{C}; or
C_{y2a} is selected from 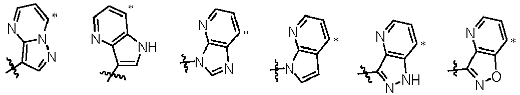 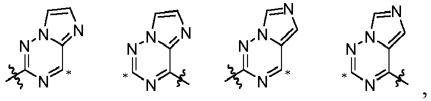 and the C_{y2a} is optionally further substituted with 1-3 groups selected from halogen and -NR^{B}R^{C}; or
C_{y2a} is selected from

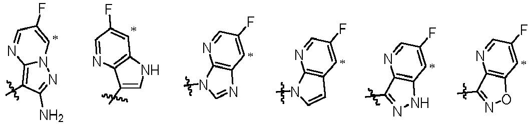

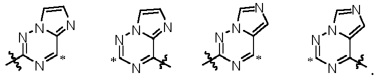

10. The compound of formula (I), or the stereoisomer, the tautomer, the nitrogen oxide, the solvate, the metabolite, the pharmaceutically acceptable salt, the co-crystal or the prodrug thereof according to claim 2, wherein
C_{y1} is selected from 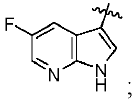 and C_{y2a} is selected from

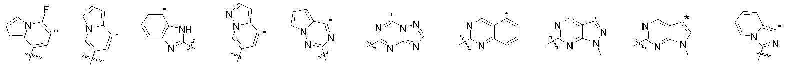

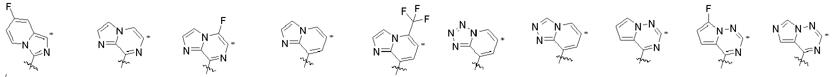

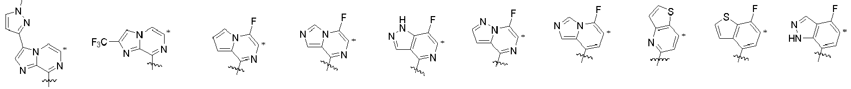

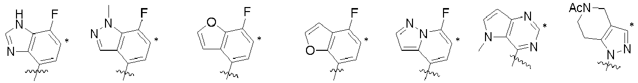

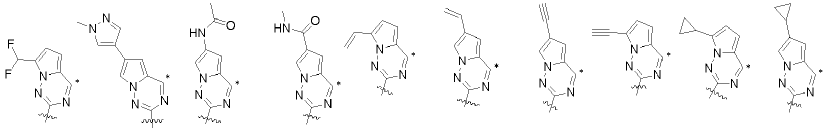

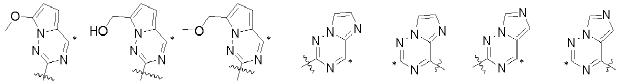

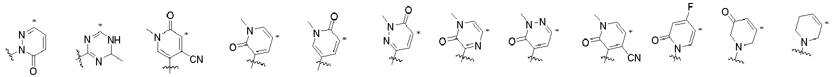

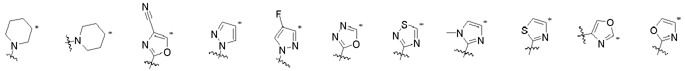

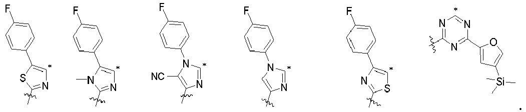

11. The compound of formula (I), or the stereoisomer, the tautomer, the nitrogen oxide, the solvate, the metabolite, the pharmaceutically acceptable salt, the co-crystal or the prodrug thereof according to claim 2, wherein
C_{y1} is selected from 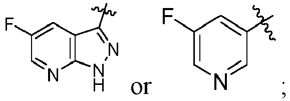 and C_{y2a} is selected from

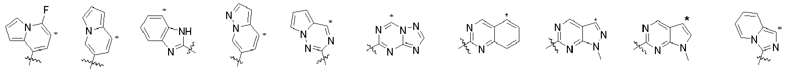

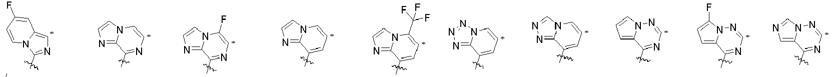

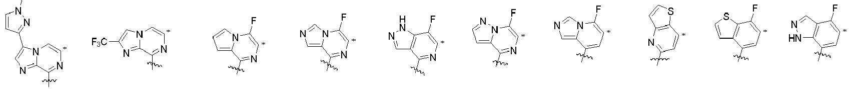

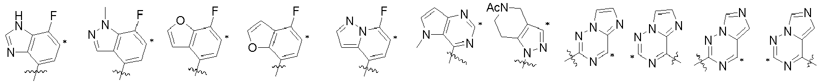

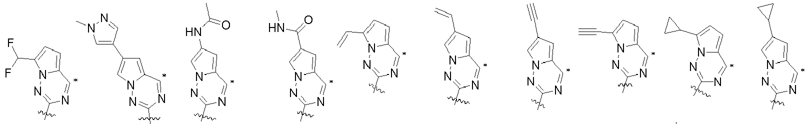

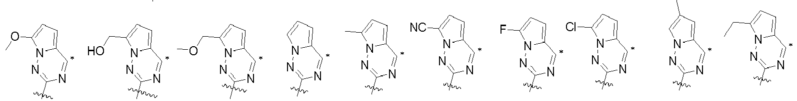

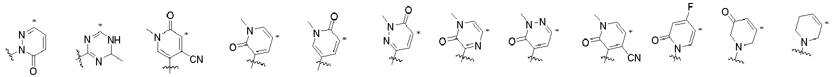

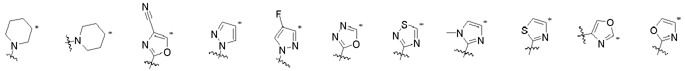

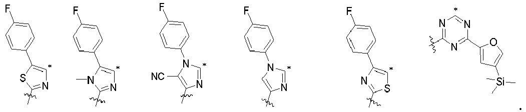

12. The compound of formula (I), or the stereoisomer, the tautomer, the nitrogen oxide, the solvate, the metabolite, the pharmaceutically acceptable salt, the co-crystal or the prodrug thereof according to claim 2 or 3, wherein
C_{y2b} is selected from 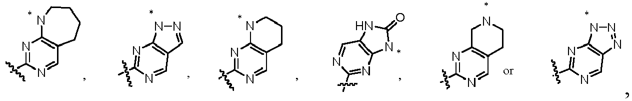 wherein * represents the coupling site of C_{y2b} with

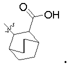

13. The compound of formula (I), or the stereoisomer, the tautomer, the nitrogen oxide, the solvate, the metabolite, the pharmaceutically acceptable salt, the co-crystal or the prodrug thereof according to claim 2 or 3, wherein
C_{y2c} is selected from 5-membered heteroaryl, 6-membered heteroaryl or C₃₋₆ cycloalkyl, and the C_{y2c} is optionally further substituted with 1-5 groups selected from R^{A}; or
C_{y2c} is selected from 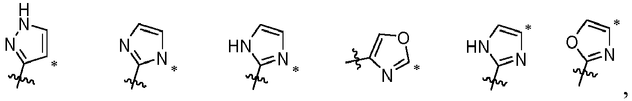 and the C_{y2c} is optionally substituted with 1-3 groups selected from R^{A}; or
C_{y2c} is selected from

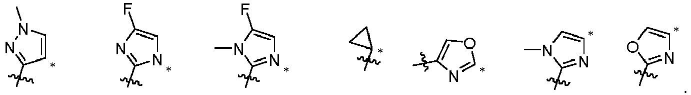

14. The compound of formula (I), or the stereoisomer, the tautomer, the nitrogen oxide, the solvate, the metabolite, the pharmaceutically acceptable salt, the co-crystal or the prodrug thereof according to any one of claims 1 to 3, wherein
each R^{A} is independently selected from halogen, C₁₋₄ alkyl, 5-6-membered heteroaryl, phenyl, -NR^{B}C(O)C₁₋₄ alkyl, -C(O)NR^{B}C₁₋₄ alkyl, -C(O)C₁₋₄ alkyl, CN, =O, -C(O)C₁₋₄ alkoxy, C₂₋₆ alkenyl, C₂₋₆ alkynyl, -COOH, OH, -NR^{B}R^{C}, deuterium, -SH, -S(C₁₋₄ alkyl), -S(O)₂(C₁₋₄ alkyl) and C₃₋₆ monocyclic cycloalkyl, and the R^{A} is optionally further substituted with 1-3 groups selected from halogen, C₁₋₄ alkyl, C₁₋₄ alkoxy, halo C₁₋₄ alkyl, halo C₁₋₄ alkoxy, C₁₋₆ alkylamino, -Si(C₁₋₄ alkyl)₃, OH, 5-6-membered heteroaryl, C₃₋₆ monocyclic cycloalkyl, -S(C₁₋₄ alkyl) and deuterium; or
each R^{A} is independently selected from halogen, C₁₋₄ alkyl, 5-6-membered heteroaryl, phenyl, -NR^{B}C(O)C₁₋₄ alkyl, -C(O)NR^{B}C₁₋₄ alkyl, -C(O)C₁₋₄ alkyl, CN, =O and amino, and the R^{A} is optionally further substituted with 1-3 groups selected from halogen, C₁₋₂ alkyl and -Si(C₁₋₂ alkyl)₃.

15. The compound of formula (I), or the stereoisomer, the tautomer, the nitrogen oxide, the solvate, the metabolite, the pharmaceutically acceptable salt, the co-crystal or the prodrug thereof according to any one of claims 1 to 3, wherein
R^{B} and R^{C} are each independently selected from H, deuterium, -OC(O)CH₃ or C₁₋₃ alkyl.

16. The compound of formula (I), or the stereoisomer, the tautomer, the nitrogen oxide, the solvate, the metabolite, the pharmaceutically acceptable salt, the co-crystal or the prodrug thereof according to claim 1, wherein the compound is selected from one of the following structures:

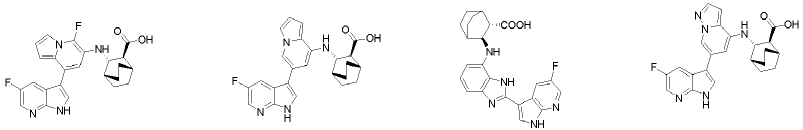

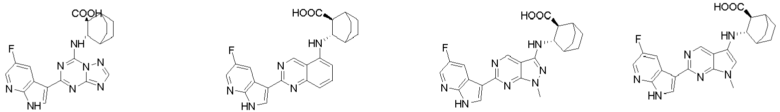

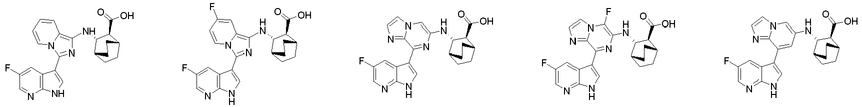

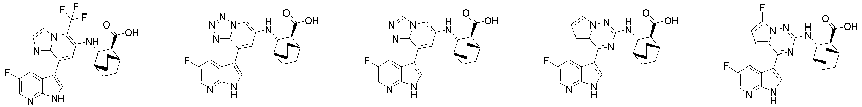

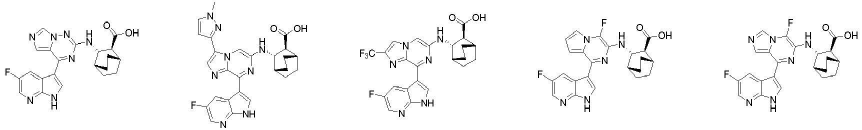

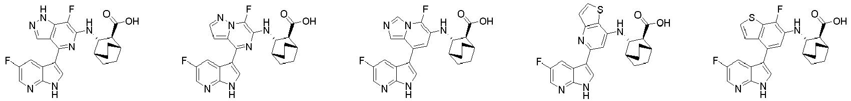

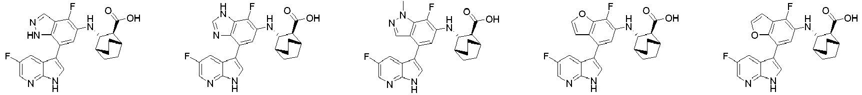

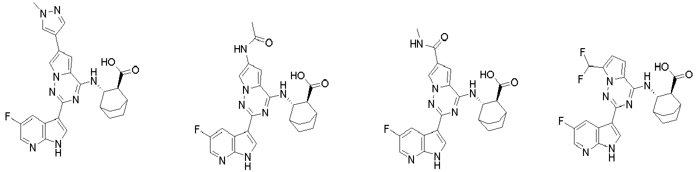

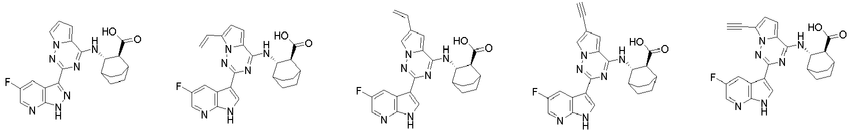

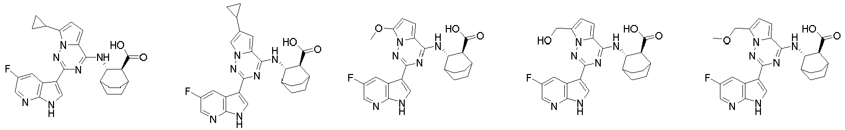

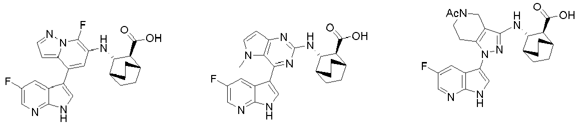

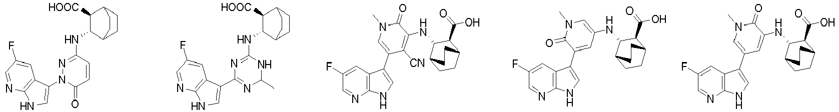

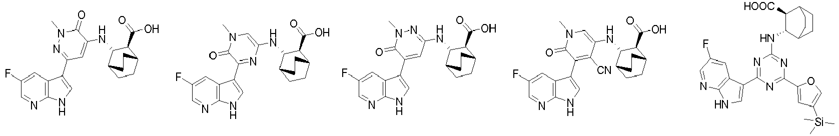

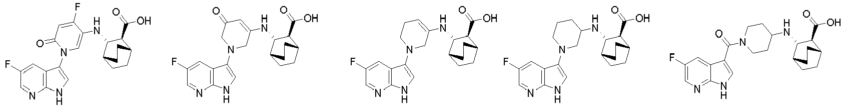

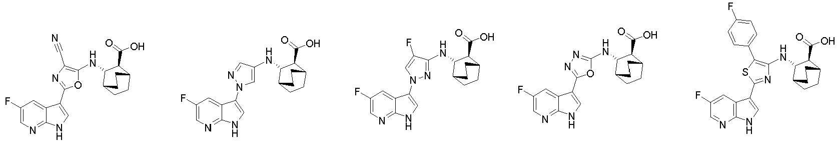

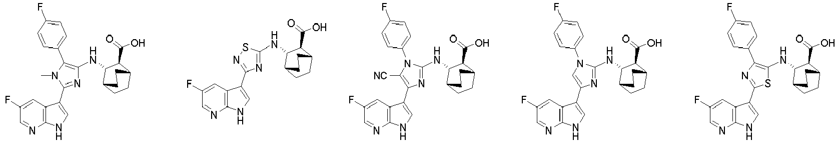

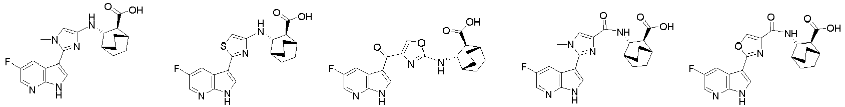

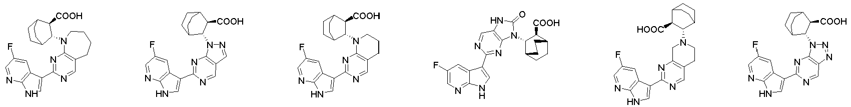

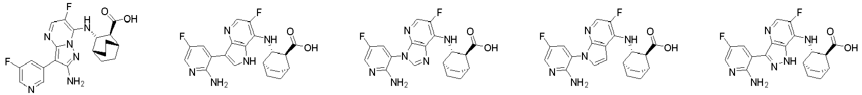

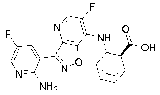

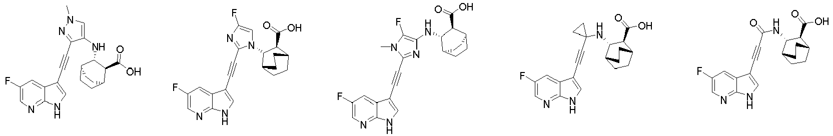

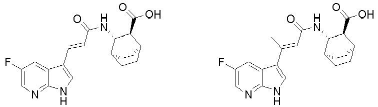

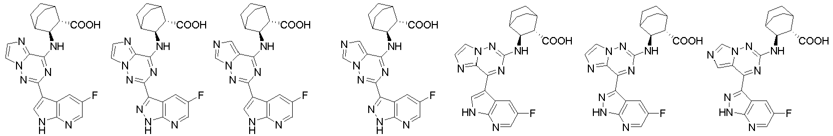

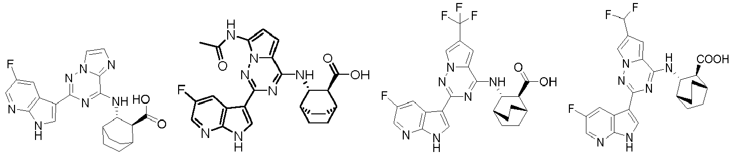

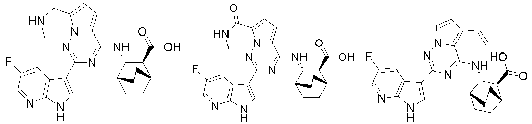

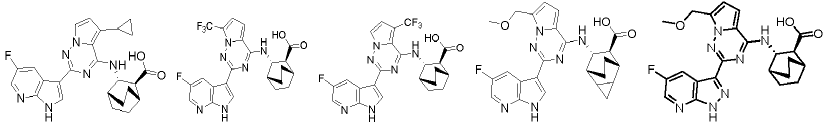

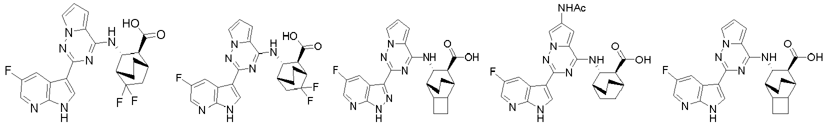

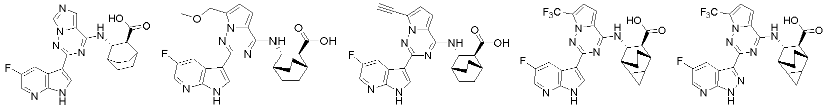

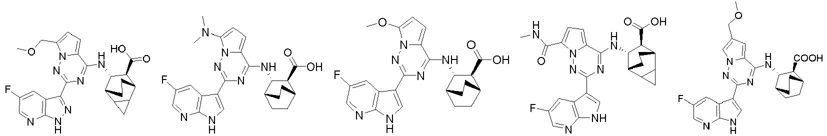

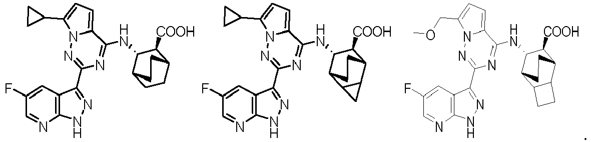

17. A pharmaceutical composition, **characterized in that** the pharmaceutical composition contains the compound, or the stereoisomer, the tautomer, the nitrogen oxide, the solvate, the metabolite, the pharmaceutically acceptable salt, the co-crystal or the prodrug thereof according to any one of claims 1 to 16, and a pharmaceutically acceptable carrier and/or excipient.

18. Use of the compound, or the stereoisomer, the tautomer, the nitrogen oxide, the solvate, the metabolite, the pharmaceutically acceptable salt, the co-crystal or the prodrug thereof according to any one of claims 1 to 16, or the composition according to claim 17 in the preparation of a drug for treating a PB2-mediated disease.

19. The use according to claim 18, **characterized in that** the PB2-mediated disease is a tumour or an autoimmune disease.
